# EUROPEAN PATENT APPLICATION

(11) **EP 4 239 061 A1**
(43) Date of publication of application: **06.09.2023**
(21) Application number: 22160074.5
(22) Date of filing: 03.03.2022
(51) Int. Cl.: C12N 5/0793, C12N 5/079, C12N 5/0797

(54) **METHOD FOR THE GENERATION OF OUTER RADIAL GLIAL (ORG) CELLS**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to a method for the generation of outer radial glial (oRG) cells of the outer sub-ventricular (oSVZ)-like region in cerebral organoids comprising (a) culturing primate stem cells in a primate stem cell medium until about day 6, thereby inducing the formation of embryonic bodies; (b) culturing the embryonic bodies as obtained in step (a) in a neural induction medium until about day 11, thereby inducing the formation of organoids; wherein an inhibitor of TGF-β, an inhibitor of BMP and an inhibitor of WNT is present from about day 2 until about day 11; (c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until about day 40, preferably at least until about day 60 and most preferably at least until about day 80, thereby obtaining cerebral organoids with oRG cells in oSVZ-like regions, wherein the organoids in step (c) are subjected to agitation from about day 15 onward, preferably by using an orbital shaker or a spinning bioreactor; and (d) optionally isolating one or more oRG cells from the oSVZ-like region.

## Description

The present invention relates to a method for the generation of outer radial glial (oRG) cells of the outer sub-ventricular (oSVZ)-like region in cerebral organoids comprising (a) culturing primate stem cells in a primate stem cell medium until about day 6, thereby inducing the formation of embryonic bodies; (b) culturing the embryonic bodies as obtained in step (a) in a neural induction medium until about day 11, thereby inducing the formation of organoids; wherein an inhibitor of TGF-β, an inhibitor of BMP and an inhibitor of WNT is present from about day 2 until about day 11; (c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until about day 40, preferably at least until about day 60 and most preferably at least until about day 80, thereby obtaining cerebral organoids with oRG cells in oSVZ-like regions, wherein the organoids in step (c) are subjected to agitation from about day 15 onward, preferably by using an orbital shaker or a spinning bioreactor; and (d) optionally isolating one or more oRG cells from the oSVZ-like region.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The brain of primates and in particular human is an extremely complex system of heterogeneous tissues and consists of a diverse array of neurons. The current knowledge of primate brains including the human brain is mostly based on post-mortem corpse brain specimens. Animal models of non-primates display several discrepancies compared to the primate and human brain. These deficiencies have posed great challenges for studying the development of the primate central nervous system (CNS) and related diseases. It is therefore a difficult task to study the brain of primates and to understand how primate brains works, in particular when it comes to neurodegenerative diseases.

In view of this difficult task cerebral organoids have been developed as an *in vitro* neurological model for studying the function of primate brains and diseases thereof *in vitro* in a more simple and variable space. Cerebral organoids can be derived from induced pluripotent stem cells and embryonic stem cells under three-dimensional culture condition. The generation of an organoid requires the self-assembly of stem cells, progenitor cells, and multiple types of differentiated cells. Organoids display structures that resemble defined brain regions and simulate specific changes of neurological disorders. While cerebral organoids have become an excellent model for investigating brain development and neurological diseases (Zhou et al. (2020), Front. Cell Dev. Biol, 8:Article 579659), there is an ongoing need for novel methods for the generation of cerebral organoids that allow to study further aspects of primate brain development and diseases of primate brains. This need is addressed by the present invention.

The present invention therefore relates in a first aspect to a method for the generation of outer radial glial (oRG) cells of the outer sub-ventricular (oSVZ)-like region in cerebral organoids comprising (a) culturing primate stem cells in a primate stem cell medium until about day 6, thereby inducing the formation of embryonic bodies; (b) culturing the embryonic bodies as obtained in step (a) in a neural induction medium until about day 11, thereby inducing the formation of organoids; wherein an inhibitor of TGF-β, an inhibitor of BMP and an inhibitor of WNT is present from about day 2 until about day 11; (c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until about day 40, preferably at least until about day 60 and most preferably at least until about day 80, thereby obtaining cerebral organoids with oRG cells in oSVZ-like regions, wherein the organoids in step (c) are subjected to agitation from about day 15 onward, preferably by using an orbital shaker or a spinning bioreactor; and (d) optionally isolating one or more oRG cells from the oSVZ-like region.

Outer radial glial (oRG) cells are a population of neural stem cells prevalent in the developing primate (including human) cortex that contribute to its cellular diversity and evolutionary expansion (Andrews et al. (2020), eLife; 9:e58737). Evolutionary expansion of the human neocortex is partially attributed to a relative abundance of oRG cells. oRG cells display a characteristic division mode, mitotic somal translocation (MST), in which the soma rapidly translocates toward the cortical plate immediately prior to cytokinesis. oRG cells are derived from ventricular radial glia (vRG), the primary neural stem cells present in all mammals. oRG cells reside primarily within the outer subventricular zone (oSVZ), closer to the cortical plate than vRG cells, and lack the apical ventricular contact characteristic of vRG cell (Ostrem et al (2014), 8(3):656-664).

During cortical development in primates, progenitor cells form via symmetrical and asymmetrical division from the ventricle lining to populate the ventricular zone (VZ) and subventricular zone (SVZ). In primates, the VZ gives rise to deeper cortical layers whose neurons make connections to deeper brain structures. The SVZ gives rise to superficial cortical layers whose neurons makes intracortical connections and an additional outer zone of neural precursors has been identified, the outer subventricular zone (oSVZ). While the VZ drives corticogenesis in rodents, the VZ declines rapidly in primates in conjunction with the appearance of the SVZ and oSVZ. Whereas in rodents the SVZ is only partially self-sustaining and must receive a supply of precursors from the VZ, the primate SVZ and oSVZ has a self-sustaining, amplifying population of oRG cells. Enlargement of the SVZ precursor pool of oRG cells in primates ensures an increase in neuron production necessary to form a more complex cortex and in turn facilitate communication between different cortical areas. The population of oSVZ is drastically overrepresented, even compared with other primates, in humans.

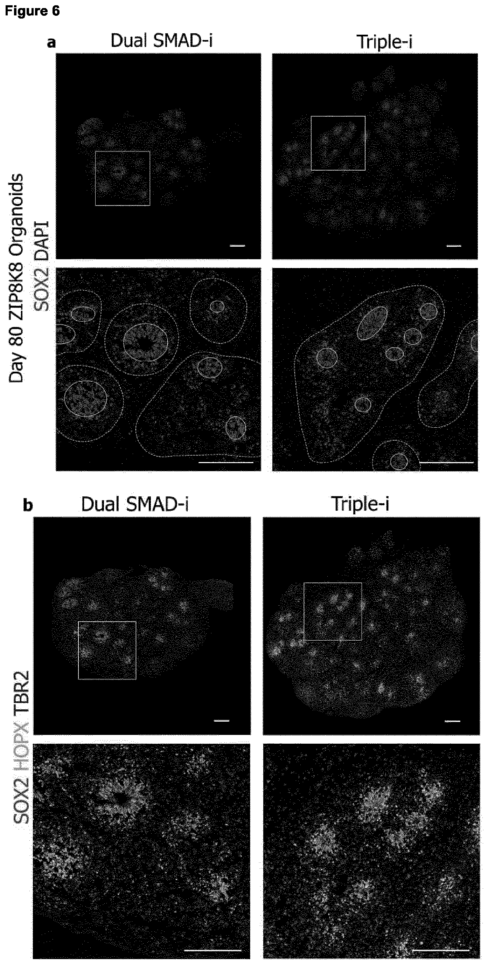

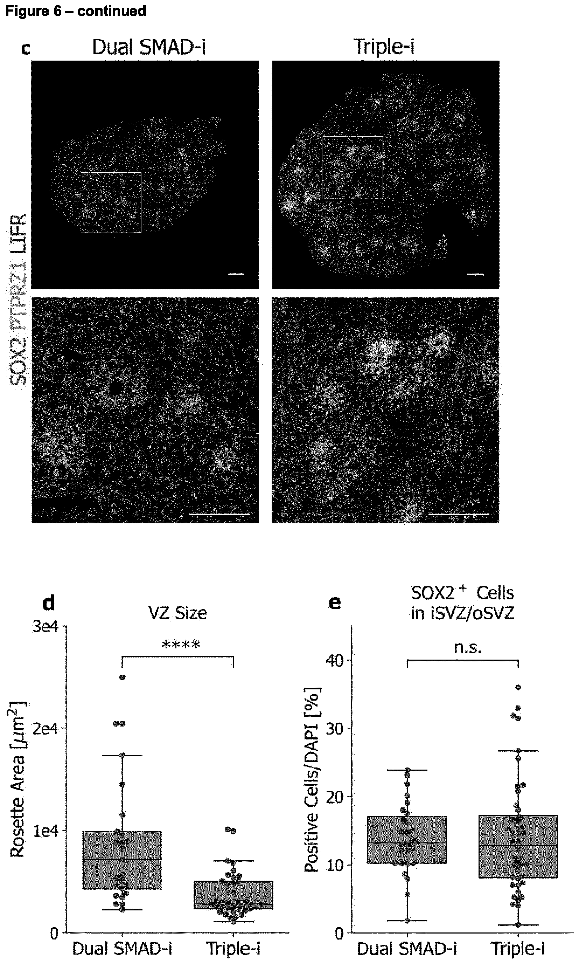

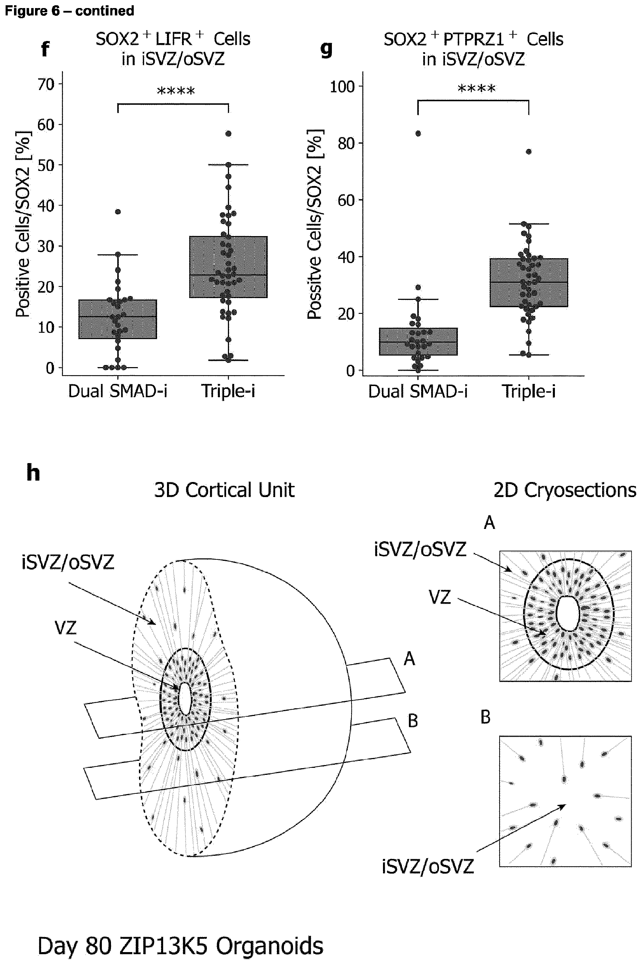

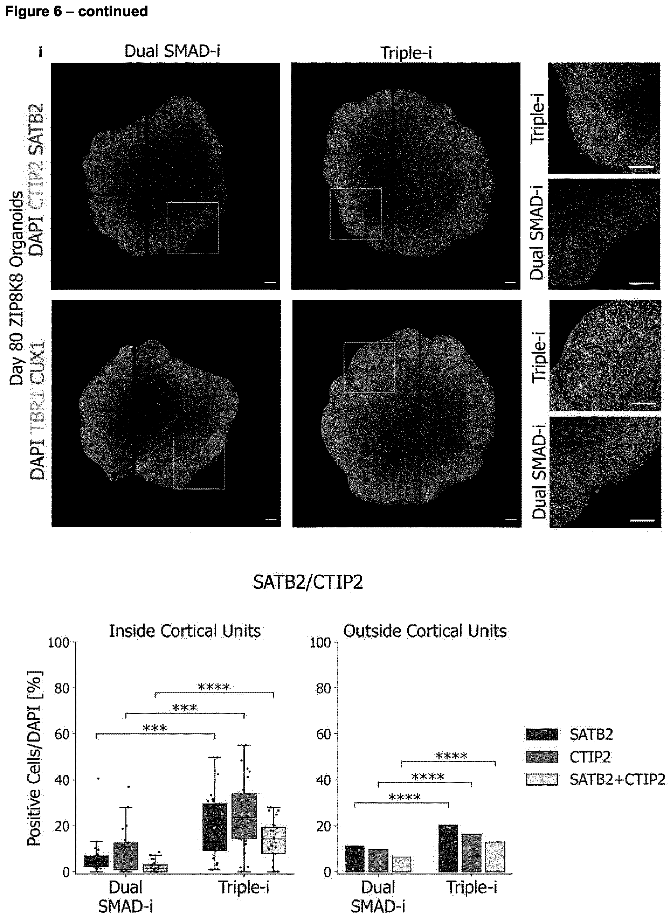

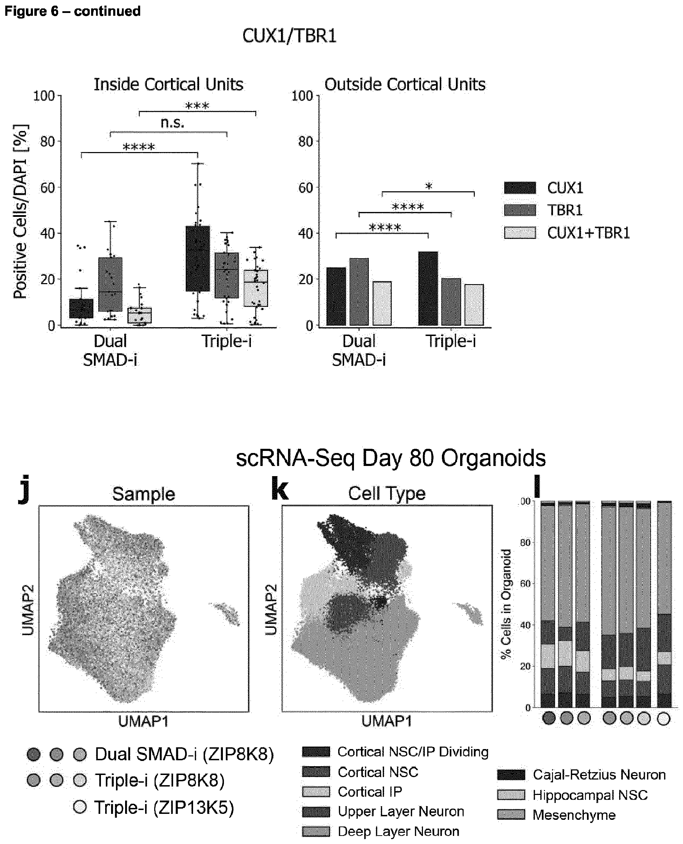

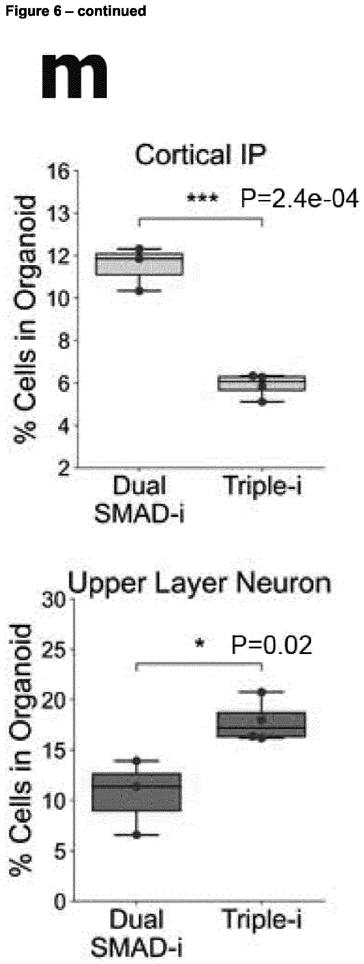

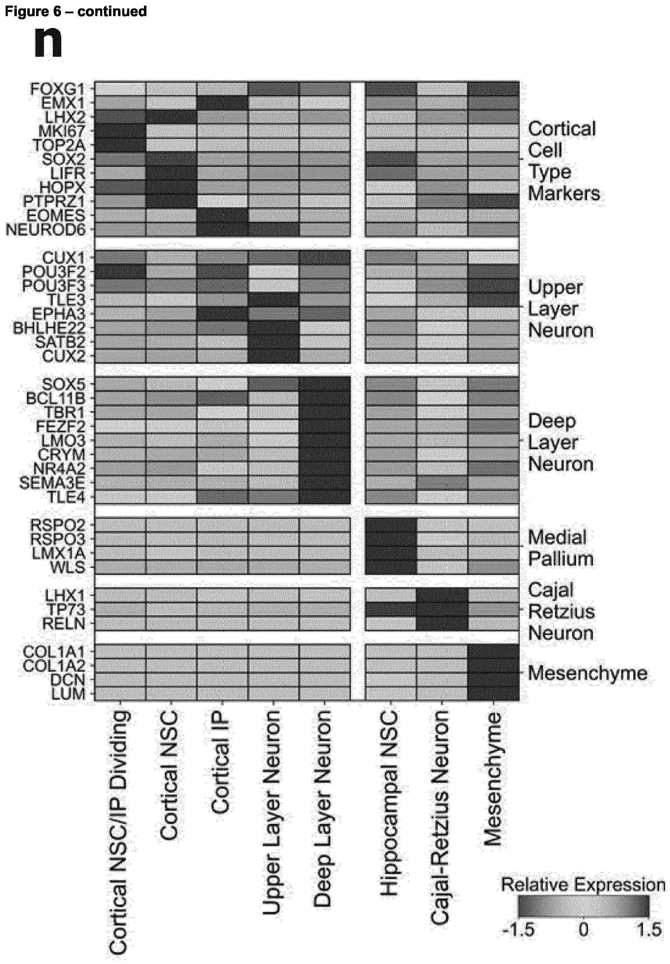

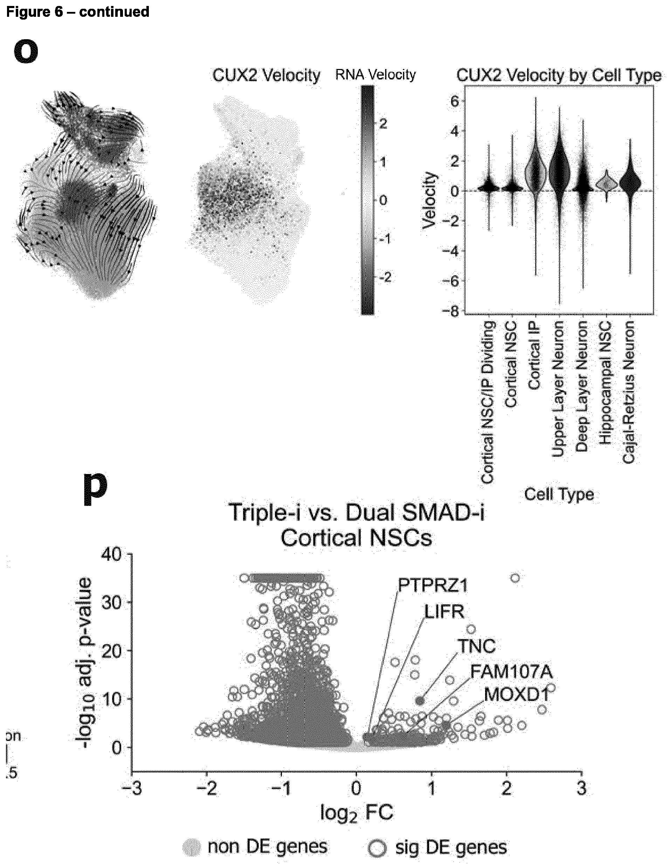

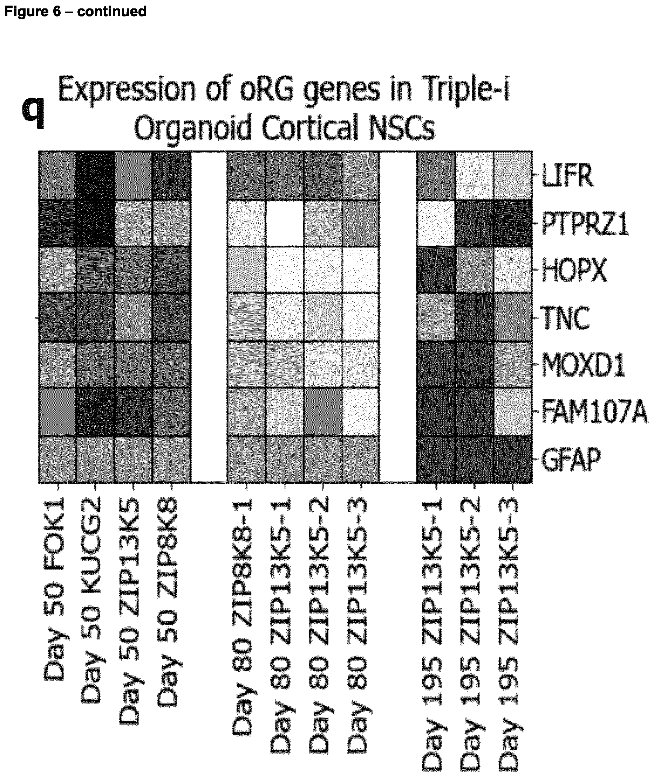

The at least until about day 80 are preferably at least about 120 day and more preferably at least about 195 days. At day 80 so-called "cortical units" were observed in the organoids (see dashed lines in Figure 6), which in particular include iSVZ/oSVZ regions. Also on day 80 the organoids were predominantly comprised of these cortical units. The cortical units in the organoids from about day 80 onward contain mainly oRG cells. Discrete cortical units can be observed being enriched with oRG cells. These cortical units containing oRG cells make it particularly easy to test and isolate oRG cells.

A cerebral organoid (or brain organoid) generally describes an artificially grown, *in vitro,* miniature organ resembling the brain. The cerebral organoids according to the invention are generated from primate pluripotent stem cells. Cerebral organoids can be used as three-dimensional *in vitro* models of the developing primate brain. This is because they display a cellular composition and structural organization being representative of the developing primate brain.

The nature of the primate stem cells to be used herein is not particularly limited as long as they display the capability of differentiating into the required cerebral organoids when subjected to the method of the present invention. In accordance with the method of the first aspect the required cerebral organoids are characterized by ventricular zone (ventricular) radial glial (vRG) cells and outer radial glial (oRG) cells of the outer sub-ventricular (oSVZ)-like region. The primate stem cells as described herein are preferably human stem cells.

Similarly, the composition of the primate stem cell medium is not particularly limited as long as the culturing of primate stem cells in the medium under suitable conditions results in the formation of embryonic bodies within about 6 days, i.e. until about day 6.

Embryonic bodies (EBs) are three-dimensional aggregates of pluripotent stem cells. They compromise the three embryonic germ layers. EBs are formed by the homophilic binding of the Ca²⁺ dependent adhesion molecule E-cadherin, which is highly expressed on undifferentiated primate stem cells. When cultured as single cells in the absence of anti-differentiation factors, primate stem cells spontaneously aggregate to form EBs.

In the course of the methods as described herein EBs are the precursors of the organoids. The organoids are generated from the EBs by culturing the EBs in a neural induction medium.

Again, the composition of the neural induction medium is not particularly limited as long as the culturing of EBs in the medium under suitable conditions results in the formation of organoids form about day 6 to about 11 days, i.e. until about day 11 of the total culture time.

In accordance with the method of the invention as described herein an inhibitor of TGF-β, an inhibitor of BMP and an inhibitor of WNT is present from about day 2 until about day 11. Hence, it is to be understood that from about day 2 to until about day 6 these three inhibitors are present in the primate stem cell medium while from about day 6 until about day 11 they are present in the neural induction medium.

In connection with all times as described herein (e.g., until about day 6, until about day 11, until about day 40, from about day 2 until about day 11, or form about day 15 onward) the term "about" means with increasing preference ± 30%, 20%, 10%, 5%, 2.5% and 1%. For instance, 6 days correspond to 144h and therefore about 6 days are with increasing preference 144±43.2h, 144±28.8h, 144±14.4h, 144±7.2h, 144±3.6h, and 144±1.44h. Similarly, 11 days correspond to 264h and therefore about 11 days are with increasing preference 264±79.2h, 264±52.8h, 264±26.4h, 264±13.2h, 264±7.6h, and 264±2.64h.

The change from one medium to another medium is generally effected herein by transferring the EBs or organoids from one medium to another medium. As an alternative the old medium may be removed and replaced by another medium while the EBs or organoids remain in the same culture dish or well.

The same medium, for example, the neural induction medium may also be replaced or partially replaced during the culture time be the same fresh medium, for example, neural induction medium, if necessary or advantageous for promoting growth and/or differentiation. In each step the respective medium to be used may be exchanged one or more times.

The combination of these three inhibitors is known as "Triple-i" (Thesis of Naresh Mutukula, "Modeling cortical development and microcephaly in human pluripotent stem cells using combinatorial pathway inhibition" (2019), Department of Biology, Chemistry and Pharmacy of Freie Universität Berlin). It is known from this thesis that Triple-i-derived cerebral organoids after 30 days display forebrain/cortical embryonic structures. Hence, early and short exposure (i.e. from about day 2 to about day 11) to these three inhibitions was found to result in the specification of growing organoids towards forebrain / neocortical fates after 30 days.

Transforming growth factor beta (TGF-β) is a multifunctional cytokine belonging to the transforming growth factor superfamily that includes three different mammalian isoforms (TGF-β 1 to 3, HGNC symbols TGFB1, TGFB2, TGFB3) and many other signalling proteins. Signalling by the transforming growth factor β (TGF-β) family is necessary for proper neural development and function throughout life. Sequential waves of activation, inhibition, and reactivation of TGF-β family members regulate numerous elements of the nervous system from the earliest stages of embryogenesis through adulthood (Meyer and Kessler (2017), Cold Spring Harb Perspect Biol. 2017 Aug; 9(8): a022244.).

Bone morphogenetic proteins (BMPs) are a group of growth factors also known as cytokines and as metabologens. Originally discovered by their ability to induce the formation of bone and cartilage, BMPs are now considered to constitute a group of pivotal morphogenetic signals, orchestrating tissue architecture throughout the body. Inhibition of BMP signalling is a prerequisite for the induction of neural plate from ectoderm. Studies identified that the ability of the Spemann organizer or the notochord in neutralizing the overlying ectoderm is due to the expression of BMP antagonist proteins like Noggin, Follistatin and Chordin. They prevent the binding of BMPs. especially BMP-4 to its receptor, thereby inhibiting the BMP pathway.

WNT signalling is an important pathway for neural induction as well as for the axis patterning process. It has roles in both cell proliferation and fate specification. Inhibition of WNT pathway establishes anterior fates. Studies in mice mutants of the WNT co-receptors Lrp5 and Lrp6 show the expansion of the anterior neuroectoderm. Loss of the WNT inhibitor Dickkopf 1 (Dkk1) in mouse prevents the formation of forebrain. Wnt1 is expressed in caudal midbrain-hindbrain regions, and in accordance therewith, loss of Wnt1 in mouse embryos leads to defects in midbrain and anterior hindbrain formation, while Wnt8c overexpression causes the expansion of midbrain fates on the expanse of anterior forebrain fates.

The inhibitors of TGFβ, BMP and WNT signalling may either inhibit the nucleic acid encoding TGFβ, BMP or a member of the WNT signalling and/or inhibit of the protein TGFβ, BMP or a protein being a member of the WNT signalling pathway. The inhibitor of the nucleic acid molecule is preferably selected from a small molecule, an aptamer, a siRNA, a shRNA, a miRNA, a ribozyme, an antisense nucleic acid molecule, a CRISPR-Cas9-based construct, a CRISPR-Cpf1-based construct, a meganuclease, a zinc finger nuclease, and a transcription activator-like (TAL) effector (TALE) nuclease. The inhibitor of the protein is preferably selected from a small molecule, an antibody or antibody mimetic, and an aptamer, wherein the antibody mimetic is preferably selected from affibodies, adnectins, anticalins, DARPins, avimers, nanofitins, affilins, Kunitz domain peptides, Fynomers^{®}, trispecific binding molecules and probodies.

The "small molecule" as used herein is preferably an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. The organic molecule is preferably an aromatic molecule and more preferably a heteroaromatic molecule. In organic chemistry, the term aromaticity is used to describe a cyclic (ring-shaped), planar (flat) molecule with a ring of resonance bonds that exhibits more stability than other geometric or connective arrangements with the same set of atoms. Aromatic molecules are very stable, and do not break apart easily to react with other substances. In a heteroaromatic molecule at least one of the atoms in the aromatic ring is an atom other than carbon, e.g. N, S, or O. For all above-described organic molecules the molecular weight is preferably in the range of 200 Da to 1500 Da and more preferably in the range of 300 Da to 1000 Da.

Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 Da, or less than about 1000 Da such as less than about 500 Da, and even more preferably less than about Da amu. The size of a small molecule can be determined by methods well-known in the art, e.g., mass spectrometry. The small molecules may be designed, for example, based on the crystal structure of the target molecule, where sites presumably responsible for the biological activity can be identified and verified in in vivo assays such as in vivo high-throughput screening (HTS) assays.

The term "antibody" as used in accordance with the present invention comprises, for example, polyclonal or monoclonal antibodies. Furthermore, also derivatives or fragments thereof, which still retain the binding specificity to the target, e.g. TGFβ, are comprised in the term "antibody". Antibody fragments or derivatives comprise, inter alia, Fab or Fab' fragments, Fd, F(ab')2, Fv or scFv fragments, single domain VH or V-like domains, such as VhH or V-NAR-domains, as well as multimeric formats such as minibodies, diabodies, tribodies or triplebodies, tetrabodies or chemically conjugated Fab'-multimers (see, for example, Harlow and Lane "Antibodies, A Laboratory Manual", Cold Spring Harbor Laboratory Press, 198; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigen. The first antigen can be found on the protein of the invention. The second antigen may, for example, be a tumor marker that is specifically expressed on cancer cells or a certain type of cancer cells. Non-limting examples of bispecific antibodies formats are Biclonics (bispecific, full length human IgG antibodies), DART (Dual-affinity Re-targeting Antibody) and BiTE (consisting of two single-chain variable fragments (scFvs) of different antibodies) molecules (Kontermann and Brinkmann (2015), Drug Discovery Today, 20(7):838-847).

The term "antibody" also includes embodiments such as chimeric (human constant domain, non-human variable domain), single chain and humanised (human antibody with the exception of non-human CDRs) antibodies.

Various techniques for the production of antibodies are well known in the art and described, e.g. in Harlow and Lane (1988) and (1999) and Altshuler et al., 2010, loc. cit. Thus, polyclonal antibodies can be obtained from the blood of an animal following immunisation with an antigen in mixture with additives and adjuvants and monoclonal antibodies can be produced by any technique which provides antibodies produced by continuous cell line cultures. Examples for such techniques are described, e.g. in Harlow E and Lane D, Cold Spring Harbor Laboratory Press, 1988; Harlow E and Lane D, Using Antibodies: A Laboratory Manual, Cold Spring Harbor Laboratory Press, 1999 and include the hybridoma technique originally described by Köhler and Milstein, 1975, the trioma technique, the human B-cell hybridoma technique (see e.g. Kozbor D, 1983, Immunology Today, vol.4, 7; Li J, et al. 2006, PNAS, vol. 103(10), 3557) and the EBV-hybridoma technique to produce human monoclonal antibodies (Cole et al., 1985, Alan R. Liss, Inc, 77-96). Furthermore, recombinant antibodies may be obtained from monoclonal antibodies or can be prepared de novo using various display methods such as phage, ribosomal, mRNA, or cell display. A suitable system for the expression of the recombinant (humanised) antibodies may be selected from, for example, bacteria, yeast, insects, mammalian cell lines or transgenic animals or plants (see, e.g., US patent 6,080,560; Holliger P, Hudson PJ. 2005, Nat Biotechnol., vol. 23(9), 11265). Further, techniques described for the production of single chain antibodies (see, inter alia, US Patent 4,946,778) can be adapted to produce single chain antibodies specific for a desired epitope, e.g. of TGFβ. Surface plasmon resonance as employed in the BIAcore system can be used to increase the efficiency of phage antibodies.

As used herein, the term "antibody mimetics" refers to compounds which, like antibodies, can specifically bind antigens, such as for example, TGFβ in the present case, but which are not structurally related to antibodies. Antibody mimetics are usually artificial peptides or proteins with a molar mass of about 3 to 20 kDa. For example, an antibody mimetic may be selected from the group consisting of affibodies, adnectins, anticalins, DARPins, avimers, nanofitins, affilins, Kunitz domain peptides, Fynomers^{®}, trispecific binding molecules and prododies. These polypeptides are well known in the art and are described in further detail herein below.

The term "affibody", as used herein, refers to a family of antibody mimetics which is derived from the Z-domain of staphylococcal protein A. Structurally, affibody molecules are based on a three-helix bundle domain which can also be incorporated into fusion proteins. In itself, an affibody has a molecular mass of around 6kDa and is stable at high temperatures and under acidic or alkaline conditions. Target specificity is obtained by randomisation of 13 amino acids located in two alpha-helices involved in the binding activity of the parent protein domain (Feldwisch J, Tolmachev V.; (2012) Methods Mol Biol. 899:103-26).

The term "adnectin" (also referred to as "monobody"), as used herein, relates to a molecule based on the 10th extracellular domain of human fibronectin III (10Fn3), which adopts an Ig-like β-sandwich fold of 94 residues with 2 to 3 exposed loops, but lacks the central disulphide bridge (Gebauer and Skerra (2009) Curr Opinion in Chemical Biology 13:245-255). Adnectins with the desired target specificity, i.e., for example, against TGFβ, can be genetically engineered by introducing modifications in specific loops of the protein.

The term "anticalin", as used herein, refers to an engineered protein derived from a lipocalin (Beste G, Schmidt FS, Stibora T, Skerra A. (1999) Proc Natl Acad Sci U S A. 96(5):1898-903; Gebauer and Skerra (2009) Curr Opinion in Chemical Biology 13:245-255). Anticalins possess an eight-stranded β-barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape.

As used herein, the term "DARPin" refers to a designed ankyrin repeat domain (166 residues), which provides a rigid interface arising from typically three repeated β-turns. DARPins usually carry three repeats corresponding to an artificial consensus sequence, wherein six positions per repeat are randomised. Consequently, DARPins lack structural flexibility (Gebauer and Skerra, 2009).

The term "avimer", as used herein, refers to a class of antibody mimetics which consist of two or more peptide sequences of 30 to 35 amino acids each, which are derived from A-domains of various membrane receptors and which are connected by linker peptides. Binding of target molecules occurs via the A-domain and domains with the desired binding specificity, e.g. for TGFβ, can be selected, for example, by phage display techniques. The binding specificity of the different A-domains contained in an avimer may but does not have to be identical (Weidle UH, et al., (2013), Cancer Genomics Proteomics; 10(4):155-68).

A "nanofitin" (also known as affitin) is an antibody mimetic protein that is derived from the DNA binding protein Sac7d of Sulfolobus acidocaldarius. Nanofitins usually have a molecular weight of around 7kDa and are designed to specifically bind a target molecule, such as e.g. TGFβ, by randomising the amino acids on the binding surface (Mouratou B, Béhar G, Paillard-Laurance L, Colinet S, Pecorari F., (2012) Methods Mol Biol.; 805:315-31).

The term "affilin", as used herein, refers to antibody mimetics that are developed by using either gamma-B crystalline or ubiquitin as a scaffold and modifying amino-acids on the surface of these proteins by random mutagenesis. Selection of affilins with the desired target specificity, i.e., for example, against TGFβ, is effected, for example, by phage display or ribosome display techniques. Depending on the scaffold, affilins have a molecular weight of approximately 10 or 20kDa. As used herein, the term affilin also refers to di- or multimerised forms of affilins (Weidle UH, et al., (2013), Cancer Genomics Proteomics; 10(4):155-68).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDA and domains with the required target specificity, i.e., for example, against TGFβ, can be selected by display techniques such as phage display (Weidle et al., (2013), Cancer Genomics Proteomics; 10(4):155-68).

As used herein, the term "Fynomer^{®}" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

The term "trispecific binding molecule" as used herein refers to a polypeptide molecule that possesses three binding domains and is thus capable of binding, preferably specifically binding to three different epitopes. At least one of these three epitopes is an epitope of the protein of the fourth aspect of the invention. The two other epitopes may also be epitopes of the protein of the fourth aspect of the invention or may be epitopes of one or two different antigens. The trispecific binding molecule is preferably a TriTac. A TriTac is a T-cell engager for solid tumors which comprised of three binding domains being designed to have an extended serum half-life and be about one-third the size of a monoclonal antibody.

As used herein, the term "probody" refers to a protease-activatable antibody prodrug. A probody consists of an authentic IgG heavy chain and a modified light chain. A masking peptide is fused to the light chain through a peptide linker that is cleavable by tumor-specific proteases. The masking peptide prevents the probody binding to healthy tissues, thereby minimizing toxic side effects.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

Nucleic acid aptamers are nucleic acid species that normally consist of (usually short) strands of oligonucleotides. Typically, they have been engineered through repeated rounds of in vitro selection or equivalently, SELEX (systematic evolution of ligands by exponential enrichment) to bind to various molecular targets such as small molecules, proteins, nucleic acids, and even cells, tissues and organisms.

Peptide aptamers are usually peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys-loop (SEQ ID NO: 8) in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminatory recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamers' inherently low molecular weight. Unmodified aptamer applications currently focus on treating transient conditions such as blood clotting or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin or other half-life extending proteins etc. are available to scientists such that the half-life of aptamers can be increased for several days or even weeks.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well-defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene for which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1 to 5 nucleotides, more preferably from 1 to 3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair. 2'-deoxynucleotides in the 3' overhangs are as efficient as ribonucleotides but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics).

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules. Endogenously present miRNA molecules regulate gene expression by binding to a complementary mRNA transcript and triggering of the degradation of said mRNA transcript through a process similar to RNA interference. Accordingly, exogenous miRNA may be employed as an inhibitor of, for example, TGFβ after introduction into the respective cells.

A ribozyme (from ribonucleic acid enzyme, also called RNA enzyme or catalytic RNA) is an RNA molecule that catalyses a chemical reaction. Many natural ribozymes catalyse either their own cleavage or the cleavage of other RNAs, but they have also been found to catalyse the aminotransferase activity of the ribosome. Non-limiting examples of well-characterised small self-cleaving RNAs are the hammerhead, hairpin, hepatitis delta virus, and *in vitro*-selected lead-dependent ribozymes, whereas the group I intron is an example for larger ribozymes. The principle of catalytic self-cleavage has become well established in recent years. The hammerhead ribozymes are characterised best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: A region of interest of the RNA, which contains the GUC (or CUC) triplet, is selected. Two oligonucleotide strands, each usually with 6 to 8 nucleotides, are taken and the catalytic hammerhead sequence is inserted between them. The best results are usually obtained with short ribozymes and target sequences.

A recent development, also useful in accordance with the present invention, is the combination of an aptamer, recognizing a small compound, with a hammerhead ribozyme. The conformational change induced in the aptamer upon binding the target molecule can regulate the catalytic function of the ribozyme.

The term "antisense nucleic acid molecule", as used herein, refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

CRISPR/Cas9, as well as CRISPR-Cpf1, technologies are applicable in nearly all cells/model organisms and can be used for knock out mutations, chromosomal deletions, editing of DNA sequences and regulation of gene expression. The regulation of the gene expression can be manipulated by the use of a catalytically dead Cas9 enzyme (dCas9) that is conjugated with a transcriptional repressor to repress transcription a specific gene, here, for example, the TGFβ gene. Similarly, catalytically inactive, "dead" Cpf1 nuclease (CRISPR from Prevotella and Francisella-1) can be fused to synthetic transcriptional repressors or activators to downregulate endogenous promoters, e.g. the promoter which controls TGFβ expression. Alternatively, the DNA-binding domain of zinc finger nucleases (ZFNs) or transcription activator-like effector nucleases (TALENs) can be designed to specifically recognize the TGFβ gene or its promoter region or its 5'-UTR thereby inhibiting the expression of the TGFβ gene.

Inhibitors provided as inhibiting nucleic acid molecules that target the gene of interest, e.g. the TGFβ gene or a regulatory molecule involved in target gene expression are also envisaged herein. Such molecules, which reduce or abolish the expression of the target gene or a regulatory molecule include, without being limiting, meganucleases, zinc finger nucleases and transcription activator-like (TAL) effector (TALE) nucleases. Such methods are described in Silva et al., Curr Gene Ther. 2011;11(1):11-27; Miller et al., Nature biotechnology. 2011;29(2):143-148, and Klug, Annual review of biochemistry. 2010; 79:213-231.

In the method of the first aspect the invention the organoids (c) as obtained after steps (a) and (b) - provided that they display a size of at least 300 µm - are embedded into a hydrogel that mimics the extracellular matrix (ECM), preferably Matrigel. The Martigel-embedded organoids are then and cultured in a cerebral differentiation medium at least until about day 40, preferably at least until about day 60 and most preferably at least until about day 80 (each total culturing time staring with the stem cells on day 1). Thereby cerebral organoids with oRG cells in oSVZ-like regions are obtained. In addition, the Martigel-embedded organoids are subjected to agitation from about day 15 onward. The agitation is preferably achieved by using an orbital shaker or a spinning bioreactor and most preferably by an orbital shaker. The agitation is preferably conducted at about 86 rpm, wherein the term "about" means with increasing preference ± 30%, 20%, 10%, 5%, 2.5% and 1%.

The at least until about day 40 are with increasing preference at least until about day 45, at least until about day 50, at least until about day 55, at least until about day 60, at least until about day 65, at least until about day 70, and at least until about day 75, at least until about day 80, at least until about day 120 and at least until about day 195.

Organoids displaying a size of at least 300 µm in diameter are generally self-sustained; i.e. the organoids established intracellular connections that resemble tissue. The size of at least 300 µm in diameter is preferably at least 350 µm in diameter and most preferably at least 400 µm in diameter.

The upper size of the organoids in diameter is preferably 600 µm and more preferably. All possible ranges of the lower and upper diameter are also described herein, such as 300 to 600 µm or 400 to 500 µm.

The hydrogel that mimics the extracellular matrix (ECM) preferably resembles the laminin/collagen IV-rich basement membrane extracellular environment found in many tissues and is used by cell biologists as a substrate (basement membrane matrix) for culturing cells. The extracellular matrix (ECM) as provided by the hydrogel is an important component in generating 3D models, in order to provide biochemical cues and structural support, such as porosity and stiffness which mediates signalling for cell migration, cell behaviour and polarization in organoid structures.

The hydrogel is preferably Matrigel. Matrigel is the most widely used hydrogel that mimics the extracellular matrix (ECM). Matrigel is the trade name for the solubilized basement membrane matrix secreted by Engelbreth-Holm-Swarm (EHS) mouse sarcoma cells produced by Corning Life Sciences. Hence, may be defined as the solubilized basement membrane matrix secreted by Engelbreth-Holm-Swarm (EHS) mouse sarcoma cells. A commercially available alternatives of Matrigel is, for example, GrowDex cell culture matrix mix. Synthetic alternatives to Matrigel are reviewed, for example, in Aisenbrey and Murphy (2020), Nat Rev Mater; 5(7):539-551.

Agitation at low shear stress of the hydrogel-embedded, preferably Matrigel-embedded organoids in suspension culture, preferably by using an orbital shaker or a spinning bioreactor, is required for nutrient diffusion, which preserves tissue structure (Goto-Silva (2019), BMC Developmental Biology, 19:Article number:3).

The method of the first aspect of the invention optionally comprises the step of isolating one or more oRG cells from the oSVZ-like region. Means and methods for isolating cells from organoids are art-established. For instance, the cells of an organoid may be dissociated and plated. The dissociated cells can be maintained in culture for up to at least 3 weeks (Yan et al (2020), Methods Mol Biol; 10.1007/7651_2020_328). Dissociated cells may also be sorted by fluorescence-assisted cell sorting (FACS) including gating and sorting implementation (Janssens et al. (2019), Curr Protoc Stem Cell Biol; 48(1):e65).

In an exemplary but preferred embodiment step (a) is conducted in 6 cm dishes or a 6 well plate, step (b) may be conducted in 24-well plates and step (c) in 6 well plates.

Hydrogel-embedding, such as Matrigel-embedding may be conducted in parafilm dimples as described in the thesis of Naresh Mutukula, loc. lit.

As described herein above, the combination of an inhibitor of TGF-β, an inhibitor of BMP and an inhibitor of WNT is known as "Triple-i" from the thesis of Naresh Mutukula, loc. lit. in a method of the generation of cerebral organoids from human embryonic stem cells. Is also known from this thesis that Triple-i-derived cerebral organoids after 30 days display forebrain/cortical embryonic structures. It was now surprisingly found that if the organoids are kept in culture for longer times (at least until about day 40, preferably at least until about day 60 and most preferably at least until about day 80) outer radial glial (oRG) cells of the outer sub-ventricular (oSVZ)-like region can be identified in the cerebral organoids. Hence, it was found that the Triple-i inhibitor enables the emergence of oRG cells. The oRG cells emerged in a clearly distinct oSVZ region. The number of oRG cells in the defined oSVZ regions further increases when expanding the culture time from at least 40 day until 80 days. To the best knowledge of the inventors the day 40 and fortiori the day 80 organoids as obtained by the present invention display in their oSVZ regions the highest number of and most enriched population of *ex vivo* oRG cells. Since oRG cells are only observed in primates and the evolutionary expansion of the human neocortex is partially attributed to a relative abundance of oRG cells, cerebral organoids with an area displaying a high number of oRG cells offer new possibilities of studying primate brain development as well as brain diseases that affect or might affect oRG cells.

The present invention relates in a second aspect to a method for determining whether a test compound is an inhibitor or activator of the generation of outer radial glial (oRG) cells comprising (a) culturing primate stem cells in a primate stem cell medium until about day 6, thereby inducing the formation of embryonic bodies; (b) culturing the embryonic bodies as obtained in step (a) in a neural induction medium until about day 11, thereby inducing the formation of organoids; wherein an inhibitor of TGF-β, an inhibitor of BMP, an inhibitor of WNT and the test compound are present from about day 2 until about day 11; and (c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until day 40, thereby obtaining cerebral organoids with oRG cells in pSVZ-like regions, wherein the organoids in step (c) are subjected to agitation from about day 15 onward, preferably by using an orbital shaker or a spinning bioreactor, wherein the test compound is an inhibitor of the generation of oRG cells if the number of oRG cells in oSVZ-like regions is reduced as compared to the absence of the test compound, and wherein the test compound is an activator of the generation of oRG cells if the number of oRG cells in oSVZ-like regions is increased as compared to the absence of the test compound.

The test compound is an inhibitor of the generation of oRG cells if the number of oRG cells in oSVZ-like regions is reduced with increasing preference by at least 10%, at least 20%, at least 30%, at least 40%, at least 50%, at least 60%, at least 70%, at least 80%, and at least 90% as compared to the absence of the test compound.

Similarly, the test compound is an activator of the generation of oRG cells if the number of oRG cells in oSVZ-like regions is increased with increasing preference by at least 10%, at least 20%, at least 30%, at least 40%, at least 50%, at least 60%, at least 70%, at least 80%, and at least 90% as compared to the absence of the test compound.

The method of the second aspect may also be conducted as a high-throughput assays, wherein several test compounds are tested in parallel. This is generally performed in wells of microtiter plates. Handling of the plates, including incubation at temperatures other than ambient temperature, and bringing into contact the test compounds with the assay mixture is preferably effected by one or more computer-controlled robotic systems including pipetting devices. In case large libraries of test compounds are to be screened and/or screening is to be effected within a short time, mixtures of, for example 10, 20, 30, 40, 50 or 100 test compounds may be added to each well. In case a well exhibits the expected activity, said mixture of test compounds may be de-convoluted to identify the one or more test compounds in said mixture giving rise to said activity.

The nature of the test compound is not particularly limited. The test compound is preferably a compound being described as inhibitor in connection with the first aspect of the invention, such as a small molecule or an antibody.

While the test compound is added in step (b) it is also described herein to add the test compound in addition or alternatively at step(s) (a) and/or (c).

The method of the second invention is particularly useful for identifying candidate compounds that can be used for treating brain diseases that are associated with oRG cells. A non-limiting example is microcephaly. Microcephaly can have a genetic cause or an external cause, such as a Zika virus infection or drug consumption.

In accordance with a preferred embodiment of the first and second aspect of the invention the primate stem cells may be feeder-dependent or alternatively, feeder-independent.

Feeder-dependent primate stem cells are maintained on a layer of feeder cells that are often inactive murine embryonic fibroblast (MEF). Feeder-dependent primate stem cells can be maintained under these conditions for several passages without compromising the cells' proliferation or pluripotency and differentiation potential.

Feeder-independent primate stem cells do not require feeder cells. Feeder-independent culture systems improve the safety and efficiency of stem cell technology since they enable large-scale cultures.

For conducting the examples herein below human embryonic stem cells were initially grown in feeders, while they were then grown feeder-free to validate the hESC results in iPSCs. Meanwhile essentially all cell lines can also be grown under feeder free conditions.

In accordance with a further preferred embodiment of the first and second aspect of the invention the primate stem cells are primate embryonic stem cells or primate induced pluripotent stem cells.

The primate embryonic stem cells and primate induced pluripotent stem cells are preferably human embryonic stem cells and human induced pluripotent stem cells.

Embryonic stem cells (ES cells or ESCs) are pluripotent stem cells derived from the inner cell mass of a blastocyst, an early-stage pre-implantation embryo. Human embryos reach the blastocyst stage 4-5 days post fertilization, at which time they consist of 50-150 cells. The primate embryonic stem cells, in particular in the case of human embryonic stem cells were preferably obtained by a method that does not require the destruction of an embryo.

Induced pluripotent stem cells (also known as iPS cells or iPSCs) are a type of pluripotent stem cell that can be generated directly from a somatic cell. Because iPSCs can be derived directly from adult tissues, they bypass the need for embryos and can also be made in a patient-matched manner, which means that each individual could have their own pluripotent stem cell line.

In accordance with another preferred embodiment of the first and second aspect of the invention (a) the inhibitor of TGF-β is SB-431542, (b) the inhibitor of BMP is Noggin, and/or (c) the inhibitor of WNT is XAV-939.

One, any two or all three of these particular three inhibitors are particularly preferred since they are used in the examples. SB-431542 is a selective inhibitor of TGF-βRI and also known as 4-[4-(1,3-benzodioxol-5-yl)-5-(2-pyridinyl)-1H-imidazol-2-yl]benzamide.

In certain embodiments of the invention only one of the specific inhibitors mentioned above may be used whereas for the other two targets different inhibitors the specific one mentioned above are used. Similarly, in further embodiments two of the specific inhibitors mentioned above may be is used whereas the third inhibitor is different from the one specifically mentioned above. For instance, only Noggin may be used together with other inhibitors of BMP and WNT or SB-431542 and Noggin may be used together another inhibitor of WNT.

Noggin, also known as NOG, is a protein that is involved in the development of many body tissues, including nerve tissue, muscles, and bones. In humans, noggin is encoded by the NOG gene. Noggin is an inhibitor of several bone morphogenetic proteins (BMPs): it inhibits at least BMP2, BMP4, BMP5, BMP6, BMP7, BMP13, and BMP14. The noggin is preferably mouse recombinant noggin and more preferably comprises of consists of a sequence being with increasing preference at least 80%, at least 90%, at least 95%, at least 97%, at least 98%, at least 99%, and 100% identical to SEQ ID NO: 1. The mouse noggin is preferably fused via the peptide linker of SEQ ID NO :2 to the human IgG₁ (Pro100-Lys330) of SEQ ID NO: 3. This fusion protein is shown in SEQ ID NO: 4. Hence, the Noggin comprising a sequence being with increasing preference at least 80%, at least 90%, at least 95%, at least 97%, at least 98%, at least 99%, and 100% identical to SEQ ID NO: 1 is preferably a sequence comprising or consisting of a sequence being with increasing preference at least 80%, at least 90%, at least 95%, at least 97%, at least 98%, at least 99%, and 100% identical to SEQ ID NO: 4.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schaffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences. The NCBI BLAST algorithm is available for protein (Protein BLAST) and nucleotides (Nucleotide BLAST). For Protein BLAST the algorithm parameters are preferably: max target sequences: 100, with automatically adjust parameters for short input sequences, expect threshold 0.05, word size 6, Max matches in a query range 0, matrix BLOSUM62, cap cost existence: 10 extension: 1, and compositional adjustment. For Nucleotide BLAST the algorithm parameters are preferably: max target sequences: 100, with automatically adjust parameters for short input sequences, Expect threshold 0.05, word size 28, Max matches in a query range 0, match/mismatch scores 1,-2, cap costs linear, low complexity regions filter, and ,ask for look up table only. These are the standard algorithm parameters for protein BLAST and Nucleotide BLAST and they can adjusted, if needed.

The WNT pathway inhibitor XAV-939 (CAS No. 284028-89-3) inhibits tankyrase (TNKS) 1 and 2 (IC₅₀ = 11 and 4 nM, respectively). By inhibiting TNKS activity, XAV939 increases the protein levels of the axin-GSK3β complex and promotes the degradation of β-catenin, thereby inhibiting WNT pathway downstream actions. It chemical name is 3,5,7,8-tetrahydro-2-[4-(trifluoromethyl)phenyl]-4H-thiopyrano[4,3-d]pyrimidin-4-one.

In the primate stem cell medium as well as in neural induction medium the concentration of SB-431542 is preferably about 10 µM, the concentration of Noggin is preferably about 250 ng/ml, and/or the concentration of XAV-939 is preferably about 3.3 µM. In this connection the term "about" means with increasing preference ± 30%, 20%, 10%, 5%, 2.5% and 1%.

In accordance with a yet further preferred embodiment of the first and second aspect of the invention the primate stem cell medium and/or the neural induction medium comprises a ROCK inhibitor until the embryonic bodies reach a size of at least 300 µm in diameter, wherein the ROCK inhibitor is preferably Y27632.

Herein, the size of at least 300 µm in diameter is preferably at least 350 µm in diameter and most preferably at least 400 µm in diameter. As discussed above, organoids with this size are self-sustained.

Rho-kinase inhibitors (rho-associated protein kinase inhibitor or ROCK inhibitor) are a series of compounds that target rho kinase (ROCK) and inhibit the ROCK pathway. Clinical trials have found that inhibition of the ROCK pathway contributes to the cardiovascular benefits of statin therapy. Furthermore, ROCK inhibitors may have clinical applications for anti-erectile dysfunction, antihypertension, and tumor metastasis inhibition.

The use of a ROCK inhibitor is advantageous since it was found to increase cell viability. In the art and also in the appended examples Y27632, usually Y27632 dihydrochloride is used as ROCK inhibitor. Y27632 inhibits ROCK1 (p160 ROCK) and ROCK2. Its chemical name is trans-4-[(1R)-1-aminoethyl]-N-4-pyridinylcyclohexanecarboxamide dihydrochloride.

Y27632 dihydrochloride is preferably used at a concentration of about 10µM, wherein the term "about" means with increasing preference ± 30%, 20%, 10%, 5%, 2.5% and 1%.

As alternative to Y27632 also Y30141, Y33075 or Y39983 may be used.

In accordance with a further preferred embodiment of the first and second aspect of the invention the primate stem cell medium and/or the neural induction medium comprises medium supplement B27, preferably medium supplement B27 without retionic acid before agitation in step (c) and medium supplement B27 with retionic acid during agitation in step (c).

Medium supplement B27 is the most used medium supplement for neural cells and is commercially available for more than 25 years. A protocol for "home-made" medium B27 supplement is, for example, available from https://hannalabweb.weizmann.ac.il/wp-content/uploads/2016/02/HANNA-LAB-B22-B27-PROTOCOL-V3.pdf. This "home-made" B27 supplement contains the 21 components as discussed below, and 100gr BSA Fraction V IgG free Fatty Acid Poor (Invitrogen 30036578 × 1 unit) assembled in Neurobasal medium (Invitrogen 21103-049 × 2 units). Order these items from Invitrogen to assemble 800ml of B27. B) For 800 ml of B27 the 21 components - that can, for example, be obtained from Sigma-Aldrich - are: 1) Catalase - (store at -20 as is) C40-100MG × 1 unit 2) Glutathione reduced - (store at +4 as is) G6013-5G × 1 unit 3) # Human Insulin - (store as is -20, store aliquots at -80) 91077C-250MG × 1 unit 4) Superoxide Dismutase - (store at -20 as is) S5395-75KU × 4 units 5) Human Holo-Transferin - (store at +4 as is) T0665-100MG × 2 units 6) T3 - (store as is -20, store aliquots at -80) T6397-100MG × 1 unit 7) L-carnitine - (store at RT as is) C0283-1G × 1 unit 8) Ethanolamine - (store at RT as is) E9508-100ML × 1 unit 9) D+-galactose - (store at RT as is) G0625-100G × 1 unit 10) Putrescine - (store at RT as is) P5780-5G × 1 unit 11) Sodium selenite - (store at RT as is, aliquots at -80) S9133-1MG × 1 unit 12) Corticosterone - (store at RT as is, aliquots at -80) C2505-500MG × 1 unit 13) Linoleic acid - (store as is -20, store aliquots at -80) L1012-100MG × 1 unit 14) Linolenic acid - (store as is -20, store aliquots at -80) L2376-500MG × 1 unit 15) Progesterone - (store at RT, store aliquots at -80) P8783-1G × 1 unit 16) Retinol acetate - (store at RT, store aliquots at -80) R7882-1G × 1 unit 17) DL-alpha tocopherol (vit E) (store @+4 as is, store aliquots at -80) T3251-5G × 1 unit 18) DL-alpha tocopherol acetate (store @RT as is, store aliquots at -80) T3001-10G × 1 unit 19) Oleic acid - (store as is -20, store aliquots at -80) 01383-1G × 1 unit 20) Pipecolic acid- (store as is -20, store aliquots at -80) P2519-100MG × 1 unit 21) Biotin - (store at +4 as is) B4639-100MG × 1 unit.

The transition from medium supplement B27 without RA to with RA during agitation is advantageous because RA (retionic acid) promotes neural differentiation of pluripotent cells under a wide variety of culture conditions.

In accordance with a further preferred embodiment of the first and second aspect of the invention the neural induction medium comprises or is N2 medium.

Furthermore in accordance with a preferred embodiment of the first and second aspect of the invention, the cerebral differentiation medium comprises N2 medium and Neurobasal medium supplemented with medium supplement B27, preferably medium supplement B27 with retionic acid.

N2 medium is a serum-free, chemically defined supplement based on Bottenstein's N2 formulation (Bottenstein, J.E. (1985) Cell Culture in the Neurosciences, Bottenstein, J.E. and Harvey, A.L., editors, p. 3, Plenum Press: New York and London). It is recommended for the growth and expression of neuroblastomas and for the survival and expression of post-mitotic neurons in primary cultures from both the peripheral nervous system (PNS) and the central nervous system (CNS).

N2 medium is commercially available as N2 supplement (100×). N2 Supplement diluted into the base medium 1:100. The final concentration of N2 Supplement corresponds to 1×. For preparation of 100 ml medium add 1 ml N2 Supplement is added to 99 ml of the appropriate base medium. The components of N2 supplement are human transferrin (Holo) 10000.00 µg/ml, recombinant human insulin 500.00 µg/ml, progesterone 0.63 µg/ml, putrescine 1611.00 µg/ml and sodium selenite 0.52 µg/ml.

The N2 medium is preferably the "homemade" N2 medium as used in the examples. This N2 medium consists of 6.5g of DMEM/F12 powder (Thermo-Fisher Scientific) and supplements containing 0.775g of D-Glucose, 1g of Sodium bicarbonate, 12.5mg Insulin, 5mg Apo-transferin, 30µl of 500µM Sodium Selenite, 100µl of 830nM Putrescine, 100µl of 100µM Progesterone, and 1% Penicillin/Streptomycin (e.g., from Sigma Aldrich) in 500 ml.

In accordance with another preferred embodiment of the first and second aspect of the invention, the method further comprises the identification of the oRG cells in the cerebral organoids by the detection of the expression of one or more of the marker genes LIFR, PTPRZ1 and HOPX together with the expression of SOX2.

In accordance with a different preferred embodiment of the first and second aspect of the invention, the method further comprises the identification of the oSVZ-like region in the cerebral organoids by the detection of the expression of TBR2 alone or with one or more of the marker genes HOPX, LIFR, PTPRZ1 and SOX2 (preferably HOPX and/or SOX2).

As discussed herein above, in the methods according to the first and second aspect of the invention outer radial glial (oRG) cells of the outer sub-ventricular (oSVZ)-like region are generated in cerebral organoids.

These outer radial glial (oRG) cells can be identified in the cerebral organoids by the expression of SOX2 with one or more, preferably all of the marker genes LIFR, PTPRZ1 and HOPX and/or the oSVZ-like region can be identified in the in cerebral organoids by the expression of one or more, preferably all of the marker genes TBR2, HOPX, and SOX2.

For example, fluorescently labelled antibodies may be used against the proteins being encoded by these marker genes in order to visualize the outer radial glial (oRG) cells and/or the outer sub-ventricular (oSVZ)-like region. These marker genes may also be used for isolating one or more oRG cells from the oSVZ-like region, for example, by deconstruction of the cerebral organoids and FACS sorting.

The LIFR (Leukemia Inhibitory Factor Receptor) gene encodes a protein that belongs to the type I cytokine receptor family. This protein combines with a high-affinity converter subunit, gp130, to form a receptor complex that mediates the action of the leukemia inhibitory factor, a polyfunctional cytokine that is involved in cellular differentiation, proliferation and survival in the adult and the embryo.

The PTPRZ1 (Protein Tyrosine Phosphatase Receptor Type Z1) gene encodes a member of the receptor protein tyrosine phosphatase family. Expression of this gene is restricted to the central nervous system (CNS), and it may be involved in the regulation of specific developmental processes in the CNS. Alternatively spliced transcript variants encoding different isoforms have been described for this gene.

The SOX2 (SRY-Box Transcription Factor 2) gene encodes a member of the SRY-related HMG-box (SOX) family of transcription factors involved in the regulation of embryonic development and in the determination of cell fate. The product of this gene is required for stem-cell maintenance in the central nervous system, and also regulates gene expression in the stomach.

The TBR2 (T-Box Brain Transcription Factor 2) gene encodes a transcription factors of the T-box family. These transcription factors play varied yet crucial roles throughout development. The founding member of the family, brachyury (from the Greek for short tail), was discovered following studies of a short-tailed mouse that harbored a mutation affecting tail length and embryonic development

The HOPX (HOP Homeobox) gene encodes a homeodomain protein that lacks certain conserved residues required for DNA binding. It was reported that choriocarcinoma cell lines and tissues failed to express this gene, which suggested the possible involvement of this gene in malignant conversion of placental trophoblasts.

The LIFR (LIF Receptor Subunit Alpha) gene encodes a protein that belongs to the type I cytokine receptor family. This protein combines with a high-affinity converter subunit, gp130, to form a receptor complex that mediates the action of the leukemia inhibitory factor, a polyfunctional cytokine that is involved in cellular differentiation, proliferation and survival in the adult and the embryo.

The PTPRZ1 (Protein Tyrosine Phosphatase Receptor Type Z1) gene encodes a member of the receptor protein tyrosine phosphatase family. Expression of this gene is restricted to the central nervous system (CNS), and it may be involved in the regulation of specific developmental processes in the CNS.

The present invention relates in a third aspect to an oRG cell that has been produced or is producible by the method of any the first or second aspect of the invention.

The oRG cell is preferably an isolated oRG cell that has been isolated from the cerebral organoid.

The present invention relates in a fourth aspect to a method for the generation of blood brain barrier (BBB) cells in cerebral organoids comprising (a) culturing primate stem cells in a primate stem cell medium until about day 6, thereby inducing the formation of embryonic bodies; (b) culturing the embryonic bodies as obtained in step (a) in a neural induction medium until about day 11, thereby inducing the formation of organoids; wherein an inhibitor of TGF-β, an inhibitor of BMP and an inhibitor of WNT is present from about day 2 until about day 11; and wherein miR20b is recombinantly expressed from about day 2 onward; (c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until about day 50, thereby obtaining cerebral organoids with BBB cells, wherein the organoids in step (c) are subjected to agitation from about day 15 onward, preferably by using an orbital shaker or a spinning bioreactor; and (d) optionally isolating one or more BBB cells.

In this connection the present invention also relates to a method for determining whether a test compound is an inhibitor or activator of the generation of BBB cells comprising (a) culturing primate stem cells in a primate stem cell medium until about day 6, thereby inducing the formation of embryonic bodies; (b) culturing the embryonic bodies as obtained in step (a) in a neural induction medium until about day 11, thereby inducing the formation of organoids; wherein an inhibitor of TGF-β, an inhibitor of BMP and an inhibitor of WNT is present from about day 2 until about day 11; and wherein miR20b is recombinantly expressed from about day 2 onward; and (c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until about day 50, thereby obtaining cerebral organoids with BBB cells, wherein the organoids in step (c) are subjected to agitation from about day 15 onward, preferably by using an orbital shaker or a spinning bioreactor, wherein the test compound is an inhibitor of the generation of BBB cells if the number of BBB cells in the organoids is reduced as compared to the absence of the test compound, and wherein the test compound is an activator of the generation of BBB cells if the number of BBB cells in the organoids is increased as compared to the absence of the test compound.

In this connection the present invention also relates to a method for determining whether a test compound is capable of passing blood brain barrier (BBB) cells in a cerebral organoid as obtained by the method of the fourth aspect of the invention, comprising (a) contacting the cerebral organoid with the test compound, and (b) determining whether the passes the blood brain barrier (BBB) cells in the cerebral organoid.

The definitions and preferred embodiments of the first and second aspect of the invention as described herein above apply *mutatis mutandis* to the fourth aspect of the invention a far as they are amenable for combination with the fourth aspect.

miR20b (microRNA 20b) is an RNA Gene, and is affiliated with the miRNA class. Diseases know to be associated with miR20b include T-cell lymphoblastic leukemia/lymphoma and multiple sclerosis. Among its related pathways are Parkinsons Disease Pathway. The sequence of miR20b is preferably the sequence of human miR20b-5p.

The human miR20b-5p preferably comprises or consists of SEQ ID NO: 5 and optionally further SEQ ID NO: 6. The miR-20b-5p sequence of SEQ ID NO: 5 (RNA sequence) can be found in the miRNA database "miR-base" and SEQ ID NO: 6 is the complementary sequence (DNA sequence) thereof. SEQ ID NO: 5 and SEQ ID NO: 6 can hybridize to each other and preferably form a hair-pin structure. Instead SEQ ID NO: 6 also the corresponding RNA sequence can be used. The human miR20b-5p more preferably comprises or consists of SEQ ID NO: 7. In addition to SEQ ID NOs 5 and 6 SEQ ID NO: 7 comprises a Xho1 restriction site and an E.CoRI sequence which can be used in cloning the sequence in to a plasmid, such as the LT3GEPIR plasmid as used in the appended examples.

The BBB is a highly selective semipermeable border of endothelial cells that prevents solutes in the circulating blood from non-selectively crossing into the extracellular fluid of the central nervous system where neurons reside. The BBB is formed by endothelial cells of the capillary wall, astrocyte end-feet (i.e. button-like terminals of axons that make synaptic connections with other nerve-cells) ensheathing the capillary, and pericytes embedded in the capillary basement membrane.

The recombinant expression of miR20b is preferably achieved by modifying the primate stem cells, so that they harbour an expression vector wherein the miR20b gene is under the control of an inducible promoter. Preferably the expression vector is a tetracycline-controlled Tet-Off and Tet-On miR20b gene expression system. The expression vector for the miR20b gene is preferably the LT3GEPIR plasmid (commercially available from Addgene). If this is desired, the expression can be stopped at a certain point in time by tetracycline or doxycycline in a Tet-off system or *vice versa* in a Tet-on system.

The at least until about day 50 is with increasing preference at least until about day 60, at least until about day 70 and at least until about day 80.

After 50 days the cerebral organoids as obtained by the method of the fourth aspect form a choroid plexus-like structure that at least predominantly consists of BBB cells. For this reason the BBB cells are also referred to in the examples as choroid plexus. The choroid plexus is a plexus of cells that arises from the tela choroidea in each of the ventricles of the brain and a plexus is generally a branching network of vessels or nerves. In view of thus choroid plexus-like structure it can be tested whether a test compound is capable of passing blood brain barrier (BBB) cells in a cerebral organoid as obtained by the method of the fourth aspect of the invention.

The nature of test compound is not particularly limited and can be, for example, a nucleic molecule (e.g. an mRNA vaccine or antisense molecule (siRNA, shRNA, Gapmer, etc.), a peptide or protein (e.g. an antibody or enzyme), or a small organic molecule (preferably having a molecular weight of below 1kDa).

The test compound can be out into the choroid plexus-like structure (e.g. by microinjection) and it can be determined whether test compound goes out or the test compound can be placed outside the choroid plexus-like structure (e.g. in a suitable culture medium) and it can be determined whether test compound goes in.

Means and methods for monitoring the test compound are known in the art. The test compound, can for example, be labelled by a fluorescent dye or by a radioactive isotope.

The BBB cells can be identified in cerebral organoids by expression markers and, for example, by the expression of TTR (transthyretin) (Marchi et al. (2003), J Neurosci; 23(5):1949-55). It is shown in the examples that in the claimed method the expression of miR20b in early neural stem cells converts cortical organoids towards cortical organoids displaying choroid plexus structures and in particular a high number of BBB cells. The overexpressing hsa-miR-20b-5p in the claimed method revealed that this miRNA is involved in the stepwise specification of choroid plexus of archicortex, by caudalizing the early cortical nueral stem cells (NSC), due to modulation of WNT and BMP signalling components. In addition, cellular immunostainings displayed a characteristic presence of giant but thin single-layer vesicles positive for choroid plexus markers in miR-20b overexpressing organoids, as opposed to pseudostratified cortical neural rosettes in wild-type organoids.

The present invention relates in a fifth aspect to a method for the generation of hippocampal cells in cerebral organoids comprising (a) culturing primate stem cells in a primate stem cell medium until about day 6, thereby inducing the formation of embryonic bodies; (b) culturing the embryonic bodies as obtained in step (a) in a neural induction medium until about day 11, thereby inducing the formation of organoids; wherein an inhibitor of TGF-β, an inhibitor of BMP and an inhibitor of WNT is present from about day 2 until about day 11; and wherein miR20b and CCND1 are recombinantly expressed, preferably from about day 2 onward; (c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until about day 30, thereby obtaining cerebral organoids with hippocampal cells, wherein the organoids in step (c) are subjected to agitation from about day 15 onward, preferably by using an orbital shaker or a spinning bioreactor; and (d) optionally isolating one or more hippocampal cells.

In this connection the present invention also relates to a method for determining whether a test compound is an inhibitor or activator of the generation of hippocampal cells comprising, (a) culturing primate stem cells in a primate stem cell medium until about day 6, thereby inducing the formation of embryonic bodies; (b) culturing the embryonic bodies as obtained in step (a) in a neural induction medium until about day 11, thereby inducing the formation of organoids; wherein an inhibitor of TGF-β, an inhibitor of BMP and an inhibitor of WNT is present from about day 2 until about day 11; and wherein miR20b and CCND1 are recombinantly expressed, preferably from about day 2 onward; and (c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until about day 30, thereby obtaining cerebral organoids with hippocampal cells, wherein the organoids in step (c) are subjected to agitation from about day 15 onward, preferably by using an orbital shaker or a spinning bioreactor, wherein the test compound is an inhibitor of the generation of hippocampal cells if the number of hippocampal cells in the organoids is reduced as compared to the absence of the test compound, and wherein the test compound is an activator of the generation of hippocampal cells if the number of hippocampal cells in the organoids is increased as compared to the absence of the test compound.

The definitions and preferred embodiments of the first and second aspect of the invention as described herein above apply *mutatis mutandis* to the fifth aspect of the invention a far as they are amenable for combination with the fifth aspect.

The CCND1 gene encodes the cyclin D1 protein. Cyclin D1 belongs to the highly conserved cyclin family, whose members are characterized by a dramatic periodicity in protein abundance throughout the cell cycle. Cyclins function as regulators of CDKs (Cyclin-dependent kinase). CCND1 is the main target of miR-20b.

The hippocampus is a major component of the brain of humans and other vertebrates. Humans and other mammals have two hippocampi, one in each side of the brain. The hippocampus is part of the limbic system, and plays important roles in the consolidation of information from short-term memory to long-term memory, and in spatial memory that enables navigation. The hippocampus is located in the allocortex, with neural projections into the neocortex in humans, as well as primates.

The recombinant expression of miR20b and CCND1 is preferably achieved by modifying the primate stem cells so that they harbour one and preferably two expression vectors, wherein the miR20b gene and the CCND1 gene are under the control of inducible promoters. Preferably the expression vector(s) is/are tetracycline-controlled Tet-Off and Tet-On miR20b gene expression system(s). The expression vector for the miR20b gene is preferably the LT3GEPIR plasmid and the expression vector for the CCND1 gene is preferably a lentiviral vector. If this is desired, the expression of miR20b and/or CCND1 can be stopped at a certain point in time by tetracycline or doxycycline in a Tet-off system or vice versa in a Tet-on system.

The at least until about day 30 is with increasing preference at least until about day 40, at least until about day 50 and at least until about day 60.

The hippocampal cells can be identified in cerebral organoids by expression markers and, for example, by the expression of LMX1A (LIM Homeobox Transcription Factor 1 Alpha) (Grandi et al. (2020), Front. Mol. Neurosci., https://doi.org/10.3389/fnmol.2020.00083) as well as RSPO3 (R-Spondin 3) and OTX2 (Orthodenticle Homeobox 2).

It is shown in the examples herein below that in the claimed method the recombinant co-expression of miR20b and CCND1 in early neural stem cells converts cortical organoids towards cortical organoids with a high number of hippocampal cells. Hence, it was found that co-overexpression of miR-20b with its main target CCND1, leads to the generation of cortical hem/hippocampus organoids.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.

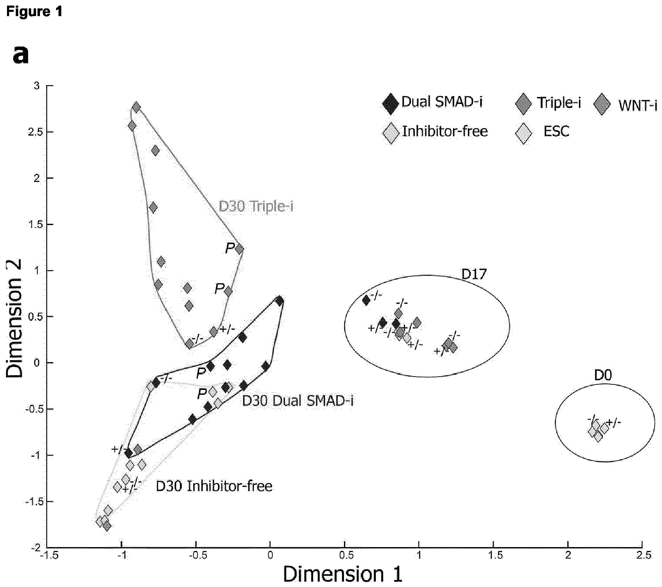

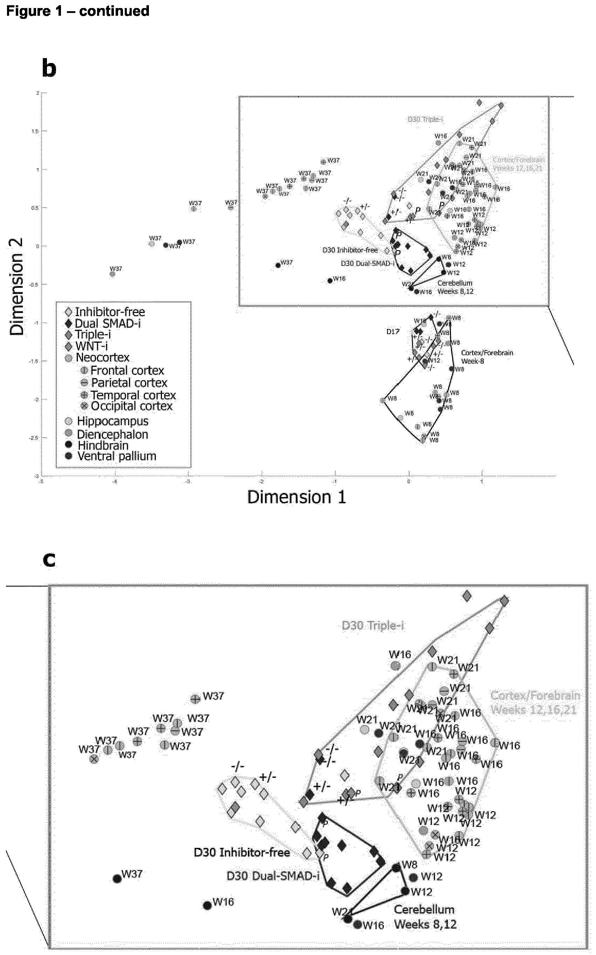

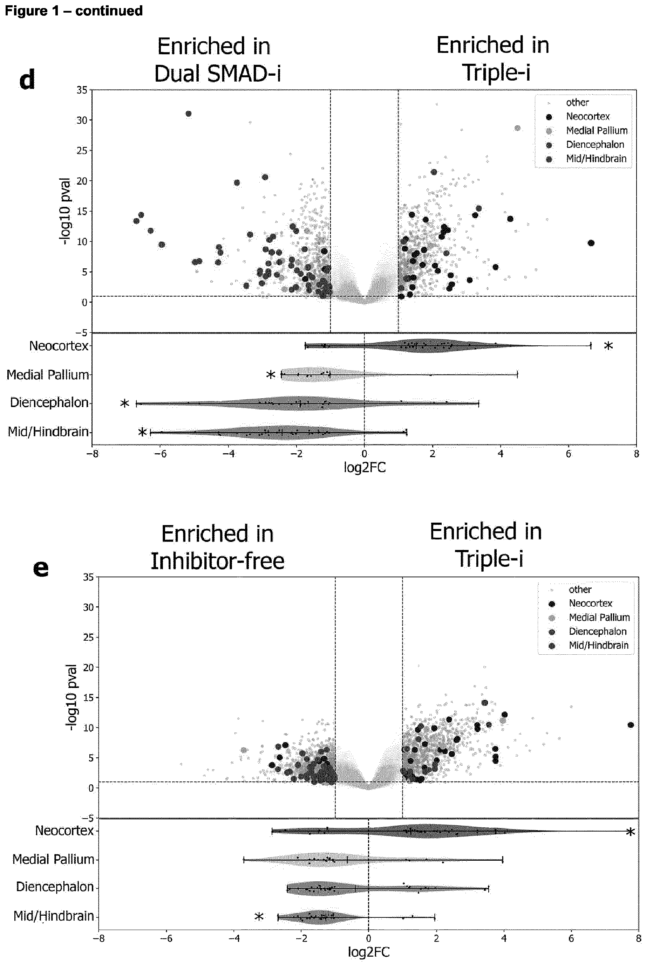

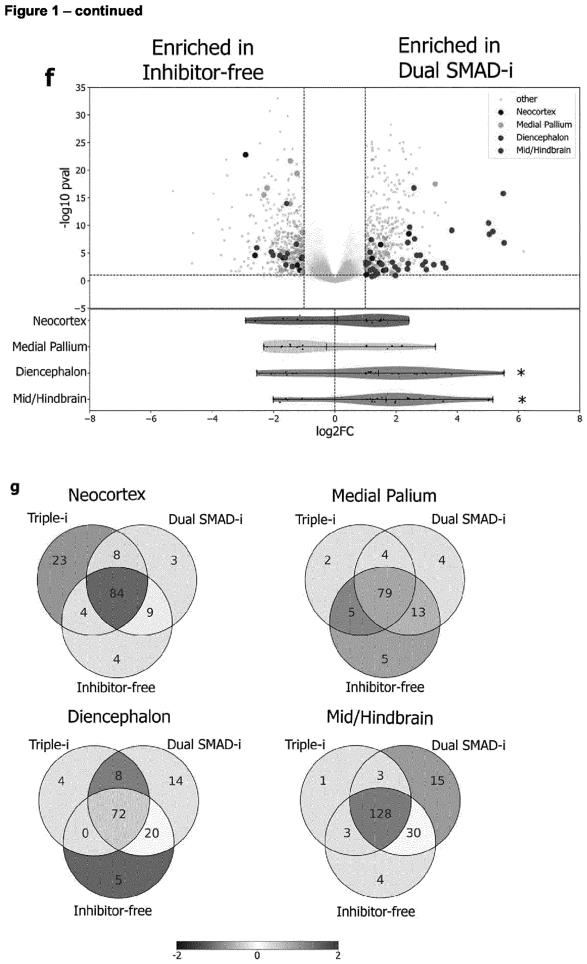

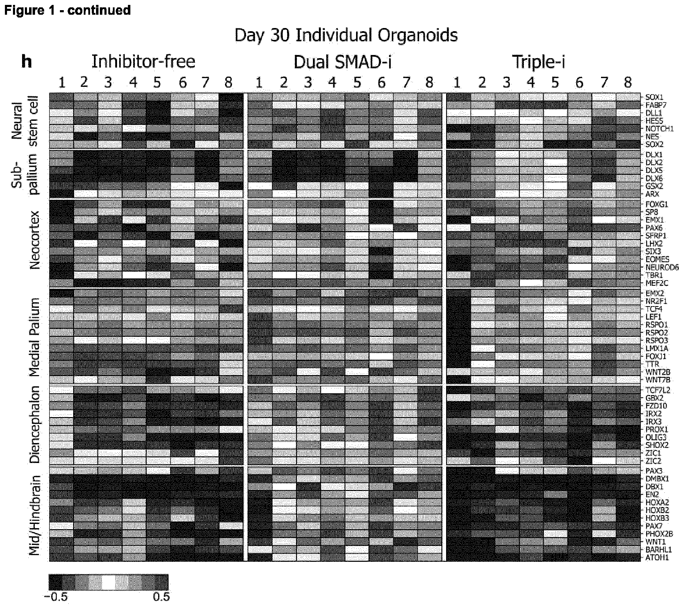

### Figure 1: Combined global gene expression analysis of H9 hESC-derived organoids and human brain samples reveal distinct brain region specification by various protocols

**a.** Correspondence analysis (CA) plot of RNA-Seq datasets obtained from undifferentiated PSCs (D0), day 17 organoids (D17) and day 30 organoids (D30) derived by indicated treatments from H9 ESC line. Transcriptome profiles were obtained from single, individually processed D30 organoids (N=8 for each protocol collected in groups of four organoids per protocol in 2 batches; N=2 WNT-i derived day 30 organoids collected in 2 batches), pool-processed D17 organoids (4 organoids pooled) and pool-processed D30 organoids (3 organoids pooled). See legend for color-coding. *P*: Pooled organoids. -/- and +/-: homozygous and heterozygous microcephaly mutant organoids. These microcephaly mutant organoids will be described in a later section of the paper.
**b.** CA plots calculated for RNA-Seq datasets obtained from **(a)** and then integrated with human brain region specific RNA-Seq datasets (Allen Human Brain Atlas: http://human.brain-map.org/). Symbols for organoid samples appear as in **(a).** Brain region-specific samples appear as circles (see legend for color-coding). See methods for further sub-divisions according to Šestan and colleagues³².
**c.** Large scale cluster distribution of **(b).**
**d.** Volcano plot of day 30 Triple-i organoids vs Dual SMAD-i organoids. Log2 fold changes and adjusted p-values are estimated from DESeq2. Significant DE region-specific Allen Brain genes are highlighted and log2 fold changes plotted in the violin plot. A star to the right or left of the violin plot indicates significantly up- or down-regulated gene set measured by GSEA and Benjamini-Hochberg correction for multiple hypothesis testing.
**e.** Volcano plot of day 30 Triple-i organoids vs Inhibitor-free organoids. See **(d)** for plot description.
**f.** Volcano plot of day 30 Dual SMAD-i organoids vs Inhibitor-free organoids. See **(d)** for plot description.
**g.** Venn diagrams of protocol-specific DE and non-DE genes from Allen Brain Atlas regional gene sets. Color of Venn diagram sections represents the relative expression levels of these genes compared to other protocol-specific regional genes across Allen Brain Atlas samples from weeks 12-21.
**h.** A heatmap representing expression values for selected genes categorized according to NSC markers and additional groups of regional markers in individual day 30 organoids derived under indicated treatments. Color-coded scale represents relative expression levels of each gene (row) across treatments.

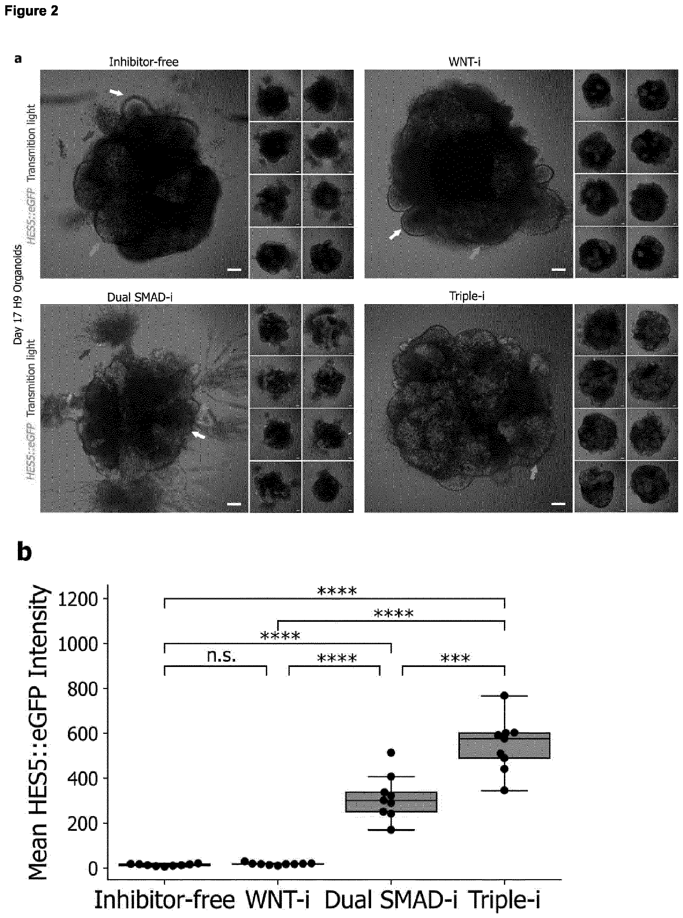

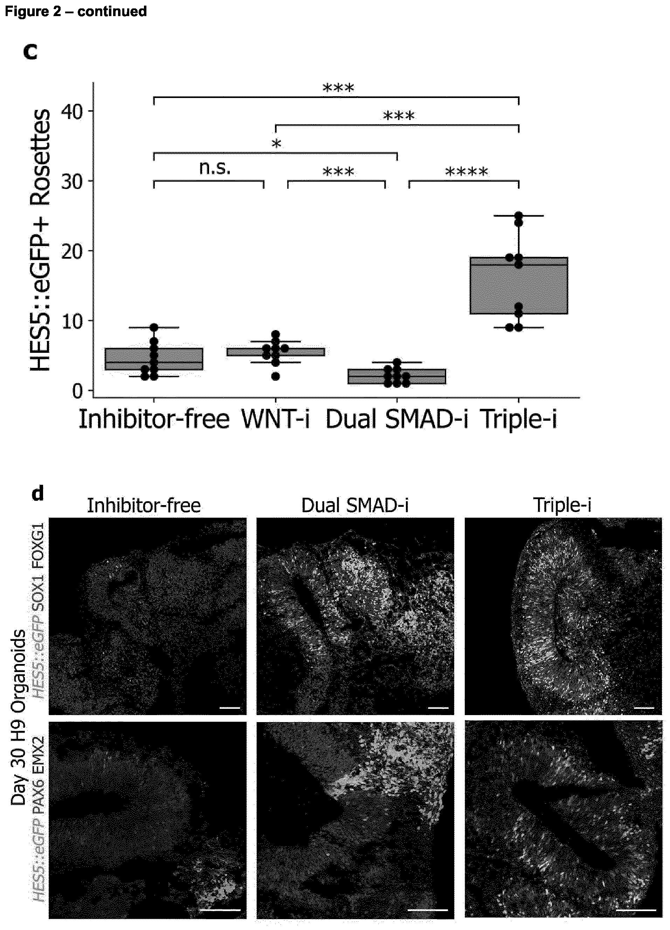

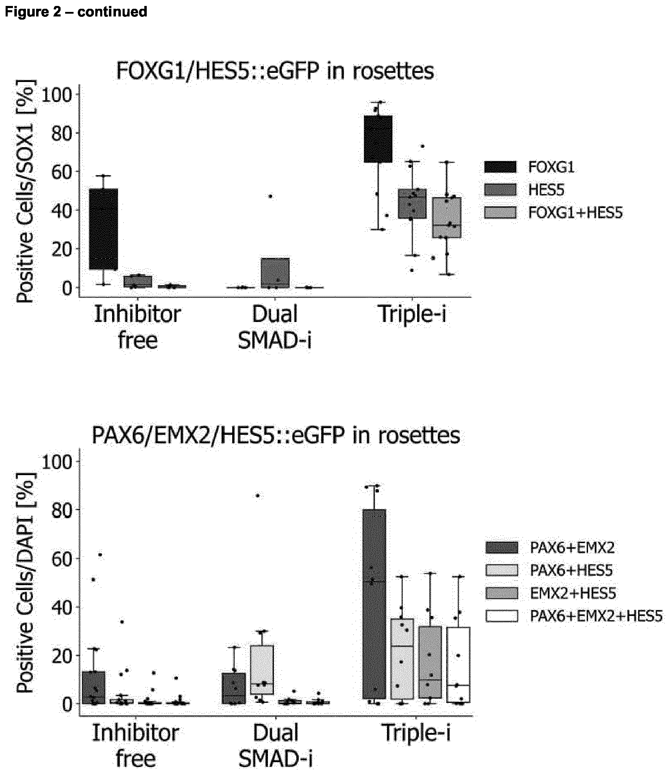

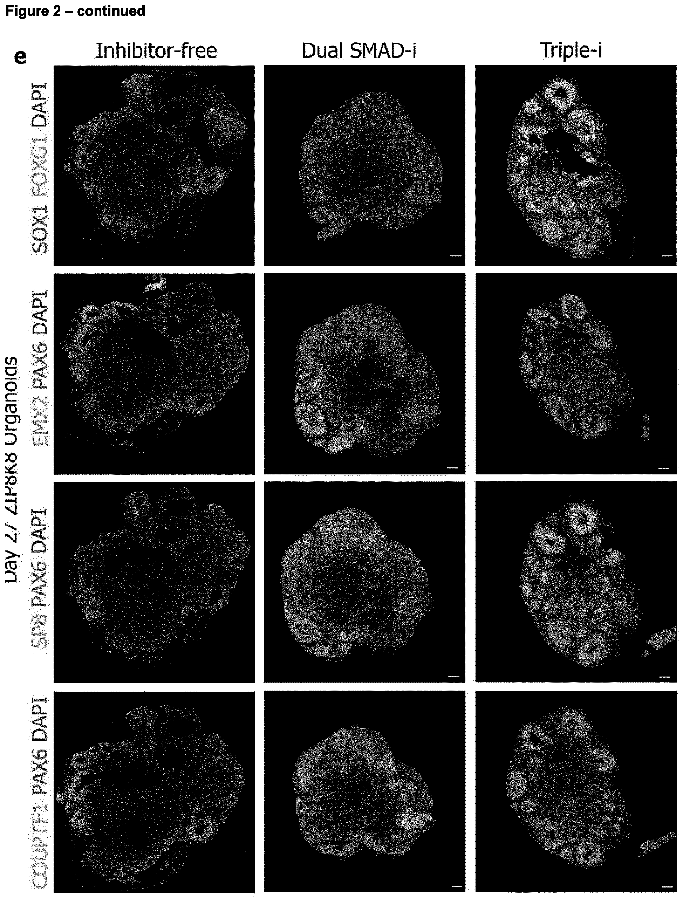

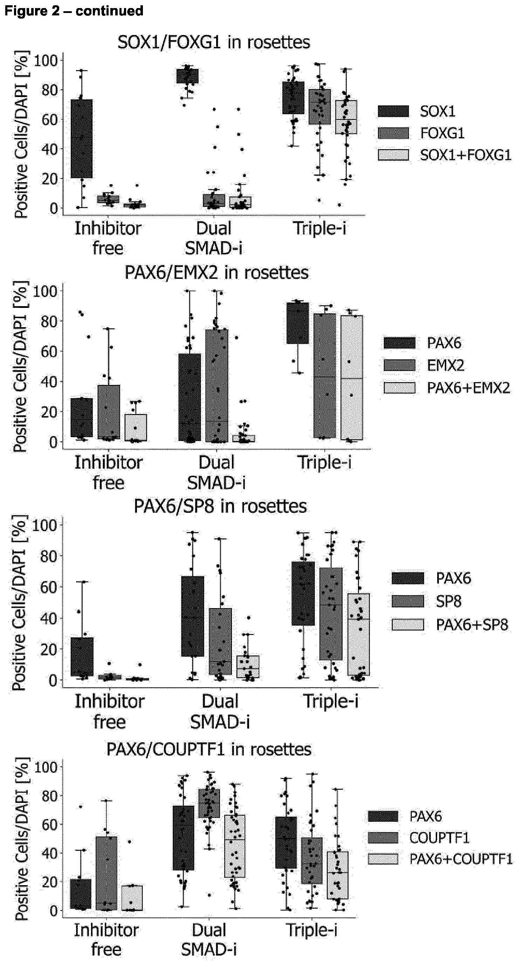

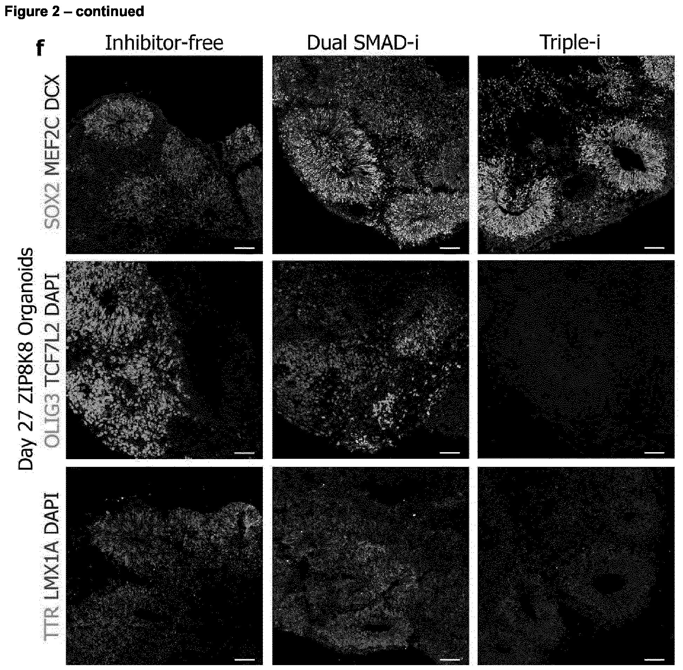

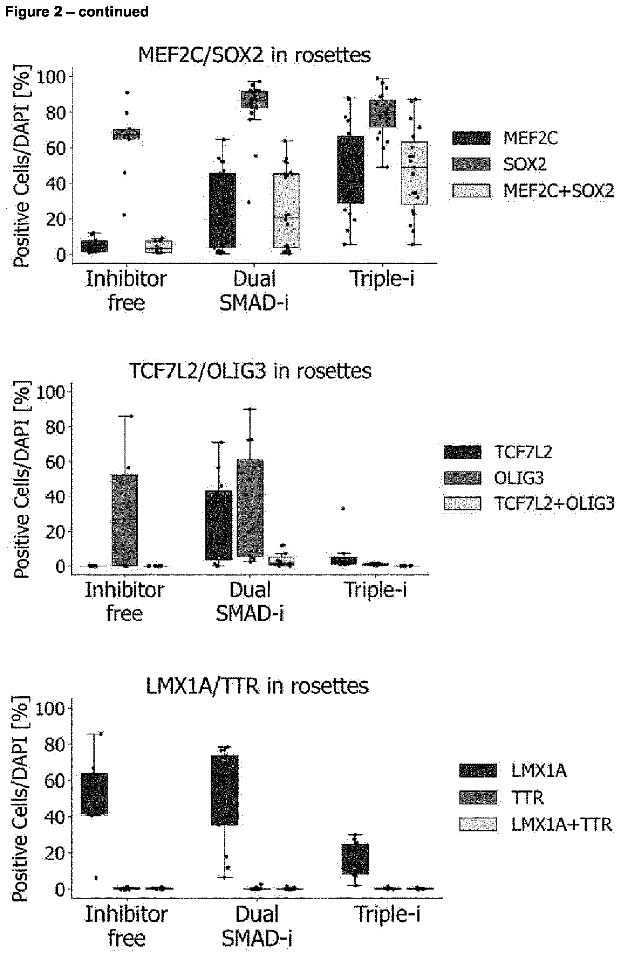

### Figure 2: Enhanced Notch activation and efficient radial organization co-localize with cortical markers in organoids derived by Triple-i

**a.** Merged bright field images and their matched H9 *HES5::eGFP* confocal images taken from day 17 representative organoids (N=9). Shown are maximum intensity projections for H9 *HES5::eGFP* confocal images taken at 15µm intervals across each. Green arrows represent enhanced Notch active and radially organized regions (vesicles), white arrows refer to radially organized regions lacking enhanced Notch activation, and red arrows refer to non-neuroepithelial extensions. Scale bar: 100 µm.
**b.** Mean *HES5::eGFP* intensity levels measured for images from **(a).** Statistical test: *two-sided t-test* with Benjamini-Hochberg multiple hypothesis correction; *P<0.05; **P<0.01 ***P<0.001; ****P<0.0001.
**c.** Number of rosettes expressing *HES5::eGFP* counted in organoid images from **(a).** Statistical test: *two-sided t-test* with Benjamini-Hochberg multiple hypothesis correction; *P<0.05; **P<0.01 ***P<0.001; ****P<0.0001.
**d.** Immunostaining images for SOX1 and FOXG1 **(top)** (Triple-i: n=13 rosettes, N=2 organoids; Dual SMAD-i: n=4 rosettes, N=1 organoid; Inhibitor-free: n=5 rosettes, N=1 organoid) and PAX6 and EMX2 **(bottom)** (Triple-i: n=10 rosettes, N=1 organoid; Dual SMAD-i: n=10 rosettes, N=1 organoid; Inhibitor-free: n=18 rosettes, N=2 organoids) with respect to Notch activation (*HES5::eGFP*) and radial organization in day 30 organoids derived from H9 hESCs by indicated treatments. Cell counts and co-localization analysis of *HES5::eGFP* expression and indicated markers within individual rosettes are presented in the right panel. Scale bar: 100 µm. Note co-expression of FOXG1 and HES5, as well as PAX6, EMX2 and HES5::eGFP was significantly higher in Triple-i.
**e.** Immunostainings of EMX2 (Triple-i: n=8 rosettes, N=1 organoid; Dual SMAD-i: n=38 rosettes, N=3 organoids; Inhibitor-free: n=14 rosettes, N=1 organoid), SP8 (Triple-i: n=35 rosettes, N=3 organoids; Dual SMAD-i: n=23 rosettes, N=3 organoids; Inhibitor-free: n=12 rosettes, N=1 organoid) and COUP-TF1 (Triple-i: n=36 rosettes, N=3 organoids; Dual SMAD-i: n=44 rosettes, N=3 organoids; Inhibitor-free: n=12 rosettes, N=1 organoid) with respect to PAX6, as well as of FOXG1 and SOX1 (Triple-i: n=40 rosettes, N=2 organoids; Dual SMAD-i: n=29 rosettes, N=2 organoids; Inhibitor-free: n=14 rosettes, N=1 organoid), in day 27 organoids derived from ZIP8K8 iPSCs by indicated treatments. Cell counts and co-localization analysis within individual rosettes are presented in the right panel. Scale bar: 100 µm. Note co-expression of FOXG1 and SOX1, PAX6 and EMX2, and PAX6 and SP8 was significantly higher in Triple-i.
**f.** Immunostainings of cortical markers MEF2C with respect to SOX2 and DCX (Triple-i: n=19 rosettes, N=2 organoids; Dual SMAD-i: n=22 rosettes, N=2 organoids; Inhibitor-free: n=9 rosettes, N=1 organoid), non-cortical markers OLIG3 and TCF7L2 (thalamic markers) (Triple-i: n=7 rosettes, N=1 organoid; Dual SMAD-i: n=7 rosettes, N=1 organoid; Inhibitor-free: n=11 rosettes, N=1 organoid) as well as TTR and LMX1A (medial pallium markers) (Triple-i: n=10 rosettes, N=1 organoid; Dual SMAD-i: n=13 rosettes, N=1 organoid; Inhibitor-free: n=9 rosettes, N=1 organoid) in day 27 organoids derived from ZIP8K8 iPSCs by indicated treatments, alongside cell counts and colocalization analysis within individual rosettes. Scale bar: 100 µm. Note co-expression of SOX2 and MEF2C was significantly higher in Triple-i, and expression of LMX1A was significantly lower in Triple-i. Statistical test: *two-sided t-test* with Benjamini-Hochberg multiple hypothesis correction within each experiment.

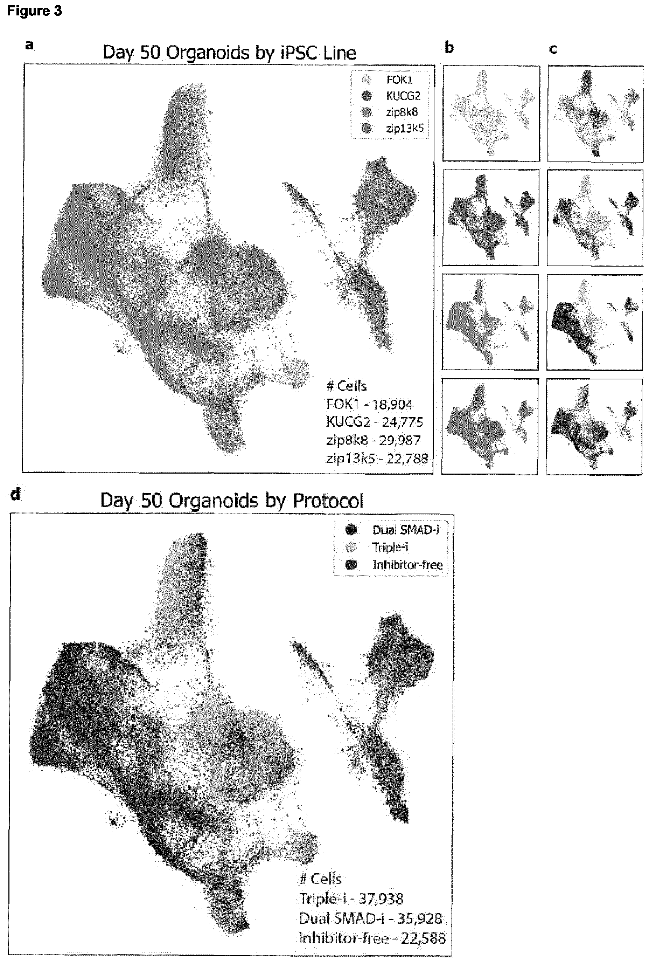

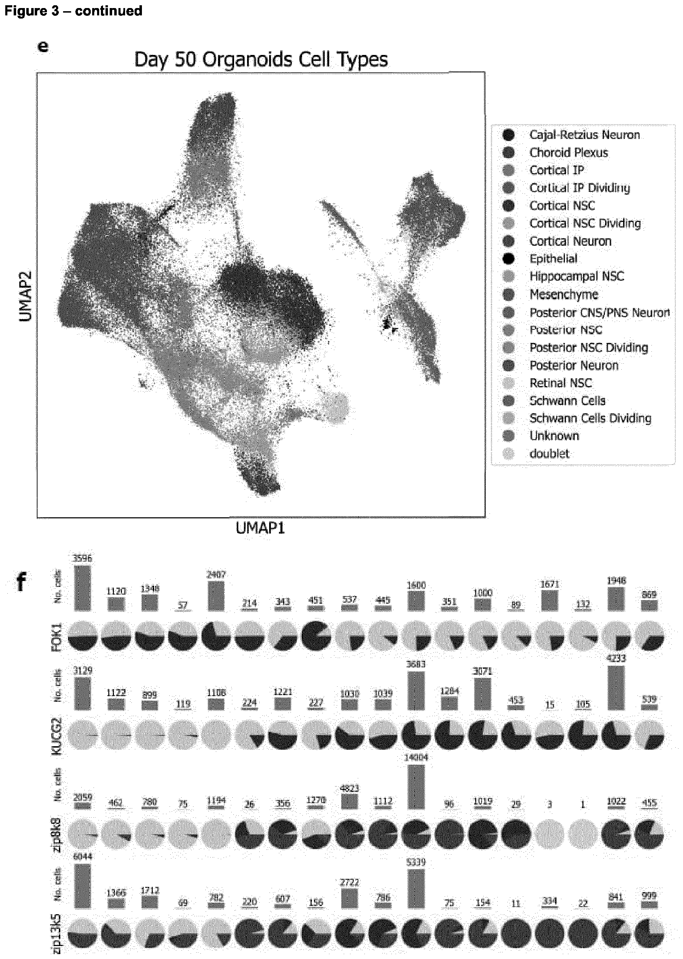

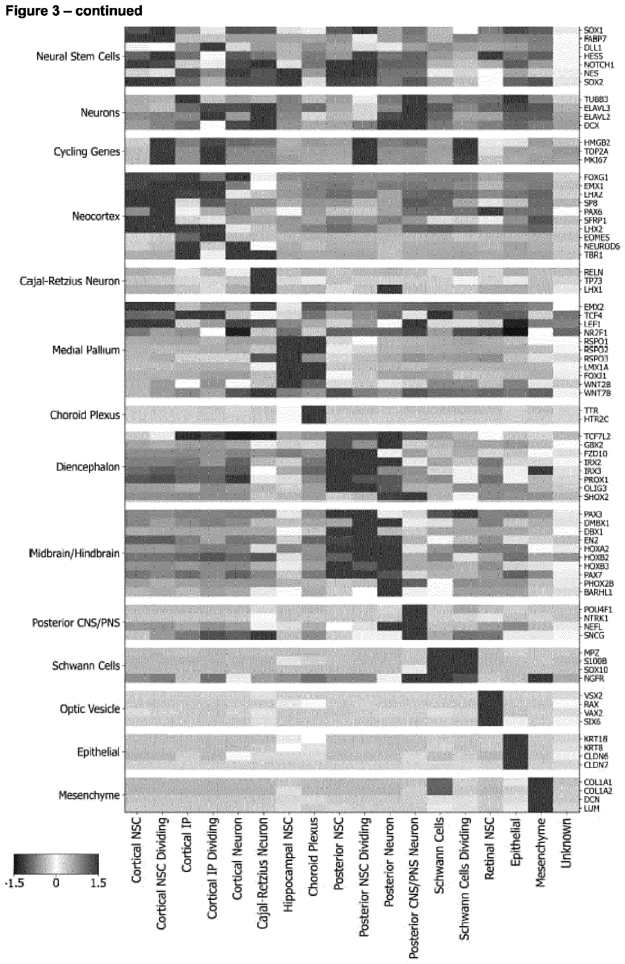

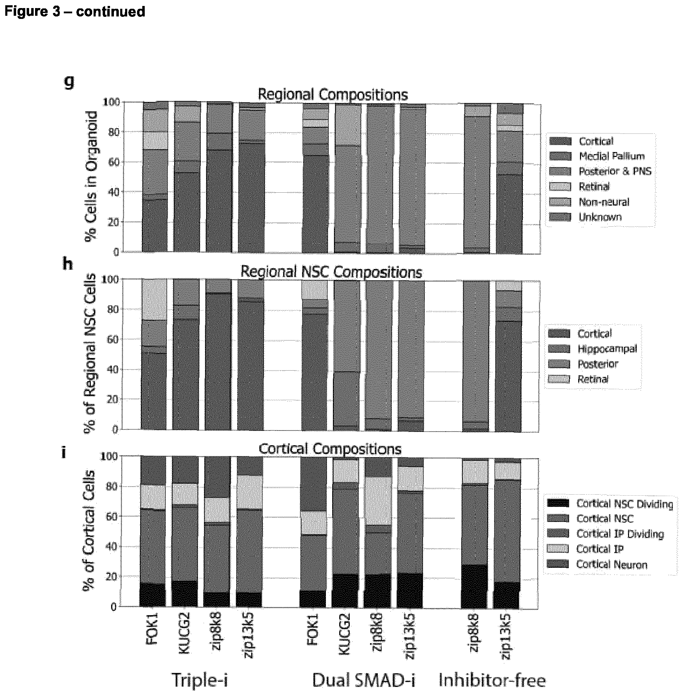

### Figure 3. Triple-i induces robust cortical identity and suppresses non-cortical fates across 4 iPSC lines

**a.** A UMAP derived from scRNA-Seq data of day 50 Triple-i, Dual SMAD-i and Inhibitor-free organoids was estimated and each cell was colored by its respective iPSC line (FOK1, KUCG2, ZIP8K8 and ZIP13K5).
**b.** Cells from individual iPSC lines were then plotted separately using the same UMAP embedding in **(a).**
**c.** Cells derived from Triple-i, Dual SMAD-i and Inhibitor-free derivation protocols were plotted using the same UMAP embedding in **(a)** separated according to iPSC line **(b).**
**d.** All day 50 organoid cells were plotted in the same UMAP embedding colored by derivation protocol.
**e.** Regional and cell type annotations were then assigned to individual clusters (see Methods) and are plotted on the same UMAP embedding.
**f.** Heatmap displays relative expression values after z-score normalization of average log-normalized expression values for each gene across annotated cell types for selected genes categorized according to neural stem cell, neuronal, cycling, regional, and other cell type markers. Pie charts above each cell type display percentage of cells from each derivation protocol across all 4 iPSC lines. Pie charts are colored with a gray color if less than 10 cells from that iPSC line were assigned to the given cell type. Bar plots display the total number of cells within each iPSC line assigned to the given cell type.
**g.** Stacked bar plot shows the regional composition of all cells separated by cell line and derivation protocol.
**h.** Stacked bar plot shows the regional composition of all neural stem cells separated by cell line and derivation protocol.
**i.** Stacked bar plot shows the composition of all cortical cells separated by cell line and derivation protocol.

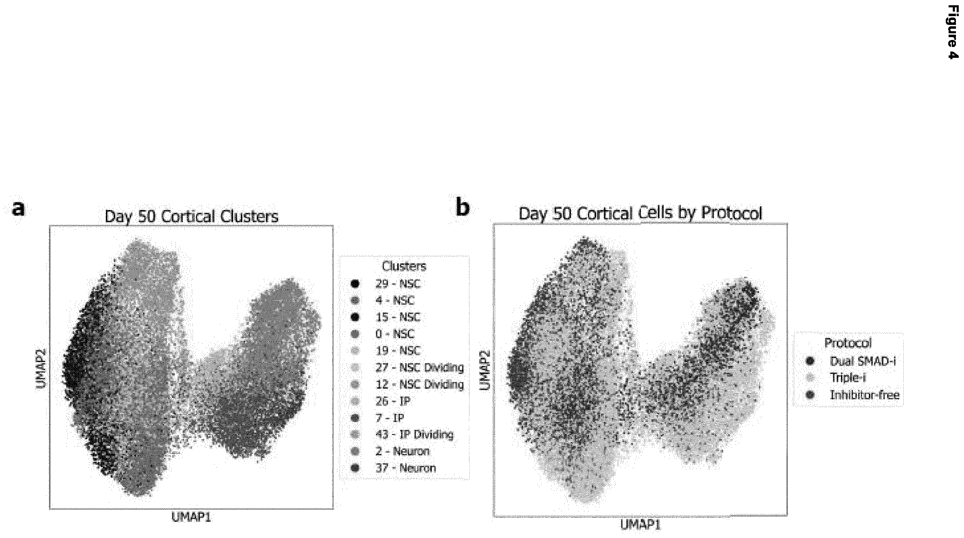

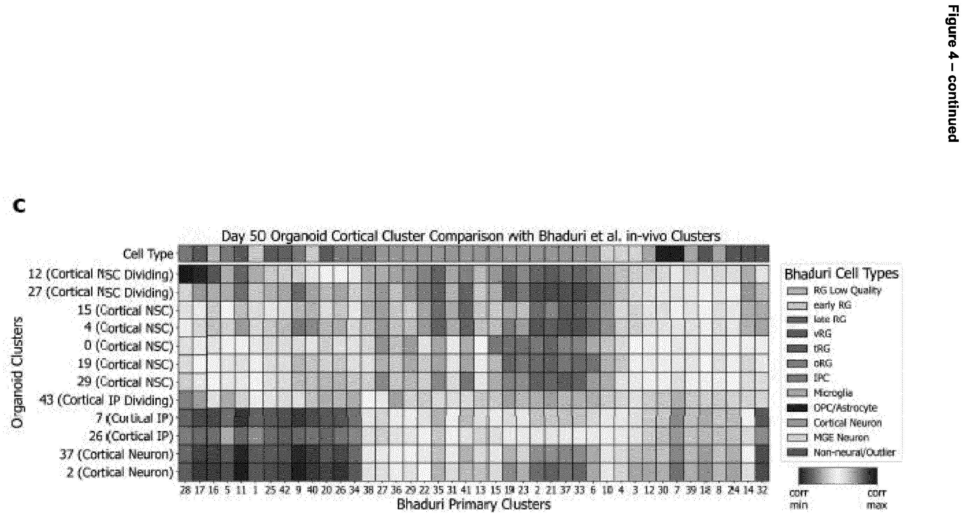

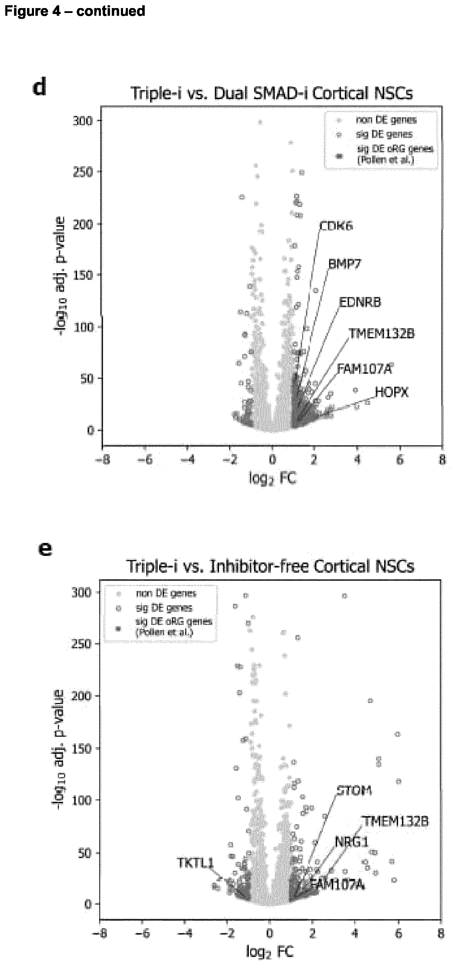

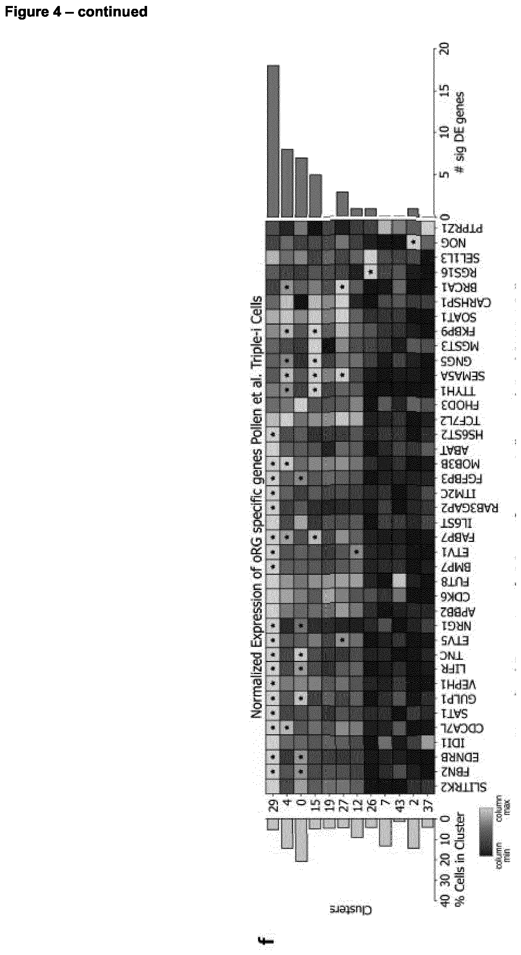

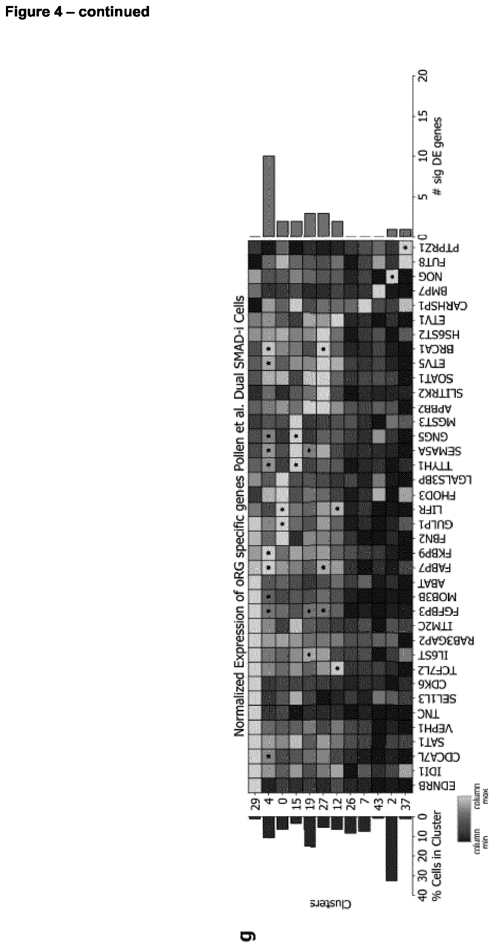

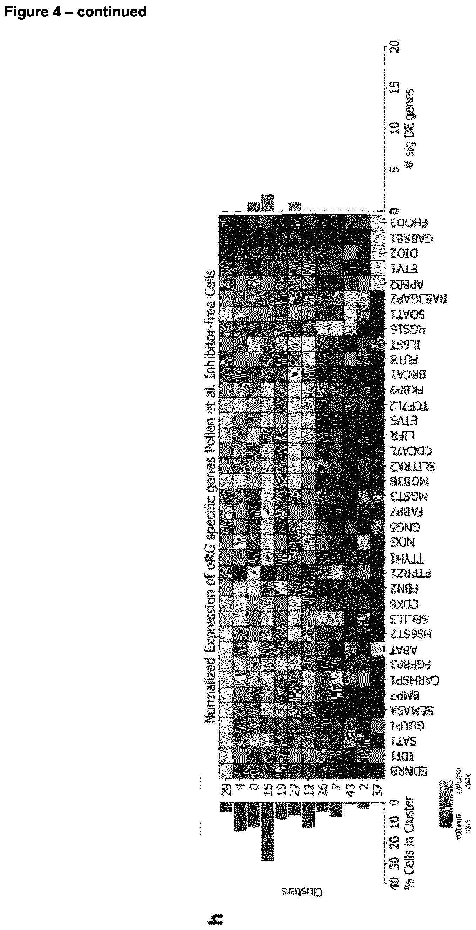

### Figure 4. Triple-i organoids specifically enrich for oRG cells across 4 iPSC cell lines

**a.** A UMAP of all cortical cells derived from scRNA-Seq data of day 50 Triple-i, Dual SMAD-i and Inhibitor-free organoids derived from FOK1, KUCG2, ZIP8K8 and ZIP13K5 cell lines was estimated and each cell is colored by its respective cluster. Annotated cell types for each cluster are written in the legend.
**b.** Cells derived from Triple-i, Dual SMAD-i and Inhibitor-free derivation protocols were plotted using the same UMAP embedding in **(a).**
**c.** Pearson correlation coefficients measured using average normalized expression levels of highly variable genes across all cells within a cluster were estimated between cortical clusters found in this study and in-vivo clusters found in Bhaduri et al. study²⁹ and are shown in the heatmap.
**d.** The volcano plot shows the results of a differential gene expression analysis comparing all cortical NSCs from Triple-i organoids and all cortical NSCs from Dual SMAD-i organoids (see Methods). Significantly differentially expressed genes are highlighted in black with oRG specific marker genes derived from Pollen et al.³⁹ annotated and highlighted in blue. The p-value 0.0594 of a gene set enrichment analysis using a Fisher's exact test of the set of oRG genes within up-regulated genes in Triple-i cortical NSCs is highlighted.
**e.** The volcano plot shows the results of a differential gene expression analysis comparing all cortical NSCs from Triple-i organoids and all cortical NSCs from Inhibitor-free organoids, as in **(b),** with p-value of oRG gene set enrichment in Triple-i cortical NSCs measured to be 0.0209.
**f.** The heatmap shows the relative expression levels of all oRG specific markers across cortical clusters within Triple-i organoids. Significantly up-regulated genes within a cluster are highlighted with a *. The left bar plot shows the relative percent of cortical cells from Triple-i organoids within a given cluster. The right bar plot shows the number of significantly up-regulated genes in a given cluster.
**g.** The heatmaps shows the same as in **(d)** after subsetting to cortical cells from Dual SMAD-i organoids.
**h.** The heatmaps shows the same as in **(d)** after subsetting to cortical cells from Inhibitor-free organoids.

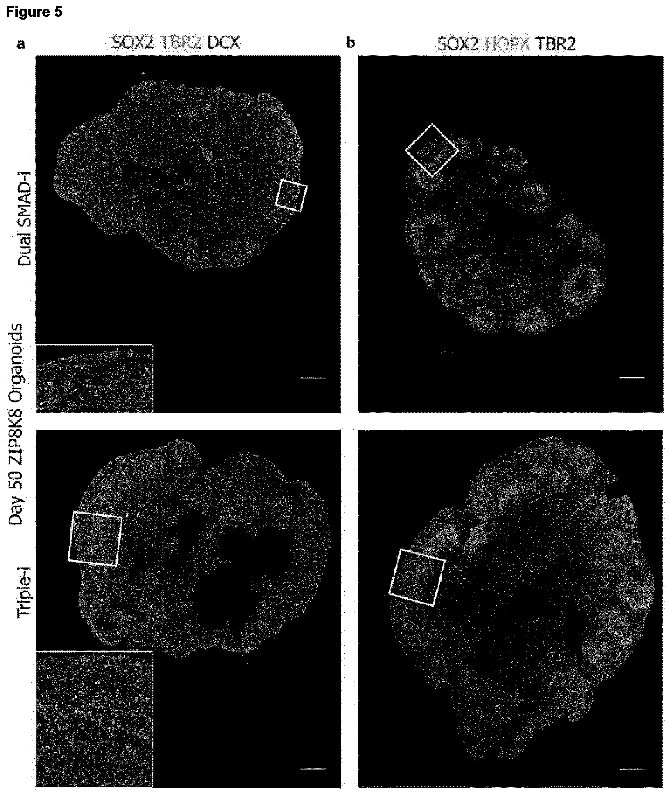

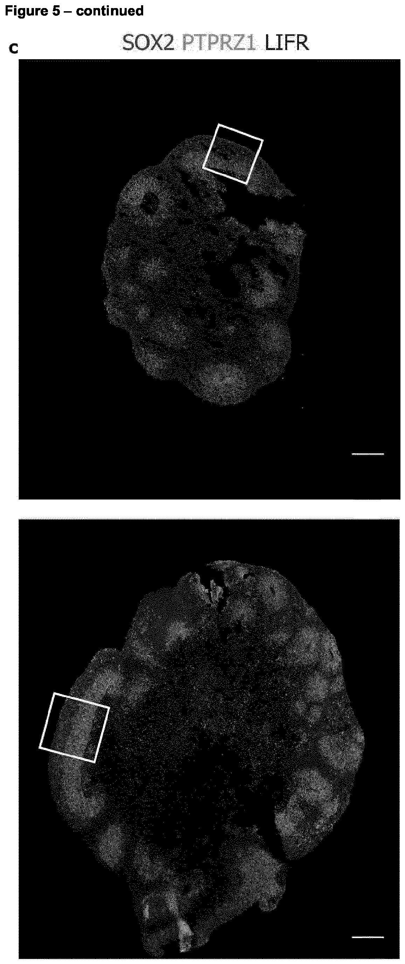

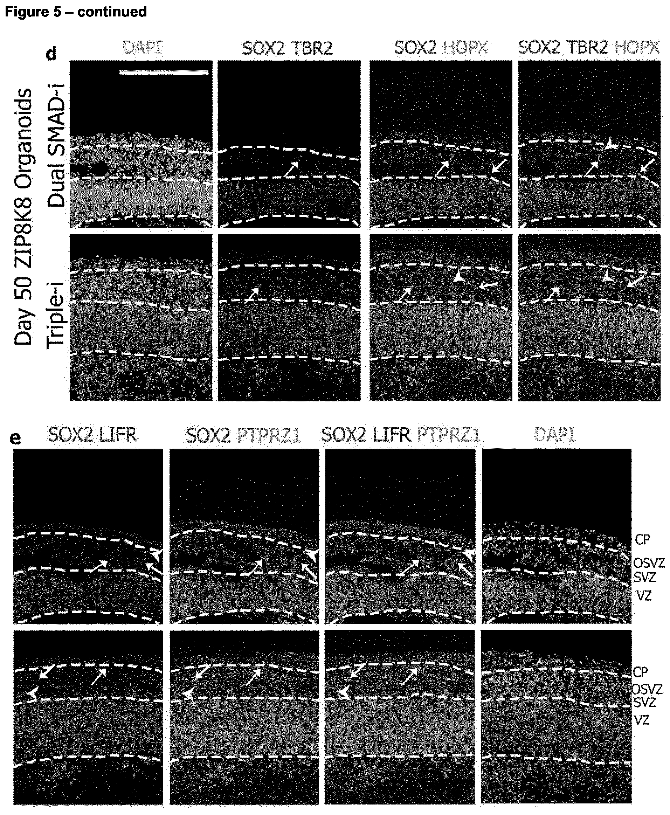

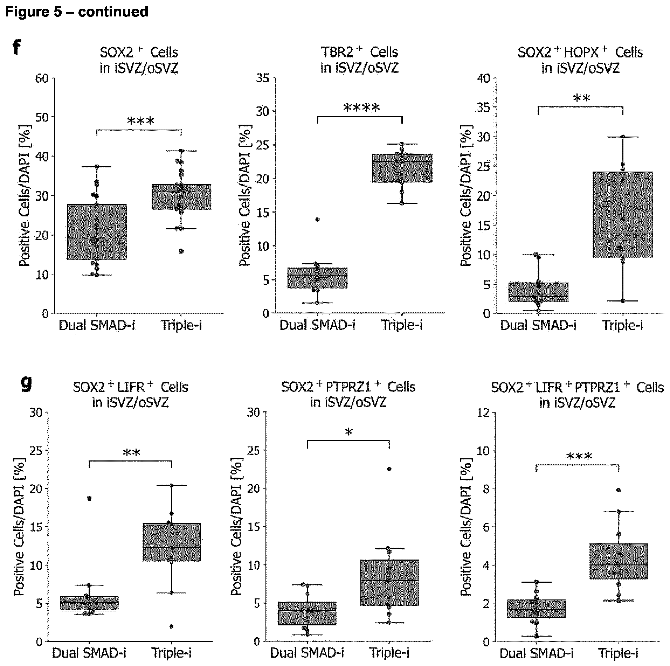

### Figure 5: oRG cells demarcate oSVZ regions in Triple-i organoids

**a.** Immunostaining for SOX2, TBR2 and DCX, in day 50 organoids derived from ZIP8K8 iPSCs by indicated treatments. The inset represents a zoom-in region from the full organoid vesicle from respective treatment. Scale bar: 200 µm. Note the presence of a thicker TBR2+ region outside of the VZ in Triple-i organoids demarcating the iSVZ.
**b.** Immunostainings for iSVZ/oSVZ markers TBR2, HOPX, along with SOX2 in day 50 ZIP8K8 iPSC organoids derived under indicated treatments. Scale bar: 200 µm.
**c.** Immunostainings for iSVZ/oSVZ markers LIFR, PTPRZ1 along with SOX2 in day 50 ZIP8K8 iPSC organoids derived under indicated treatments. Note the presence of strong PTPRZ1+ area demarcating the oSVZ specifically in Triple-i organoids. Scale bar: 200 µm.
**d.** Immunostainings of the zoom-in section for the insets from **(b)** for TBR2, HOPX with respect to SOX2 under indicated treatment. Arrows represent tracking of specific cells with co-expression of different markers. Scale bar: 200 µm.
**e.** Immunostainings of the zoom-in section for the insets from **(c)** for LIFR and PTPRZ1 with respect to SOX2 under indicated treatment. Arrows represent tracking of specific cells with co-expression of different markers. Scale bar: 200 µm.
**f.** Immunostainings cell counts and co-localization analysis in iSVZ/oSVZ regions for for four iPSC lines {ZIP8K8 (n=11 vesicles, N=2 organoids); FOK1, ZIP13K5, KUCG2 (n=5 vesicles, N=2 organoids)}, Note the higher proportion of TBR2+ and SOX2+HOPX+ in Triple-i organoids as compared to Dual SMAD-i organoids. Statistical test: *two-sided t-test;* **P*<0.05; ***P*<0.01 ****P*<0.001; *****P*<0.0001.
**g.** Immunostainings cell counts and co-localization analysis in iSVZ/oSVZ regions across four iPSC lines (ZIP8K8 (n=11 vesicles across N=2 organoids for both Dual SMAD-i and Triple-i); FOK1, ZIP13K5, KUCG2 (n=5 vesicles across N=2 organoids for both Dual SMAD-i and Triple-i)), Note that Triple-i organoids showed higher proportion of SOX2+LIFR+, SOX2+PTPRZ1+ and SOX2+LIFR+PTPRZ1+ cells. Statistical test: *two-sided t-test;* **P*<0.05; ***P*<0.01 ****P*<0.001; *****P*<0.0001.

### Figure 6. Later stage Triple-i organoids exhibit an enriched oRG cell population and molecularly distinct upper and deep layer neurons

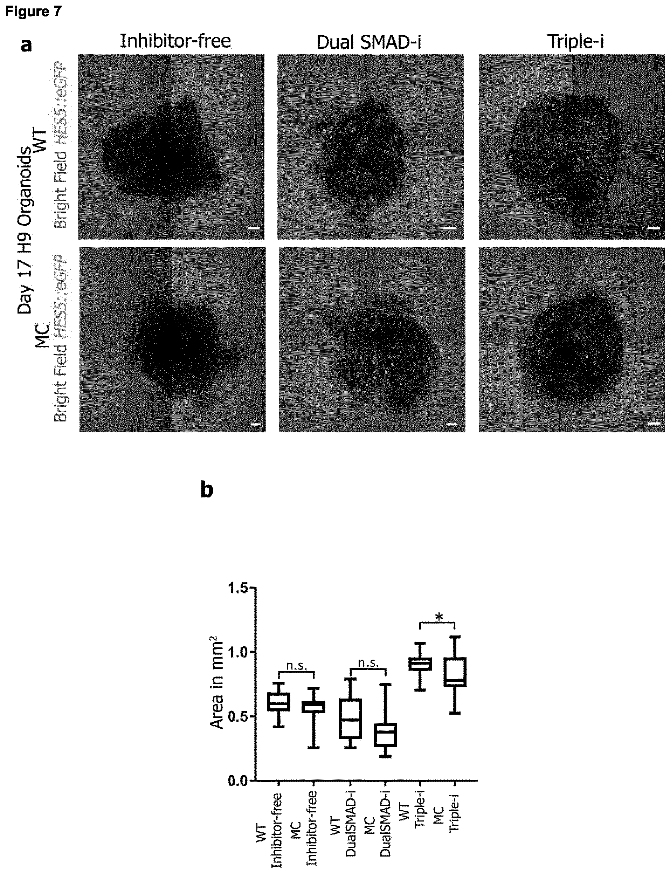

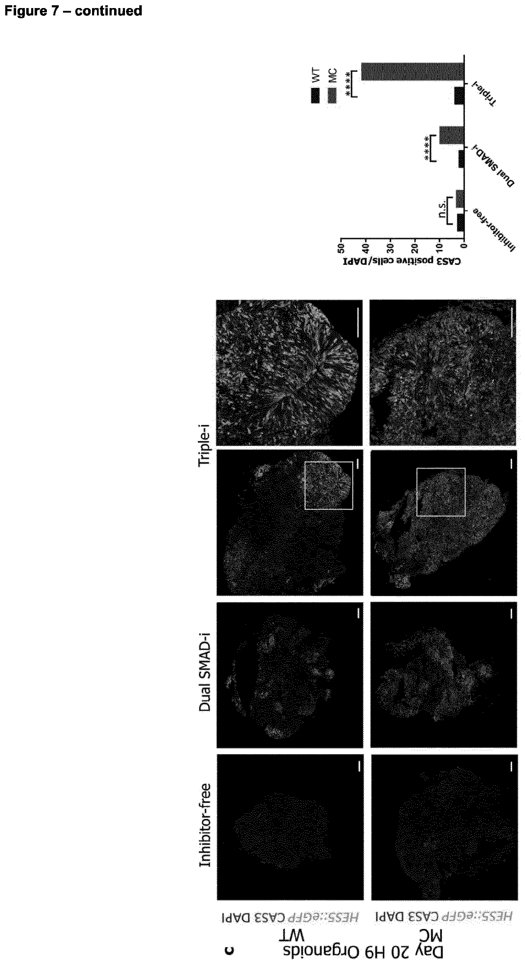

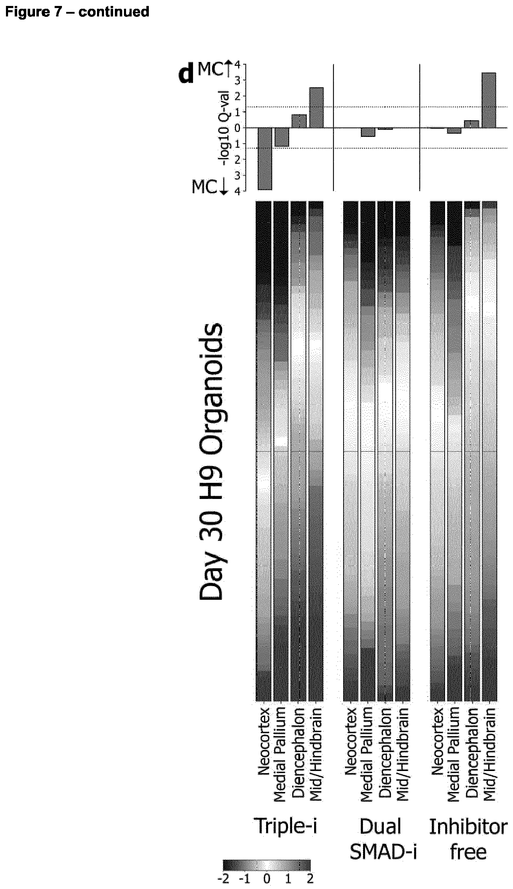

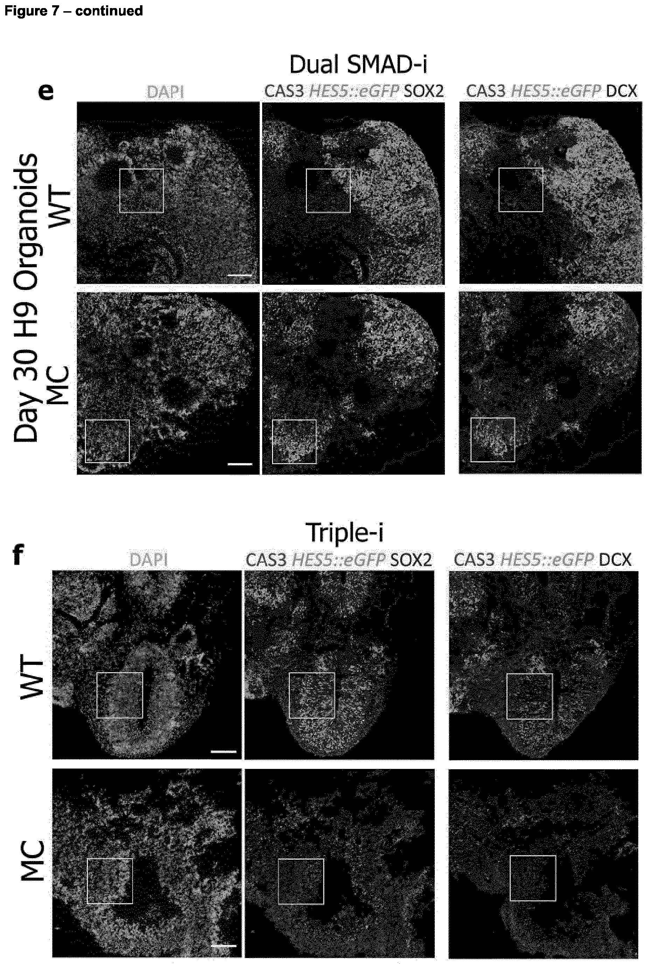

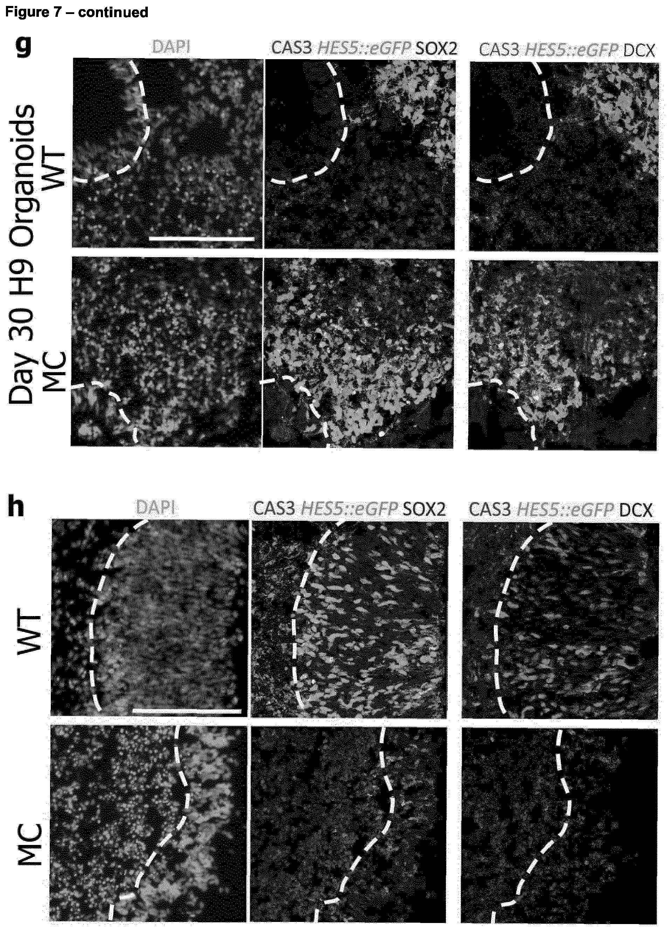

**a.** Immunostaining for stem cell marker SOX2 along with DAPI (top) in day 80 organoids derived from ZIP8K8 iPSCs. Insets (bottom) represent a zoom-in region from the full organoid vesicle. Scale bar: 200 µm. Cortical units are encircled by a dashed line and VZ regions are encircled by a solid line.
**b.** Immunostainings for iSVZ/oSVZ markers TBR2, HOPX, and SOX2 (top) derived from ZIP8K8 iPSCs. Insets (bottom) represent a zoom-in region. Scale bar: 200 µm.
**c.** Immunostainings derived from ZIP8K8 iPSCs from same organoids as in **(b)** for oRG markers LIFR, PTPRZ1, and SOX2 (top). Insets (bottom) represent a zoom-in region. Scale bar: 200 µm. Zoom-in in Triple-i organoid shows a 10x zoom-in of selected region in bottom inset with arrows highlighting SOX2+PTPRZ1- and SOX2+PTPRZ1 + cells.
**d.** Boxplots representing VZ sizes measured in *µ*m2 in Dual SMAD-i (n=25 VZ's, N=2 organoids) and Triple-i (n=37 VZ's, N=2 organoids) organoids derived from ZIP8K8 iPSCs. Statistical test: *two-sided t-test* for VZ size; P= 3.1e-5(*****P*<0.0001) and *Levene's test* for variance, P=3.5e-4(****P*<0.001).
**e.** Boxplots representing number of SOX2+ cells per DAPI within cortical units inside iSVZ/oSVZ regions in Dual SMAD-i (n=27 across N=2 organoids) and Triple-i (n=44 across N=2 organoids) organoids. Statistical test: *two-sided t-test*; P=0.88.
**f.** Boxplots representing number of SOX2+LIFR+ cells per SOX2 in the same cortical units as in **(e).** Statistical test: *two-sided t-test;* P=2.8e-05(****P<0.0001).
**g.** Boxplots representing number of SOX2+PTPRZ1+ cells per SOX2 in the same cortical units as in **(e).** Statistical test: *two-sided t-test;* p=2.3e-05(****P<0.0001).
**h.** Schematic diagram representing the VZ and iSVZ/oSVZ areas from a single cortical unit in cryosectioned organoids. Many cortical units contained purely interspersed SOX2+ cells alongside TBR2+ IP cells, but lacked well-defined rosettes, suggesting that these units were cryosectioned through their oSVZ regions.
**i.** Immunostaining images for TBR1 and CUX1 (top), CTIP2 and SATB2 (bottom) in day 80 organoids (N=1 Triple-i; N=1 Dual SMAD-i). Insets (right) represent zoom-in regions. Boxes display the median and interquartile range, and whiskers extend to 1.5x the interquartile range. Scale bar: 200 µm. Statistical test: *two-sided t-test* for cortical units (n=21 in Dual SMAD-i, and n≥29 in Triple-i) and *two-sided Fisher's exact test* for outside cortical units; **P*<0.05;***P*<0.01;****P*<0.001;*****P*<0.0001. See **Source Data** **Fig 7** for immunostaining counts.
**j.** A UMAP derived from scRNA-Seq data of day 80 ZIP13K5 Triple-i pooled organoids (3 organoids pooled together) and ZIP8K8 individual Triple-i (N=3) and Dual SMAD-i (N=3) organoids.
**k.**Cell type annotations using the same UMAP embedding in **(b).**
**l.** Cell type compositions from each scRNA-Seq experiment plotted as a stacked bar chart.
**m.** Comparison of proportion of cortical IP and upper layer neurons in each scRNA-Seq experiment grouped by Dual SMAD-i ZIP8K8 and Triple-i ZIP8K8 and ZIP13K5 organoids. Boxes display the median and interquartile range, and whiskers extend to 1.5x the interquartile range. Statistical test: *two-sided t-test;* **P*<0.05;***P*<0.01 ;****P*<0.001;*****P*<0.0001.
**n.** Heatmap displays relative expression values after z-score normalization of average log-normalized expression values for each gene across cell types.
**o.** RNA velocity stream plot for day 80 Dual SMAD-i and Triple-i organoids along with RNA velocity estimates for CUX2. Violin plots show CUX2 RNA velocity estimates per cell type.
**p.** The volcano plot shows the results of a differential gene expression analysis comparing all cortical NSCs from Triple-i ZIP8K8 and ZIP13K5 with ZIP8K8 Dual SMAD-i organoids, with selected oRG genes highlighted in blue. Log2 fold changes and adjusted p-values from t-test with overestimation of variance after Benjamini-Hochberg correction are shown (see Methods). Statistical test for gene set enrichment for oRG genes in Triple-i cortical NSCs: *two-sided Fisher's exact test* (P=1.2e-10).
**q.** Subsetting to Canonical oRG genes described in detail in the literature and validated across multiple studies gives us a much clearer picture, with most oRG genes having highest expression in day 195 compared to day 50 and day 80 (exception for LIFR and PTPRZ1); Note: GFAP astroglia gene as well

### Figure 7: Differential phenotypic modeling of microcephaly organoids in H9 derived HES5:eGFP hESC line by diverse pathway inhibition paradigms

**a.** Merged bright field images and their matched *HES5:eGFP* confocal images obtained from day 17 representative organoids derived from WT or microcephaly (MC) mutant H9 hESCs. Shown is maximum intensity projection of *HES5::eGFP* confocal images obtained at 6µm intervals across each organoid. Scale bar: 100 µm.
**b.** Boxplots representing organoid size obtained from multiple organoids (N≥15 per treatment) determined by measuring areas of collapsed maximum intensity projection images obtained from day 17 WT and MC mutant organoids. Statistical test: *t-test;* **P*<0.05; ***P*<0.01 ****P*<0.001; *****P*<0.0001.
**c.** Combined *HES5:eGFP* expression and immunostaining images of the apoptotic marker CAS-3 in day 20 WT and MC mutant organoids. A three-fold magnification image for Triple-i derived organoids is shown on the right for one representative organoid. Scale bar: 100 µm. Right: cell counts of CAS-3 in WT and MC organoids. Statistical test: *Fisher's exact test;* **P*<0.05; ***P*<0.01 ****P*<0.001; *****P*<0.0001.
**d.** Heatmap showing log2 fold changes of regional genes derived from Allen Brain Atlas samples measured between pooled homozygous microcephaly organoids and wild-type organoids. The bar plot on the left displays the Q-values from GSEA for regional gene sets calculated from genes with absolute log2 fold change ≥ 1 after Benjamini-Hochberg correction.
**e.** Combined *HES5::eGFP* expression and immunostaining images of the apoptotic marker CAS-3 together with SOX2, and DCX from an adjacent slice in Dual SMAD-i WT and MC day 30 organoids. DAPI immunostaining corresponds to SOX2. Scale bar: 200 µm.
**f.** Combined *HES5::eGFP* expression and immunostaining images of the apoptotic marker CAS-3 together with SOX2, and DCX from an adjacent slice in Triple-i WT and MC day 30 organoids. DAPI immunostaining corresponds to SOX2. Scale bar: 200 µm.
**g.** Immunostainings for the insets from **(e)** magnified 4-fold. Dashed lines encircle VZ areas in WT and MC mutant organoids. Note the CAS staining is irrespective of radial organization and *HES5::eGFP.* Scale bar: 200 µm.
**h.** Immunostainings for the insets from **(f)** magnified 4-fold. Dashed lines encircle VZ areas in WT and MC mutant organoids. Note the fragmented DAPI staining outside the VZ in Triple-i treatment. Scale bar: 200 µm.

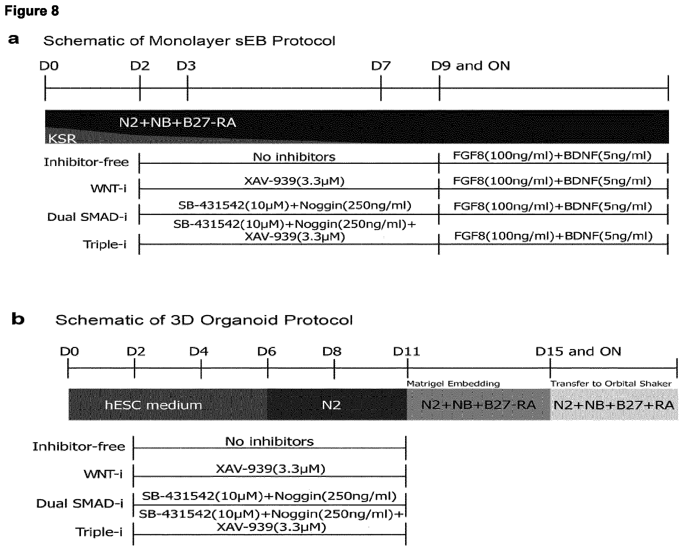

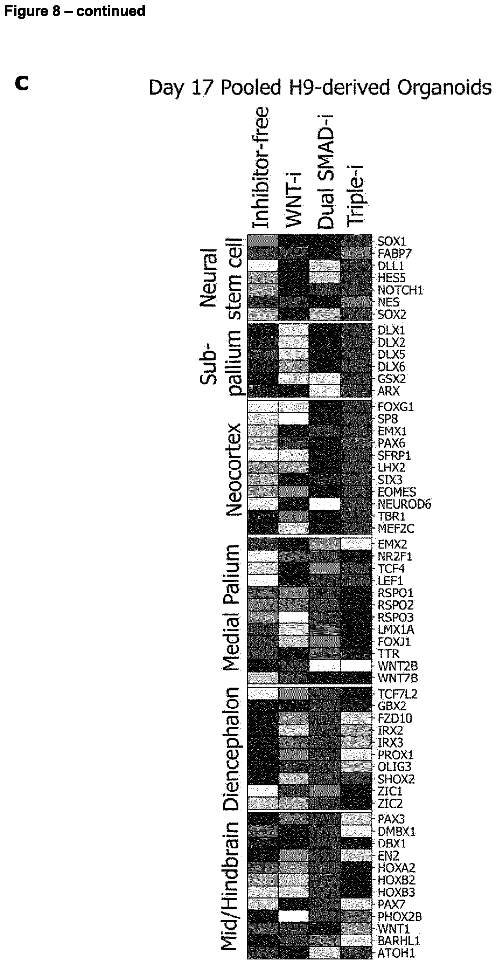

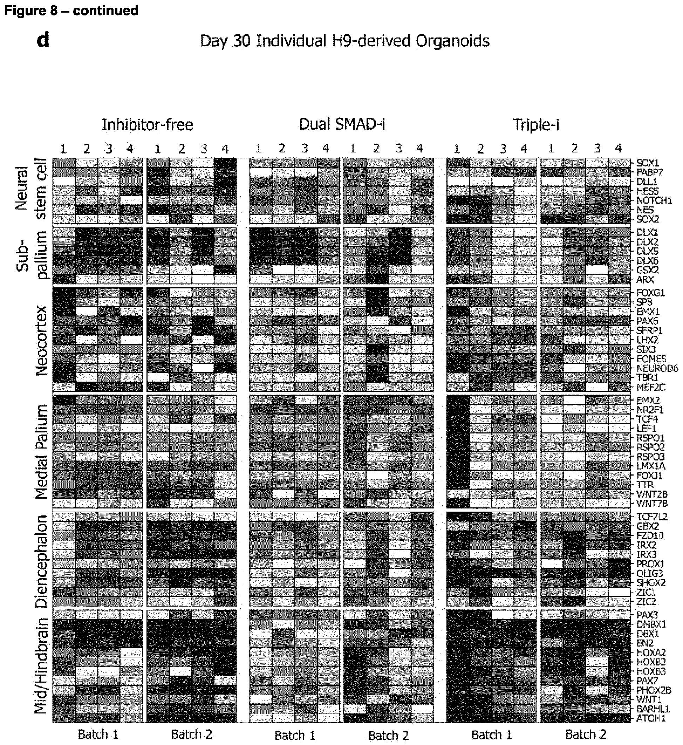

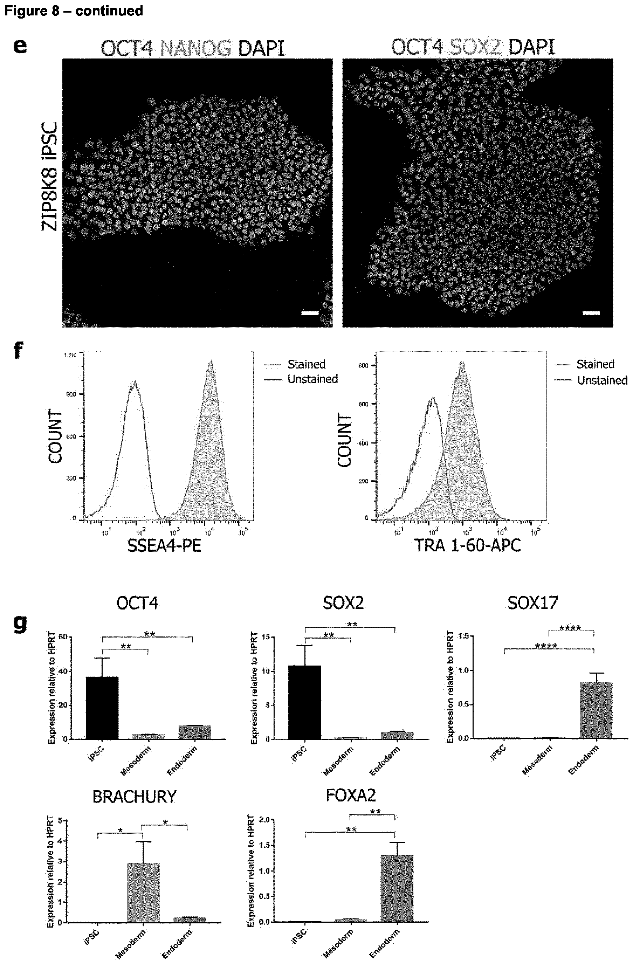

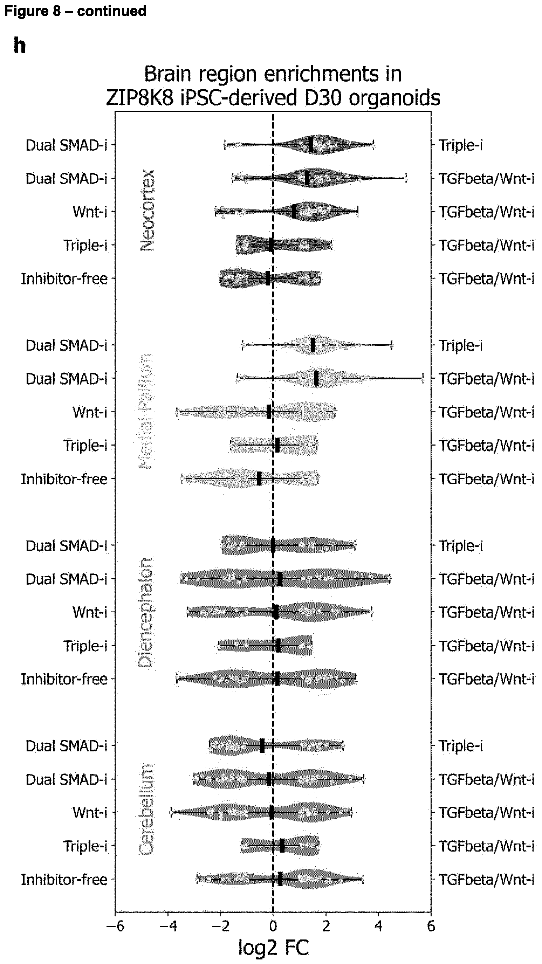

### Figure 8: Differentiation Schematics, regional transcriptomic characterization of organoids derived by various methods, and iPSC line characterization

**a.** Schematic representation of 2D monolayer neural rosette differentiation protocol from human PSCs.
**b.** Schematic showing generation of 3D cerebral organoids from human PSCs. Note that the factors were added to the culture from day 2 until day 10 (or 11) of differentiation protocol.
**c.** A heatmap representing expression values for selected genes categorized according to neural stem cell markers and additional groups of regional markers (sub-pallium, neocortex, medial pallium, diencephalon and midbrain-hindbrain) in pooled day 17 H9 hESC-derived organoids derived under indicated treatments. See Figure 1a legend for further description of number organoids used for data generation. Color-coded scale represents relative expression levels of each gene (row) across treatments.
**d.** A heatmap representing expression values for selected genes categorized according to neural stem cell markers and additional groups of regional markers (sub-pallium neocortex, medial pallium, diencephalon and midbrain-hindbrain) in individual day 30 H9 hESC-derived organoids derived under indicated treatments and separated by batch. See **Fig. 1a** legend for further description of number organoids used for data generation. Color-coded scale represents relative expression levels of each gene (row) across treatments.
**e.** Immunostainings of pluripotency markers OCT4 and NANOG as well as OCT4 and SOX2 in undifferentiated ZIP8K8 iPSCs. Scale bar: 50 µm.
**f.** Flow cytometry expression analysis of the cell surface markers SSEA4 and TRA1-60 in undifferentiated ZIP8K8 iPSCs.
**g.** Quantitative PCR (qPCR) analysis of OCT4, SOX2, BRACHURY, FOXA2 and SOX17 transcript levels obtained for undifferentiated iPSC and following a five-day differentiation in to mesoderm and endoderm lineages using the STEMdiff^{™} Tri-lineage Differentiation Kit. Statistics: Bars represent mean ± S.E. Statistical test: two-way ANOVA followed by Tukey's post hoc test: **P* < 0.05; ***P* < 0.01 ****P* < 0.001; *****P* < 0.0001.
**h.** Violin plot of differentially expressed genes (|log2 fold change| ≥ 1) among ZIP8K8 iPSC derived pooled day 30 organoids (N=1 Dual SMAD-i, 5 organoids pooled; N=1 Inhibitor-free, 5 organoids pooled; N=1 WNT-i, 4 organoids pooled; N=1 TGFbeta/WNT-i, 4 organoids pooled; N=6 Triple-i, of which 5 organoids pooled (N=1) and 4 organoids pooled (N=5)) compared under indicated treatments.

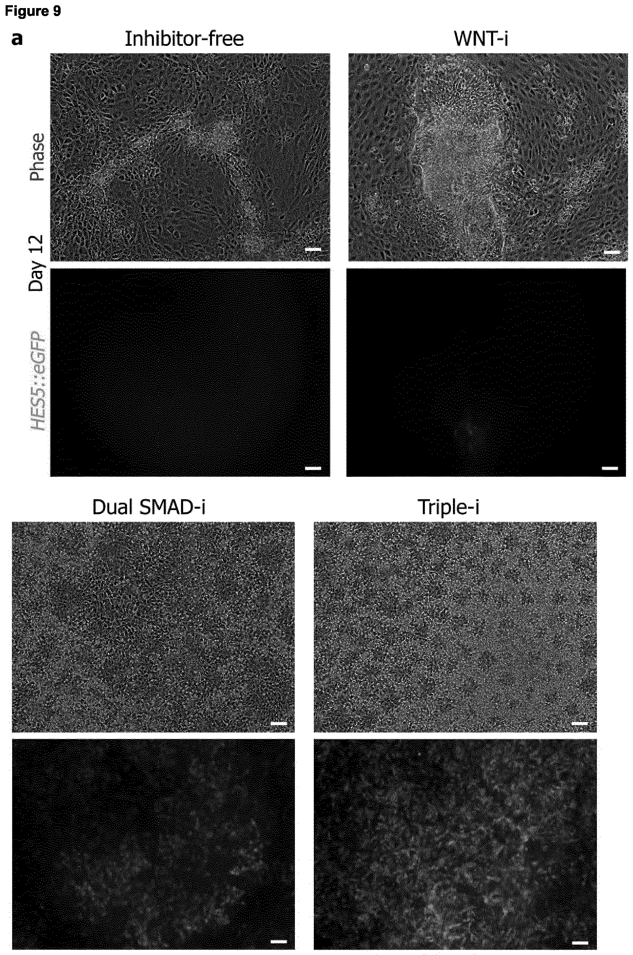

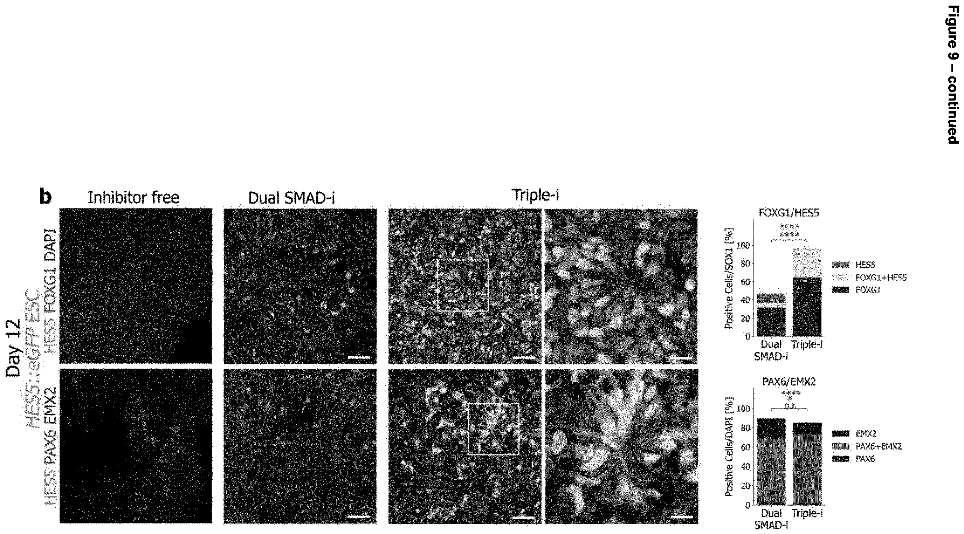

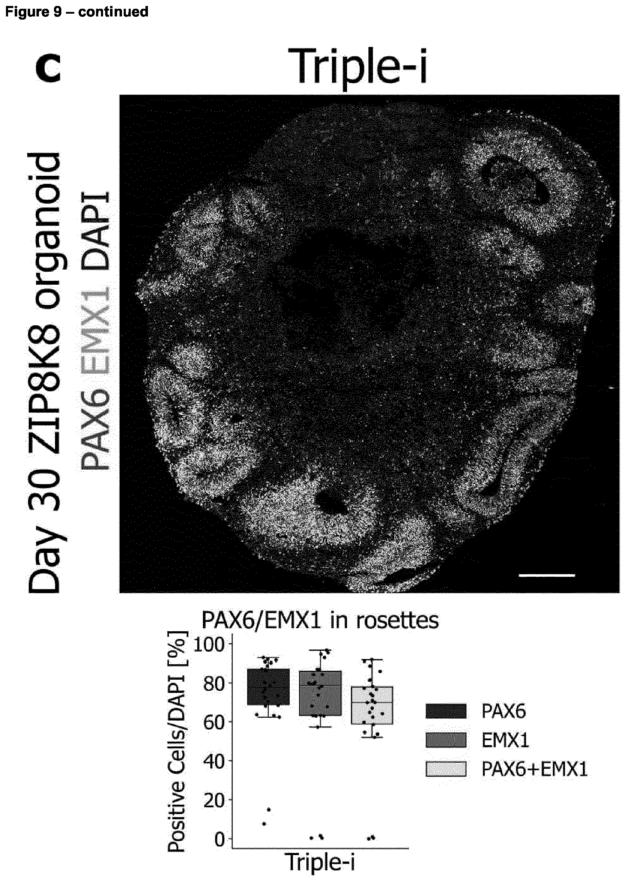

### Figure 9: Enhanced Notch activation and efficient radial organization co-localize with cortical markers in Triple-i derived monolayer cultures

**a.**Phase contrast (top) and *HES5::eGFP* (bottom) images of day 12 neural monolayer progenitors derived from small EBs (sEBs) subjected to neural induction under Inhibitor-free conditions **(Inhibitor-free),** WNT inhibition using XAV-939 **(WNT-i),** Dual SMAD inhibition using SB-431542 and Noggin **(Dual SMAD-i)** and combined Dual SMAD and WNT inhibition **(Triple-i).** Scale bar: 50 µm.
**b.** Immunostaining of FOXG1 (top) and PAX6 and EMX2 (bottom) with respect to Notch activation (HES5::eGFP) and radial organization in day 12 monolayer sEB protocol neural progenitors derived by indicated treatments. The right image represents a magnified rosette from Triple-i derived progenitors. Scale bar: 50 µm. Statistical test: *Fischer's exact test* with Benjamini-Hochberg multiple hypothesis correction; *P<0.05; **P<0.01 ***P<0.001; ****P<0.0001. Note the dramatic shift in marker co-localization together with radial organization under Triple-i. Cell counts and co-localization analysis of markers are presented in the right panel.
**c.**Immunostaining for cortical markers PAX6 and EMX1 in Triple-i derived organoids generated using ZIP8K8 hiPSC line (n=26 rosettes, N=2 organoids). Scale bar: 200 µm.

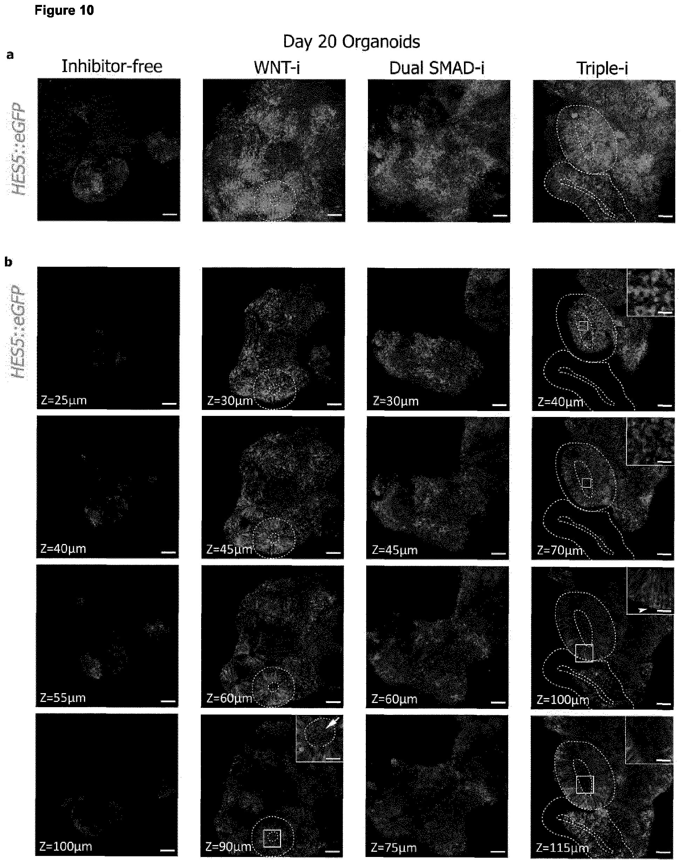

### Figure 10: Enhanced radial patterns in 101 cerebral organoids derived by Triple-i

**a.** HES5::eGFP representative confocal images taken from Day 20 organoids derived under the original protocol1 or original protocol supplemented with indicated inhibitor combinations (N≥3 for each day/treatment). Shown is maximum intensity projection of HES5::eGFP confocal images taken at 5µm intervals across each organoid (see corresponding z-sections in b). Note the sparse HES5::eGFP expression in vesicles of non-treated organoids. Note organization into rosettes but not large vesicles in WNT-I treated organoids (dashed circle). Note irregularity in shape and cytoarchitectural organization of HES5::eGFP expressing cells in Dual SMAD-i treated organoids. Note near complete vesicle organization and enhanced Notch activation in Triple-i treated organoids (dashed line). Scale bar: 100 µm.
**b.** Confocal z-sections of HES5::eGFP across the Day 20 organoids shown in (a). Shown are representative images from stacks taken for each treatment, with 33-49 planes collected per stack, depending on treatment. Four representative planes per each organoid are shown. Z-stacks appear from top to bottom images (representing stacks proximal and distal to objective). Triple-i treatment: Basal and apical sites of vesicle ventricular zones are represented by outer and inner dashed lines, respectively. Ventricular zone is represented by HES5::eGFP expressing cells located within outer and inner dashed lines. Vesicle center (surrounded by inner dashed lines) represents from top to bottom the ventricular lining of cerebral vesicles. Insets represent enlargements of indicated areas in each image (open squares) corresponding from top to bottom the ventricular lining of cerebral vesicles. WNT-i treatment: outer and inner dashed lines represent rosettes that have not developed to vesicles. Scale bars for all large images: 100 µm. Inset scale bars for Triple-i treatment, from top to bottom: 16.7 126 µm, 16.7 µm, 33 µm, 33 µm. Inset scale bar for WNT-i treatment: 33µm.

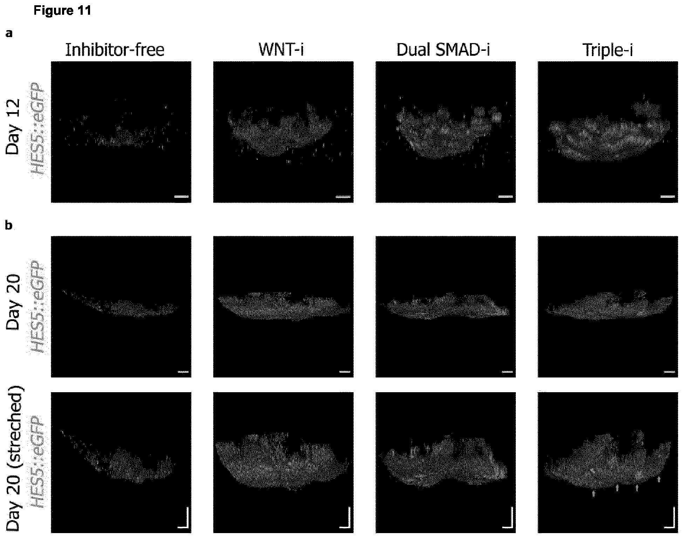

### Figure 11: Robust cerebral vesicle formation 127 in Triple-i derived organoids

**a.** 3D reconstruction analysis of consecutive confocal images taken for Day 12 representative organoids derived by indicated treatments (N≥3). Confocal images were taken at 15µm intervals across each organoid. Scale bar: 100 µm.
**b.** Day 20 organoids treated and imaged as in (a), except that confocal images were taken at 5µm rather than 15µm intervals across each organoid (N≥3). In the bottom panel, the y-axis was stretched twice as much compared to the top panel to improve vesicle visualization. Horizontal scale bar: 100 µm; vertical scale bar: 100 µm. Note enhanced HES5:eGFP expression under all treatments compared to inhibitor-free organoids. Note vesicle initiation and expansion exclusively under Triple-i. Green arrows in the Triple-i conditions marked the vesicle for better visualization.

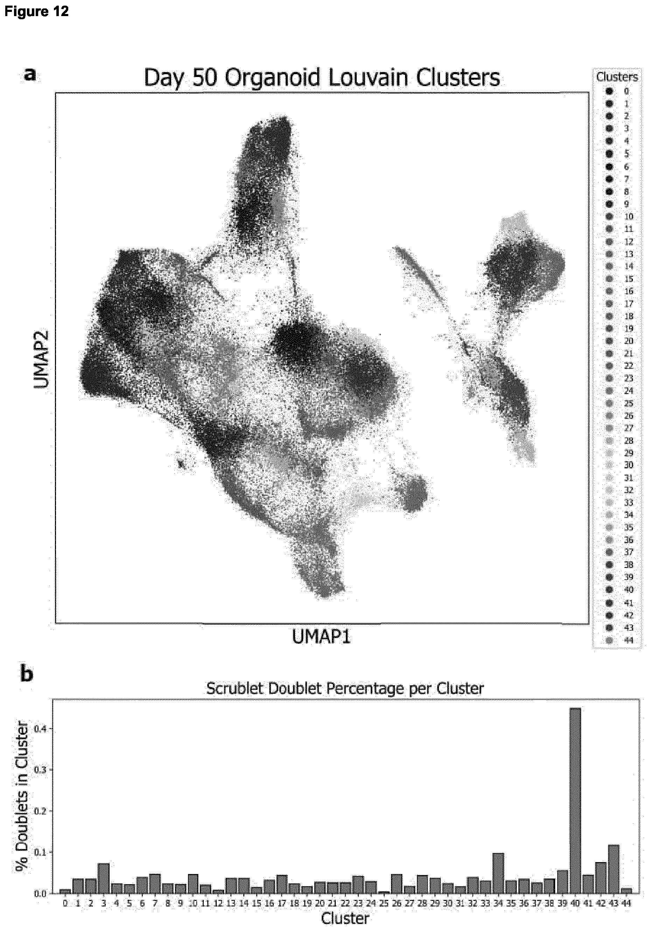

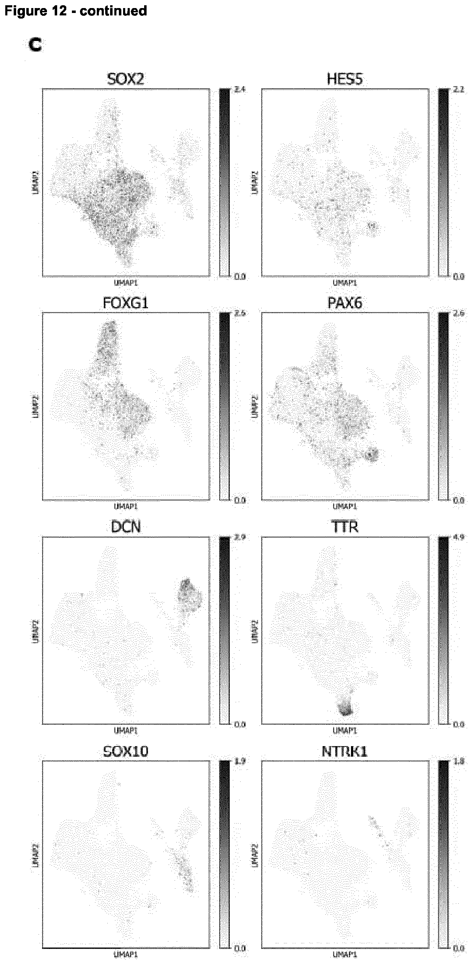

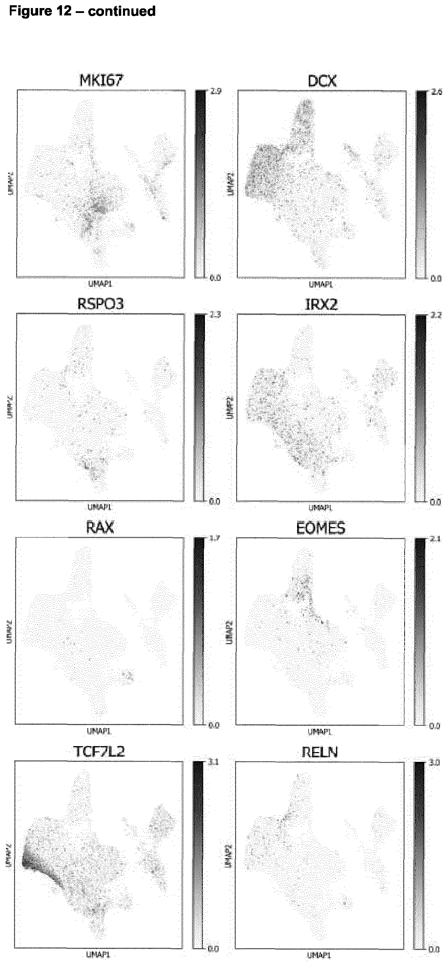

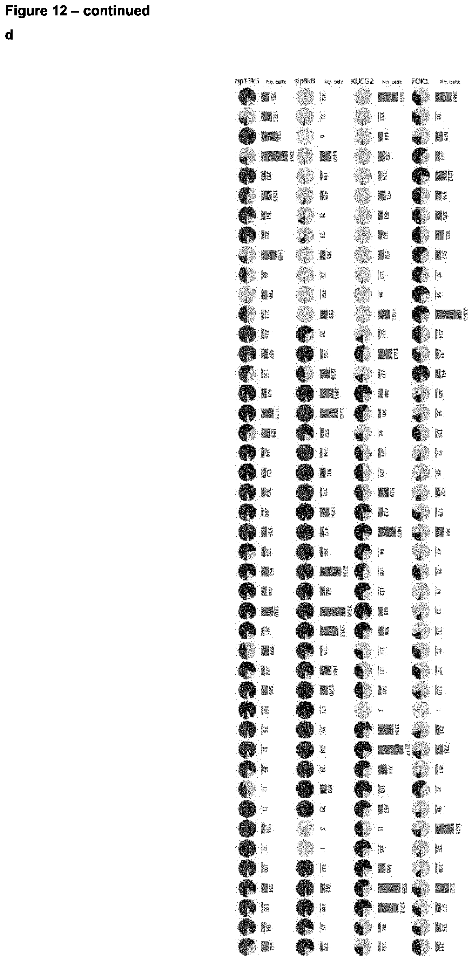

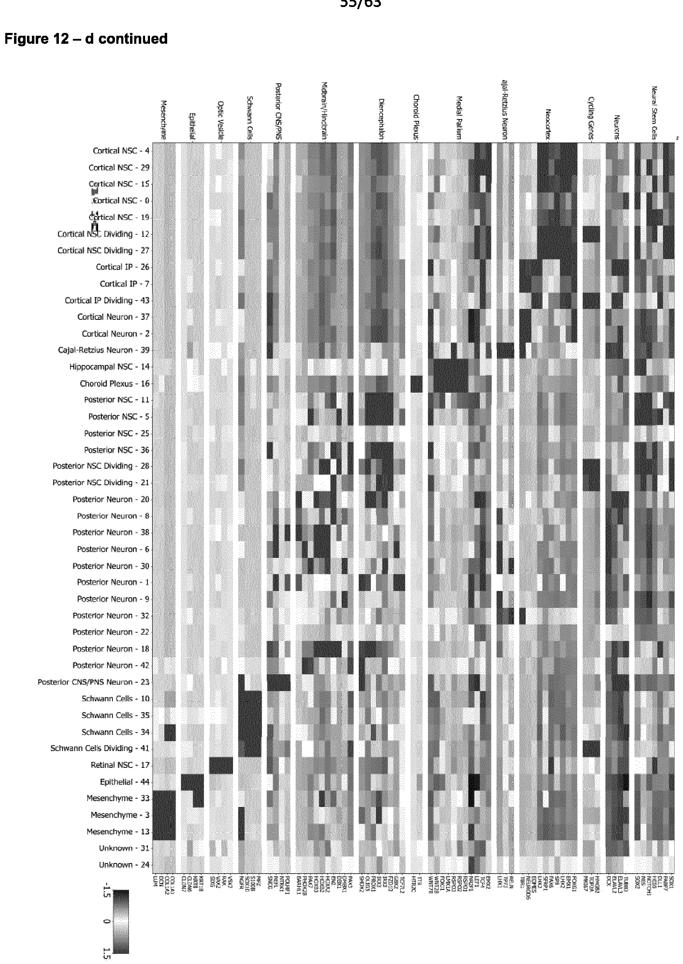

### Figure 12: Clustering of scRNA-152 Seq data obtained from day 50 organoids across four cell lines and three protocols

**a.** A UMAP derived from scRNA-Seq data of day 50 Triple-i, Dual SMAD-i and Inhibitor free organoids was estimated and Louvain clustering approach (see Methods) was used to determine clusters. Each cell is colored by its corresponding cluster. The relative percent of estimated doublets from Scrublet 2 is plotted for each cluster.
**b.** Expression levels in each cell for selected marker genes is plotted using the same UMAP embedding from (a).
**c.** Heatmap displays relative expression values after z-score normalization of average log-normalized expression values for each gene across clusters for selected genes categorized according to neural stem, neuronal, cycling, regional, and other cell type markers. Pie charts above each cell type display percentage of cells from each derivation protocol across all 4 iPSC lines. Pie charts are colored with a gray color if less than 10 cells from that iPSC line were assigned to the given cluster. Bar plots display the total number of cells within each iPSC line assigned to the given cluster.

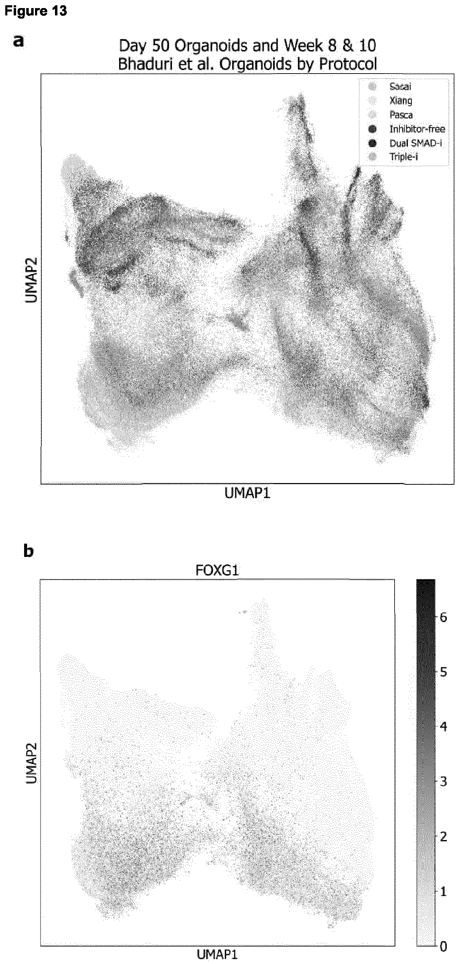

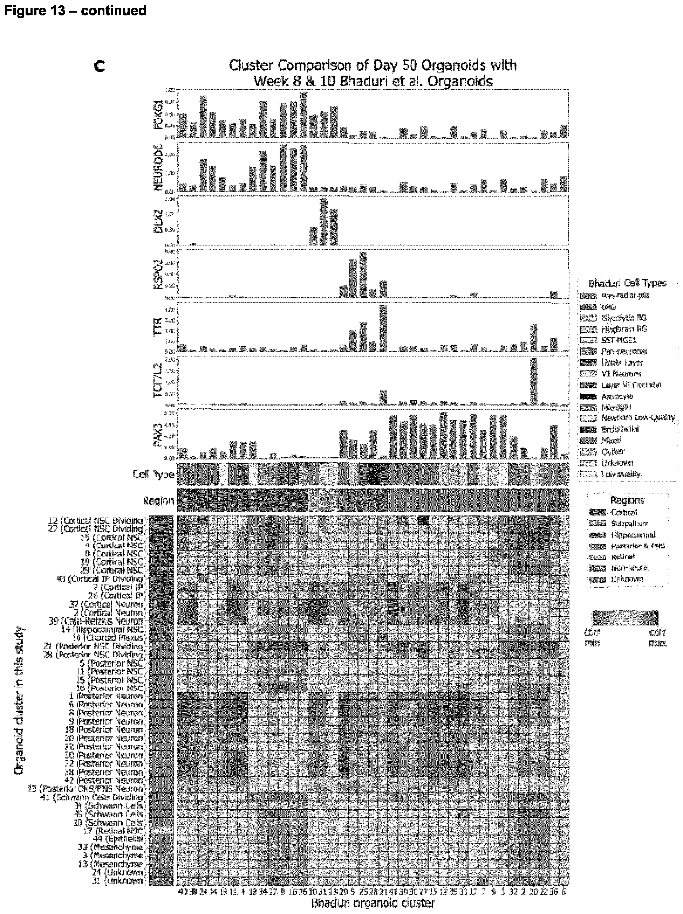

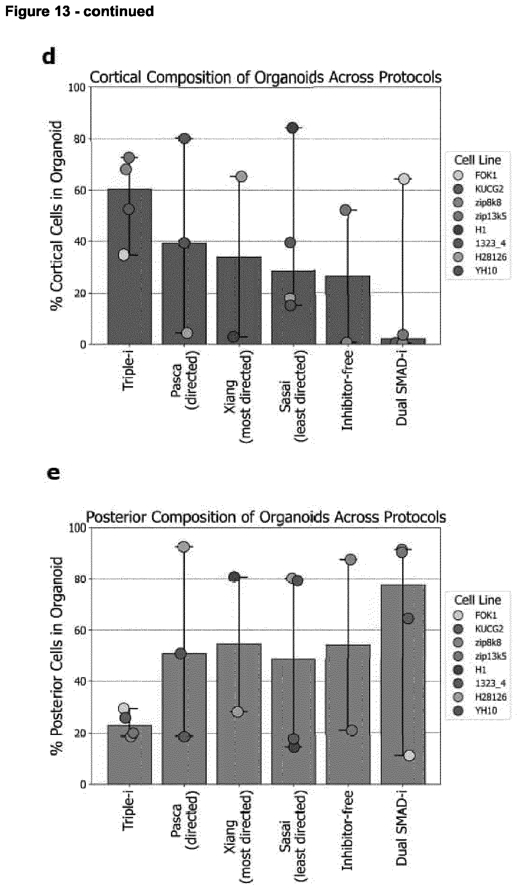

### Figure 13: Integrated analysis with public datasets highlights consistent production of cortical cell types and repression of posterior cell types in Triple-i organoids

**a.** A UMAP derived from scRNA-Seq data of day 50 Triple-i, Dual SMAD-i and Inhibitor181 free organoids from this study after integrating with all week 8 and week 10 in-vitro cells across 3 derivation method from Bhaduri et al. 3 was estimated and each cell is colored by its respective derivation method.
**b.** The normalized expression levels of FOXG1 across all cells was plotted using the same UMAP embedding from (a).
**c.** Pearson correlation coefficients measured using average normalized expression levels of highly variable genes across all cells within a cluster were estimated between clusters found in this study and organoid clusters from weeks 8 and 10 found in Bhaduri et al. study, and are shown in the heatmap. Clusters from this study and Bhaduri et al. clusters were ordered according to regional and cell type assignment. The bar plots above each column display the average expression levels across all cells within each Bhaduri et al. cluster of regional specific marker genes FOXG1 (cortical/subpallium), NEUROD6 (cortical neuron), DLX2 (subpallium), RSPO2 (hippocampal), TTR (choroid plexus), TCF7L2 (diencephalon), PAX3 (midbrain/hindbrain).
**d.** The bar plot shows the median percentage of cells annotated as cortical across individual cell lines within each derivation protocol in both our scRNA-Seq dataset (Triple-i, Dual SMAD-i and Inhibitor-free) and Bhaduri et al. datasets (Pasca, Xiang and Sasai), and are ordered in decreasing order. Each dot shows the percentage of cortical cells within each cell line, and the intervals display the full range (minimum to maximum) of cortical percentages.
**e.** The bar plot is the same as in (d) except shows the percentage of cells annotated as posterior/PNS across both datasets.

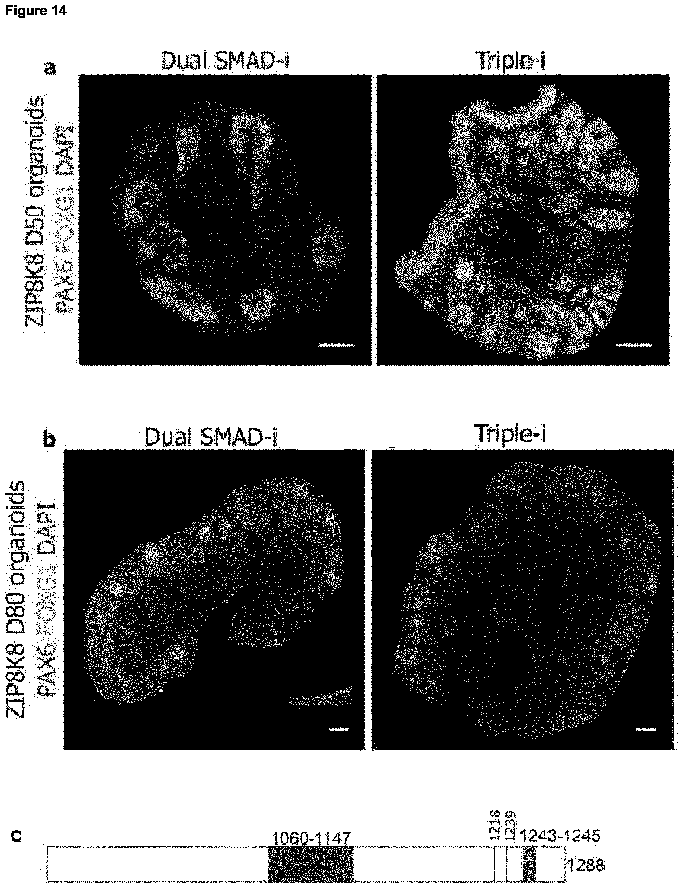

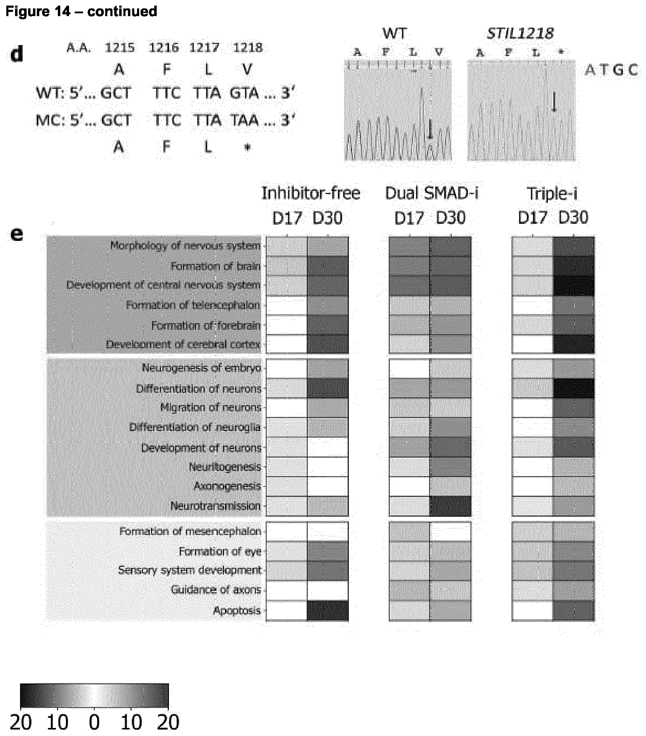

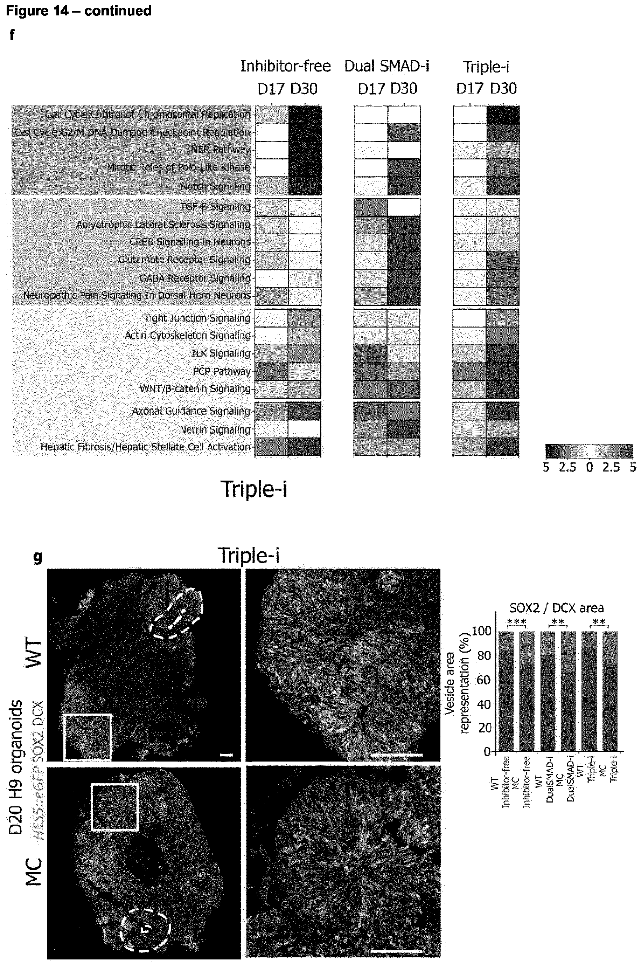

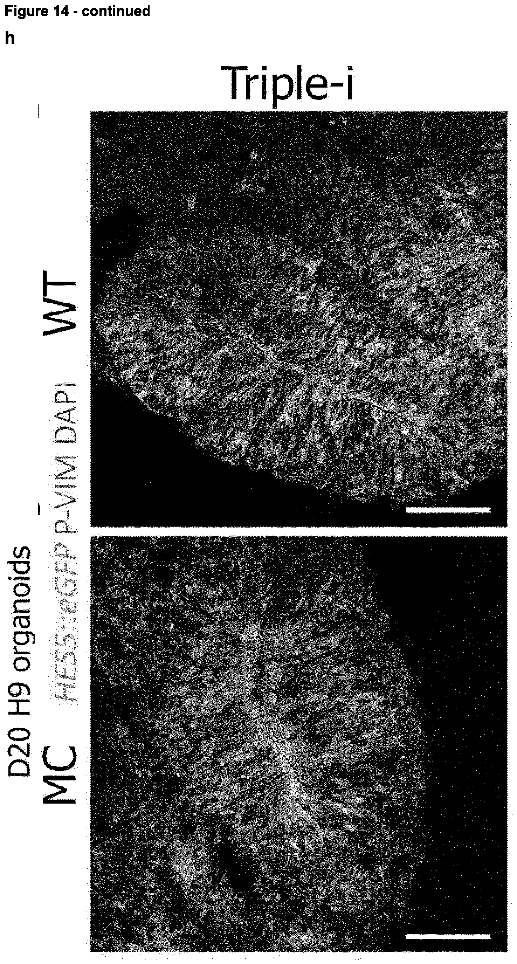

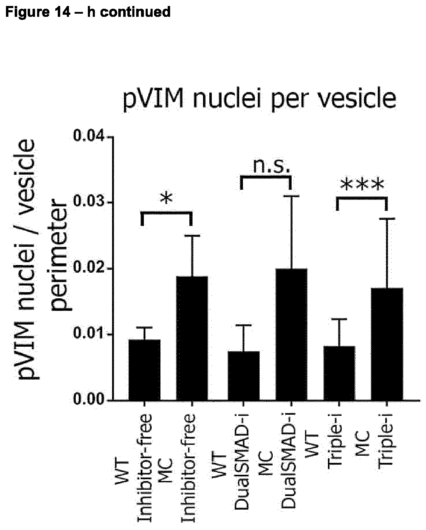

### Figure 14: FOXG1 expression in mid-221 stage and late-stage Dual SMAD-i and Triple-i organoids, and microcephaly transcriptomic and cellular analyses.

**a.** Immunostainings of cortical markers FOXG1 and PAX6 along with DAPI in day 50 organoids derived from ZIP8K8 iPSCs by indicated treatments. Scale bar: 200 µm
**b.** Immunostainings of cortical markers FOXG1 and PAX6 along with DAPI in day 80 organoids derived from ZIP8K8 iPSCs by indicated treatments. Scale bar: 200 µm
**c.** Description of the STIL protein including the STAN and KEN domains, as well as Microcephaly mutations.
**d.** Normal and planned mutated nucleotide sequence adjacent to the region 183 corresponding to the 1218a.a (left). Genome edited sequencing results (right). The generated mutation is homozygous.
**e.** Heatmap showing -log10 p-values of pathway enrichment from Ingenuity Pathway Analysis (IPA) across Diseases and Functions. For pathways which were more significantly up-regulated than down-regulated, the cells were plotted using the p-value estimate of the up-regulated genes and colored using the red color-scale. For pathways which were more significantly down-regulated than up-regulated, the cells were plotted using the p-value estimate of the down-regulated genes and colored using the blue color-scale.
**f.** Heatmap showing -log10 p-values of pathway enrichment from Ingenuity Pathway Analysis (IPA) across Canonical Pathways. See C. for a description of color-coding.
**g.** Combined *HES5:eGFP* expression and immunostaining images for the neural progenitor marker SOX2 and the early neuronal marker DCX in day 20 WT and MC mutant organoids derived under Triple-i conditions. Four-fold magnifications of regions in left images are shown on the right. Quantification of SOX2 and DCX area proportions within vesicles obtained WT or MC organoids derived by indicated treatments and derived from N=3 organoids in each treatment. Scale bar: 100 µm. Statistics: Stacked columns represent relative SOX2 (red) and DCX (blue) expression w 247 ithin selected areas from WT and mutant vesicles. Statistical test: *two-sided t-test;* **P*<0.05; ***P*<0.01 ****P*<0.001; *****P*<0.0001.
**h.** Combined *HES5:eGFP* expression and immunostaining images for the dividing RG marker P-VIM along with their corresponding DAPI images for day 20 WT and MC mutant organoids derived under Triple-i conditions. Right: cell counts of luminal P-VI in WT and MC organoids derived by indicated treatments are derived from N=3 organoids in each treatment. Statistical test: *two-sided t-test;* **P*<0.05; ***P*<0.01 ****P*<0.001; *****P*<0.0001.

The examples illustrate the claimed invention.

### Example 1 - Introduction

Correct development and expansion of diverse neural cell types in the cerebral cortex relies on the ability of early cortical neuroepithelial cells and radial glial (RG) cells-the starting neural stem cell (NSC) population of cortical development¹-to maintain adequate levels of self-renewal and differentiation capacities. Deviations from this highly ordered process, often associated with pathological conditions or evolutional changes, must entail inherent changes in cortical progenitor cell biology². Therefore, the development of gold-standard in vitro strategies for generating precise, staged-matched, homogeneous cortical cell types from various pluripotent stem cells (PSCs) or donor somatic cell-derived induced pluripotent stem cells (iPSCs) is fundamental for obtaining reliable cell sources for comparative studies of development, disease and evolution.

The advent of PSCs led to the establishment of various methods for deriving cortical fates. However, protocols are highly diverse and growing in number. Pioneering work on 2D systems launched by the Sasai group utilized Nodal and WNT pathway antagonists for derivation of general telencephalic fates from mouse PSCs³. This default neural induction mechanism was later recapitulated in human PSCs, and using the BMP antagonist Noggin isolated neural rosette-forming NSCs corresponding to early anterior RG-like NSCs^{4, 5}. This method was significantly improved later by adding TGF beta inhibition to BMP inhibition - becoming the widely accepted Dual SMAD inhibition protocol in human PSCs⁶. Additional methods either relied on intrinsic signals⁷, or employed different combinations of TGF, BMP and WNT signaling inhibition with or without FGF signaling modulation⁸⁻¹³. The Dual SMAD inhibition method was earlier utilized to isolate consecutive building blocks for corticogenesis from human PSCs based on their Notch activation state using a Notch activation reporter as readout¹⁴.

Parallel to 2D differentiation systems there has been a rapid expansion in the development and utilization of brain organoid models, enabling 3D in vivo-like views of fundamental neurodevelopmental features and processes in health and disease. Nonetheless, 3D methods are also highly variable, ranging from inhibitor-free conditions¹⁵⁻¹⁷ to Dual SMAD (TGF-beta/BMP) inhibition^{18, 19}, TGF-beta/WNT inhibition²⁰⁻²⁵ and combined Dual SMAD/WNT inhibition^{26, 27}.

Here organoids were generated by combinatorial pathway inhibition and compared standard and more directed derivation methods side-by-side at the transcriptional, cellular, and cytoarchitectural level. Bulk and single-cell RNA-Seq datasets of organoids derived by these methods were integrated together with published datasets obtained from human brain samples. This allowed us to pinpoint major differences between these derivation methods, identify the combined Dual SMAD and WNT-inhibition method as exclusive for both promoting cortical fates and suppressing non-cortical fates, validate more unequivocal markers to readout corticogenesis, and highlight phenotypic differences among methods in an organoid microcephaly model. Single-cell approaches were further used to detect regional differences in NSCs and their progeny depending on derivation method, and identify an NSC subpopulation with outer radial glia (oRG) molecular characteristics that are enriched within organoids derived by combined inhibition. Finally, it was shown that this method facilitates NSC radial organization - a hallmark of pluripotent stem cell transition to cortical fates - and the subsequent formation of well-defined cortical germinal zones, VZ, iSVZ and oSVZ. These findings underscore the indispensable role of combined inhibition in recapitulating cortical cellular diversity and uncovering unique cortex-specific disease etiologies. Furthermore, the data generated in this study can serve as valuable and powerful references to model brain development, and in particular corticogenesis, under normal and pathogenic conditions.

### Example 2 - Results

### Example 2.1 - Combined TGF/BMP/WNT inhibition is superior over other methods in enriching cortical identity while suppressing non-cortical fates

To dissect the necessity of different inhibition variants used in currently published protocols for achieving cortical fates, organoids generated by the standard, inhibition-free protocol³⁰ (denoted as Inhibitor-free), were compared to those generated by WNT inhibitor XAV939 alone (WNT-i) or TGF-β/BMP inhibitors SB-431542/Noggin together (Dual SMAD-i)⁶ as controls, as well as to organoids made by combined Dual SMAD and WNT inhibition (Triple-i) as the most directed cortical differentiation paradigm^{23, 31} (see Fig. 8a,b for detailed schematic). Bulk RNA-Seq was employed on individual day 30 hESC-derived organoids derived by these conditions, as well as several organoids pooled and analyzed together on days 17 and 30. Correspondence analysis (CA) confirmed that undifferentiated PSCs, day 17 organoids, and day 30 organoids (individual and pooled) segregated as separate clusters, reflecting transition from pluripotency to early and then later neural stages, while day 30 samples further segregated apart according to the different inhibition paradigms (Fig. 1a).

The transcriptional differences were then correlated among organoids made by the various methods to regional biases using a comparative analysis with in vivo human brain development³². To explore this correlation in an unbiased manner all weeks from the Allen Brain Atlas were included ranging from 8 to 37 gestational weeks (Fig. 1b,c). This integrated analysis showed that day 17 organoids co-clustered with week-8 brain tissues regardless of method, although regional specification was already present (Fig. 8c). On the other hand, day 30 organoids clustered with forebrain/cortical embryonic samples - particularly of weeks 12-21 - only if derived by Triple-i (Fig. 1b, c), while Dual SMAD-i appeared with some proximity to the cerebellar embryonic samples, and Inhibitor-free organoids were less associated with any of the in vivo developmental stages. This strongly indicated that early exposure (days 2-11 in our protocol) to combined Dual SMAD and WNT inhibition is sufficient to significantly promote forebrain/cortical specification.

A differential gene expression analysis confirmed that Triple-i significantly enriched for cortical markers when compared to Dual SMAD-i (Fig. 1d) or Inhibitor-free conditions (Fig. 1e). In contrast, Dual SMAD-i significantly enriched for thalamic and cerebellar fates when compared to either Triple-i or Inhibitor-free conditions (Fig. 1d, f). In support of these findings, cortex-specific Allen genes consistently upregulated in Triple-i organoids (23/135) were also the highest expressed in that cortical Allen category (Fig. 1g). Similarly, mid-hindbrain-specific genes upregulated in Dual SMAD-i organoids (15/185) were the highest expressed in the cerebellar Allen category.

It was then asked whether heterogeneity was at least in part due to differences among individual organoids generated under the same method. Individual Triple-i organoids exhibited a strong and homogenous cortical signature alongside an inconsistent subpallial signature (Fig. 1h, Fig. 8d), while Dual SMAD-i organoids exhibited a weak, heterogeneous cortical signature alongside a consistent non-cortical signature. Interestingly, Inhibitor-free organoids exhibited sporadic expression of neocortical and posterior markers, arguing that the generation of consistent regional populations in the absence of exogenous patterning is non-reproducible. Finally, medial pallium fate marker genes appeared comparable under all methods suggesting that the patterning of this conserved hippocampal organizer originating in the cortical hem region^{33, 34} is inherently resilient to pathway modulation.

The findings were further confirmed in hESCs also in iPSCs (see Fig. 8e-g for ZIP8K8 iPSC line characterization). Transcriptomic analysis of iPSC-derived day 30 organoids revealed similar treatment-dependent regional signature patterns (Fig. 8h). Furthermore, while organoids derived under TGF-beta and WNT inhibition alone enriched well for cortical fates similar to Triple-i organoids, they did not restrict posterior identity as firmly as those under Triple-i. These findings manifest the established instrumental role of BMP inhibition in acquisition of anterior fates³⁵.

### Example 2.2 - Robust radial organization (rosette formation) and Notch activation hallmarks transition towards cortical fates

Our bulk RNA-Seq analyses suggest that organoids with cortical fates are highly variable among and within methods unless Triple-i is employed. It was looked into the cytoarchitectural dynamics of NSCs in growing organoids in search of differential readouts across methods. By employing the *HES5::eGFP* Notch activation hESC line that reports for NSC activity^{14, 36}, it was highly evident that NSCs marked by Notch activation exhibit superior radial organization (rosette formation) capacity under Triple-i when compared to the other methods, in 2D monolayer cultures (Fig. 9a).

In 3D organoids, radially organized regions reminiscent of ventricular zone-like structures were observed under all treatments (Fig. 2a; Fig. 9b), in line with other studies^{15, 18, 19, 21-24. 30, 31}. Collective fluorescent signals of Notch activation throughout the entire organoid volume in multiple organoids revealed that Inhibitor-free organoids (N=9) and WNT-i organoids (N=9) exhibit minimal Notch activation (Fig. 2b) and low numbers of Notch active radially organized structures (rosettes) (Fig. 2c). On the other hand, Dual SMAD-i organoids (N=9) and Triple-i (N=9) both yielded regions with enhanced Notch activation (Fig. 2a,b). However, areas of Notch activation in Dual SMAD-i organoids did not coincide with radial organization, while many of the radially organized structures in Triple-i organoids were Notch active (Fig. 2a,c; Fig. 9b). These results demonstrate that early pathway inhibition had a robust effect on shaping cytoarchitectural features of NSCs.

To verify that the NSCs within radially organized regions within organoids are of cortical identity, a series of immunostainings of FOXG1, PAX6 and EMX2 was performed in *HES5::eGFP* hESC organoids. FOXG1 was only partially expressed under Inhibitor-free conditions and was completely absent in Dual SMAD-i organoids within rosettes, reflecting the non-cortical bias induced by these methods (Fig. 2d). However, only in Triple-i organoids FOXG1 was widely expressed and also appeared in radially organized regions (vesicle areas) together with Notch activation (Fig. 2d). PAX6 and EMX2 are expressed in the cortex rostrally and caudally with shared regions dorsally³⁷, but they are also expressed in non-cortical regions in the forebrain³⁸. Accordingly, PAX6 and EMX2 expression was observed under all treatments (Fig. 2d) regardless of FOXG1 expression, implying both cortical and non-cortical identity. Only under Triple-i organoids and 2D monolayer cultures (Fig. 9c), however, the expression of these markers greatly overlapped and was associated with radial organization of Notch active cells, linking rosette formation and dorsal cortical NSC identity.

The formation of these telencephalic VZ-like regions under Triple-i in hESC derived organoids was further shown in human iPSCs (Fig. 2e). Rosette structures were abundant and homogeneously expressed FOXG1 in Triple-i iPSC derived organoids, while organoids generated by the other methods had few rosettes and lacked FOXG1 expression (Fig. 2e). PAX6 and EMX2 expression largely overlapped within rosettes only in Triple-i organoids (Fig. 2e). A similar finding was obtained when the colocalization of PAX6 with the rostrocaudal cortical marker SP8 was examined (Fig. 2e). COUP-TF1, a distally located caudal cortical marker, was moderately expressed in Triple-i organoids (Fig. 2e). Furthermore, EMX1, a dorsal cortical marker, appeared throughout organoid vesicles in Triple-i organoids and co-localized with PAX6, together demarcating cortical ventricular zones (Fig. 9d). Together with the previous findings in hESC organoids, these results suggest that Triple-i organoids display a pan-dorsal/medial cortical identity with a rostral bias. Furthermore, they underscore the necessity of confirming the co-expression of these markers in association with radial organization of to verify cortical identity.

Finally, it was validated by immunostainings some of the region-specific genes enriched in each derivation method based on the bulk RNA-Seq analysis, and assessed their expression with respect to radial organization (Fig. 2f). It was found that the cortical marker MEF2C, which was preferentially expressed in our Triple-i organoids, appeared in both radially organized and neuronal regions. On the other hand, non-cortical markers enriched in methods other than Triple-i including OLIG3, TCF7L2, LMX1A and TTR were significantly enriched in organoids generated by Inhibitor-free and Dual SMAD-i (Fig. 2f).

### Example 2.3 - Derivation methods evoke distinct cortical and non-cortical signatures in NSCs and their progeny

To assess whether NSCs reflected cortical regional homogeneity single-cell RNA-Seq was employed on organoids across 4 iPSC lines. Day 50 organoids were analyzed in order to allow for the accumulation of diverse differentiated cell types (compared to day 30) and also to particularly inquire if such cortical NSC identity was preserved long after inhibitor withdrawal. Single cell transcriptomes exhibited a considerable overlap across all 4 iPSC lines (**Fig. 3a,b**), signifying the capability of all lines to give rise to similar cell types. However, while Triple-i cells distributed similarly irrespective of cell line, Dual SMAD-i and Inhibitor-free cells differentially partitioned depending on cell line, indicating that Triple-i enforced a robust effect on endogenous signaling (**Fig. 3c**,**d**).

Then unsupervised clustering of all single cell transcriptomes was performed across all derivation protocols and cell lines, and identified 45 clusters in total **(****Fig. 10****),** which were then assigned to 18 cell types based on enrichments of known marker gene expression (see methods) **(****Fig. 3e**,**f**). It was found that organoids generated by Triple-i exhibited consistent and robust cortical specification across all four cell lines (median of 60% cortical cells) accompanied by a repression of posterior and PNS fates (median of 23% posterior CNS / PNS cells) **(****Fig. 3f****).** In stark contrast, three out of the four cell lines differentiated under the under Dual SMAD-i method exhibited an overwhelming posterior CNS / PNS identity (median of 78%), whereas only one cell line (FOK1) contained a high level of cortical specification (64% cortical cells). Inhibitor-free conditions also inconsistently gave rise to cortical populations, with one cell line (ZIP13K5) containing 52% cortical identity, and the other (ZIP8K8) yielding merely 0.1% cortical identity **(****Fig. 3g****).** Intriguingly, the preferential enrichment of cortical identity under Triple-i as well as the regional heterogeneity seen in Dual SMAD-i and Inhibitor-free conditions was strongly reflected in organoid NSC populations **(****Fig. 3h****).** These results demonstrate that early specification by exogenous cues evoked a regional bias that persisted in NSCs and their progeny even 40 days following inhibitor withdrawal. One of the most compelling pieces of evidence for the superiority of Triple-i is the ability of this method to produce nearly identical proportions of cortical germinal zone and neuronal cell populations across all cell lines. Conversely, these proportions widely differed across cell lines when derived under Dual SMAD-i and Inhibitor-free **(****Fig 3i****).**

Next the level of cortical specification in organoids from this study was compared to those obtained from stage-matched organoids generated by various protocols^{21, 26, 27} side-by-side and analyzed at the single cell level in the comparative study by Kriegstein and colleagues²⁹. Merging the datasets revealed a strong overlap of our Triple-i organoids with FOXG1+ populations across both studies **(****Fig. 11a,b**). Then clusters from the Bhaduri et al. study were annotated with corresponding brain regions, and observed a strong correlation between corresponding cell types across studies (**Fig. 11c**). Importantly, based on these annotations it was revealed that across all protocols and lines examined in this study and the study of Bhaduri et al., our Triple-i organoids exhibited both the highest and most consistent levels of cortical fate induction accompanied by the strongest repression of posterior CNS/PNS fates (**Fig. 11d**,**e**), further emphasizing the superiority of Triple-i in generating robust cortical organoids.

### Example 2.4 - Triple-i enriches for oRG cells within oSVZ regions

It was next investigated whether oRG cells were preferentially enriched in Triple-i organoids. It was first sought to identify the clusters that exhibited strong similarities with in vivo oRG populations. Comparing our cortical clusters (**Fig. 4a,b**) to the in vivo populations derived from the scRNA-Seq data of Bhaduri et al. highlighted several potential oRG populations, including clusters 29, 4 and 27 **(****Fig. 4c****).** A global differential expression analysis among all cortical NSC populations derived from all methods showed that oRG specific marker genes extracted from the Pollen et al. study³⁹ were enriched within Triple-i derived cortical NSCs when compared with those derived from either Dual SMAD-i or those derived by Inhibitor-free conditions (**Fig. 4d,e**). Strikingly, among all Triple-i cortical cells, the strongest enrichment of oRG specific marker genes was found in the cortical NSC cluster 29 **(****Fig. 4f****).** This enrichment was not present in Dual SMAD-i cells **(****Fig. 4g****)** or Inhibitor-free cells **(****Fig. 4h****).** Taken together, these findings signify the ability of the Triple-i protocol to specifically enable the emergence of oRG cells.

Finally, some of the established Pollen oRG markers were utilized to assess the spatial expression pattern of oRG cells surrounding radially organized VZ regions in Dual SMAD-i and Triple-i day 50 organoids across all four cell lines analyzed by scRNA sequencing. First the cortical identity of organoids was assessed by FOXG1 staining. In Triple-i derived organoids, all four cell lines expressed FOXG1 both within and surrounding radially organized VZ regions, which also expressed PAX6 as well as SOX2 **(****Fig. 12****, Fig. 15a**). In contrast, FOXG1 expression in organoids derived by Dual SMAD-i could only be observed in two lines (FOK1 and ZIP8K8) **(****Fig. 12** and **Fig. 15a**, respectively). These findings validate the consistent cortical NSC identity in Triple-i organoids across all lines, as shown by scRNA-Seq. Regions outside of the VZ were examined and it was found that Triple-i organoids across all four lines had a higher number of TBR2+ cells that also formed sizeable SVZ-like structures **(****Fig. 5a,b**,**f**; **Fig. 12****).**

Finally, the contribution of derivation method to oRG cells was evaluated by looking at SOX2+ cells outside of VZ regions. It was found that SOX2/HOPX expressing cells potentially marking oRG NSCs appeared at higher proportions in Triple-i organoids across all lines (**Fig. 5b****,****d**,**f**; **Fig. 12****).** Moreover, these oRG cells were contained within a visible region demarcated by PTPRZ1 - possibly signifying an oSVZ region (**Fig. 5c**,**e**), while this region was poorly defined in Dual SMAD-i organoids. Similarly, a higher proportion of SOX2+ cells was found that co-localized with either LIFR, PTPRZ1 or combined LIFR/PTPRZ1 in these iSVZ/oSVZ regions in Triple-i across all cell lines, when compared to Dual SMAD-i organoids **(****Fig. 5g****).** HOPX, PTPRZ1 and LIFR were also widely expressed within VZ regions together with SOX2, as well as outside of VZ regions with or without either SOX2 or TBR2, further underscoring the necessity to include both spatial and molecular information to identify *bona-fide* oRG cells based on these markers.

To conclude, these findings validate the enhanced oRG gene signature in Triple-i organoids detected in scRNA-Seq also at the cytoarchitectural level, and further demonstrate the marked increase in the presence of oRG cells specifically within SVZ regions across four cell lines. Altogether, these findings highlight the ability of the Triple-i method to reproducibly generate an enriched NSC cortical identity that corresponds well with a marked diversification of germinal zone cells - the VZ, the iSVZ and the oSVZ - across different cell lines.

### Example 2.5 - Later stage Triple-i organoids reproducibly exhibit enrichment of oRG cells, cortical identity and cortical neuronal diversification

It was further investigated whether the oRG signature in Triple-i organoids persisted at a later stage of organoid development, in particular after 80 days of culture across three different cell lines. Discrete dense nuclei regions separated by fine void areas consisting of low nuclei density were first identified (Fig. 6a; illustrated in Fig. 6h). These dense regions strictly co-localized with FOXG1 expression and consisted of NSCs expressing SOX2 with or without co-expression of oRG markers (Fig. 6b,c, Fig. 13,7). SOX2+ cells were present within these regions both luminally within rosettes as well as basally interspersed along with TBR2+ IP cells and neurons but were rarely detected beyond these dense areas. This was in contrast to neurons, which were also found beyond these regions (Fig. 6i, Fig. 13,7). This suggested that these dense nuclei regions, which is termed herein cortical units, represented distinctive in-vitro counterparts of VZ and potential iSVZ/oSVZ germinal zones whereas areas beyond these regions mirrored more cortical plate-like regions. It was found that Triple-i derived organoids across all three cell lines (ZIP13K5, ZIP8K8 and H9) assessed on day 80 were predominantly comprised of these cortical units. In contrast, only Dual SMAD-i organoids produced under the ZIP8K8 cell line contained cortical units (Fig. 15b), with Dual SMAD-i organoids under ZIP13K5 and H9 cell lines completely lacking or showing sporadic FOXG1 expression respectively (Fig. 13,7).

To further investigate the role of added WNT inhibition on top of Dual SMAD-i in the generation of distinct germinal zones and their progeny, organoids were analyzed under the ZIP8K8 line, which showed comparable cortical units under both treatments. It was found that SOX2+ rosettes were variable in their size in Dual SMAD-i cortical units while predominantly uniform in size and thin in Triple-i cortical units (Fig. 6d), suggesting more developmental synchroneity under Triple-i. Concordantly, while iSVZ/oSVZ regions contained similar levels of SOX2+ cells across both treatments (Fig. 6e), Triple-i derived cortical units contained significantly higher levels of SOX2+ cells co-expressing PTPRZ1 and LIFR when compared to those derived by Dual SMAD-i (Fig. 6f,g), suggesting that Triple-i enhanced oRG specification. Together, these findings elucidate the ability of the Triple-i method to reproduce and maintain cortical identity and cortical NSC diversification across different cell lines at later stages of development.

The robust effect of Triple-i on the potency of NSCs within cortical units was further manifested by the widespread enrichment of upper and deep layer neurons within these organoids. In ZIP8K8 organoids, the deep and upper layer neuronal markers CTIP2 and SATB2 were expressed at higher levels both separately and together, and more uniformly within and outside of Triple-i cortical units, marking newly born migrating neurons within the germinal zones as well as accumulated neurons within cortical plate-like regions when compared to cortical units derived by Dual SMAD-i (Fig. 6i). The expression patterns of these markers were also recapitulated in ZIP13K5 and H9 Triple-i organoids, while sporadic and low expression of these markers was detected in ZIP13K5 and H9 Dual SMAD-i organoids, in correlation with the lack of cortical units and FOXG1 expression (Fig. 13,7). The upper layer neuronal marker CUX1 was present in Triple-i organoids within and outside cortical units among all lines (Fig. 6i, Fig. 13,7). While this marker was also expressed across all Dual SMAD-i organoids, it was not associated with the presence of cortical units/FOXG1 expression under this treatment. Interestingly, ZIP8K8 cortical units showed no difference in the expression of TBR1 across the two methods, reflecting a comparable accumulation of this early neuronal marker. Conversely, ZIP13K5 and H9 Dual SMAD-i derived organoids lacked TBR1 expression in comparison to their Triple-i counterparts, which contained widespread TBR1 expression (Fig. 13,7), in correlation with the lack of cortical units in these Dual SMAD-i organoids. Altogether, these findings show that the Triple-i method is capable of reproducing and recapitulating cortical neuronal diversification in a more enhanced and defined level across different lines in comparison to Dual SMAD-i treatment.

To further investigate the robustness of the Triple-i method across cell lines and within individual organoids, as well as to compare the cortical neuronal diversity and oRG specification in Triple-i and Dual SMAD-i organoids, scRNA-Seq of day 80 organoids was performed across ZIP8K8 (3 individual Triple-i organoids, 3 individual Dual SMAD-i organoids) and ZIP13K5 (Triple-i pooled organoids) cell lines (Fig. 6j). In accordance with immunostainings, these organoids contained predominantly cortical cell populations (Fig. 6k). Both Dual SMAD-i and Triple-i organoids exhibited a remarkable reproducibility with respect to cellular composition across protocol and cell line (Fig. 6l). In addition, Triple-i derived organoids contained a significantly higher number of upper layer neurons and significantly lower number of intermediate progenitor cells than their Dual SMAD-i counterparts (Fig. 6m), confirming our findings derived from immunostainings and suggesting a less differentiated stage in Dual SMAD-i organoids. Deep and upper cortical neuronal layer specific markers^{40,41} were enriched in distinct subpopulations, with NEUROD6, a marker for newly born cortical neurons, being more highly expressed in upper layer neurons and IP cells, suggesting a bias towards upper layer neurogenesis (Fig. 6n). This was further supported by the expression of upper layer neuronal markers in NSCs and IPs (Fig. 6n). Moreover, the spliced form of CUX2 was most highly abundant in upper layer neurons, while the rate of CUX2 gene transcription was similarly abundant in IP cells in addition to upper layer neurons (Fig. 6o), suggesting a direct IP to upper layer neuron transition. Finally, Triple-i cortical NSCs exhibited a pronounced up-regulation of oRG specific marker genes derived from Pollen et al.³⁹ when compared to Dual SMAD-i cortical NSCs (Fisher's exact test p=1.2e-10) (Fig. 6p). When combined with immunostainings, these results argue that Triple-i organoids at this later stage of development not only exhibit a more pronounced upper layer neurogenesis, but also continue to enrich for oRG cell populations.

### Example 3 - Discussion

Methods for deriving cerebral organoids are highly diverse and give rise to immensely heterogeneous populations with respect to cortical identity. Despite this fact, comparative studies measuring the homogeneity of cortical fates generated are still exceptionally sparse. Herein it is postulated that such cell type heterogeneity may arise due to inherent non-cortical fates present in the starting population, and have therefore placed particular emphasis to reveal differences among NSCs derived by the different methods.

By systematically comparing methods side by side, probing a number of developmental stages, generalizing our findings in hESCs and hiPSCs, differences between derivation methods and their respective regional biases were demonstrate. By employing bulk RNA-Seq, scRNA-Seq and immunostainings, evidence was provided that early pathway modulation in NSC starting populations by combined inhibition is critical for establishing long-lasting cortical specification. This careful analysis has revealed that organoids generated by combined inhibition exhibit a highly consistent cortical NSC identity independent of iPSC line.

Further evidence is provided that early establishment of cortical NSC identity in organoids is pivotal for the generation of a more rich cellular diversity at later stages of development. Specifically, using scRNA-Seq and immunostainings, it is shown that day 50 organoids derived by combined inhibition selectively enrich for oRG cells populating well-defined oSVZ regions surroundings VZ areas. These VZ and iSVZ/oSVZ regions further develop in day 80 into discrete cortical units, which under Triple-i are enriched with oRG cells as well as deep and upper layer neurons.

Our work also suggests that early cortical NSC homogeneity is critical for achieving robust radial organization within organoids. It was demonstrated that only when derived by combined inhibition, NSCs marked by Notch activation (*HES5::eGFP* expression) and co-localized with cortical markers exhibit a strong capacity to radially organize (form rosettes). This suggests that these structures originating in early organoids serve as the cortical germinal zone - the essential primary mechanical groundwork for the subsequent assembly of consecutively emerging cortical building blocks, which culminate into the formation of cortical units that constitute the organoid. This is in accordance with ample lines of evidence from our lab defining Notch active neural rosettes as cortical ventricular zones^{5, 14, 43, 44}. This study further suggests that the robust formation of these structures specifically under combined inhibition underlies the later emergence of diverse cortical cell types such as oRGs and distinct neuronal layers, and hence could serve as a reliable readout for transition of human PSCs towards cortical NSC starting populations.

Finally, our study shows that the meaningfulness of disease phenotypes in organoid models is highly dependent on derivation method. This study reveals that only when derived by combined inhibition, microcephaly organoids exhibit a significant reduction in size, dramatic loss in cortical gene expression and massive apoptosis. The fact that organoids derived by combined inhibition are comprised of cells with predominantly cortical identity suggests that the phenotypes recorded are cortex-specific. Nonetheless, our data also show that other potential microcephaly phenotypes such as decline in stemness, increased differentiation and cell cycle defects can be observed in organoids generated by more than one method, implying that these phenotypes are either not cortex-specific or severe enough to be revealed in organoids with a lower cortical identity. Thus, a variety of derivation methods may be essential for assessing regionally specific pathophysiological aspects of microcephaly that may be meaningful for shedding light on disease etiology.

To conclude, the systematic comparison between different methods with respect to transcriptional profiles, cytoarchitectural features and cell fate acquisition provides a valuable reference to assess which among the differentiation schemes is suitable for the topic in question.

### Example 4 - Material & Methods

### Generation and use of Pluripotent cell lines

The BAC transgenic *HES5::eGFP* Notch activation human ES cell reporter line has been derived from the WA-09, XX (H9) human ES cell (hESC) line (Wicell) and described previously³⁶.

The microcephaly STIL *HES5::eGFP* Notch activation reporter line was generated in our lab for this study as follows: Clustered regularly interspaced short palindromic repeat (CRISPR)/Cas9 genome editing was used to introduce a nonsense mutation at the *STIL* locus in the H9-derived *HES5::eGFP* hESC reporter line. This nonsense mutation mimics the microcephaly *STIL1218* **(designated in this study as MC)** mutation in which deletion of the nucleotide (nt) G (at 3655 nucleotide position) leads to a premature stop codon (Val1219X). A 21-nt guide RNA (gRNA) sequence and a 150-nt ssDNA oligo (IDT, PAGE purified) were designed to target and generate double-strand breaks within the *STIL* ultimate exon (exon no.17) upstream to the KEN domain and result in the generation of a truncation mutation via non-homologous end-joining (NHEJ) and homology-directed repair (HDR). This gRNA was cloned into pSpCas9 (BB)-2A-GFP (PX458, Addgene) plasmid downstream of U6 promoter using the Gibson assembly cloning method. hESCs (400,000 cells) were nucleofected (Amaxa) with 1.5 µg of SpCas9 plasmid cloned with gRNA and 1 µg of the ssDNA. Ninety-six hours after nucleofection, cells were sorted for GFP signal by FACS. Sorted cells were replated at clonal density (5K) on MEFs supplemented with MEF-conditioned media and 10 µM ROCK inhibitor with daily fresh FGF2. Individual colonies were manually picked and cultured in 24 well plates for a week and later expanded in 6 well plates as individual clones. Genomic DNA was extracted from each clone and targeted genomic region was sequenced.

The ZIP8K8 iPSC line (ZIP gGmbH) was derived from Human dermal fibroblast (HDF) cells were obtained from a 40-year-old Caucasian male with no history of genetically inherited, neurological or metabolic disorders using a 3 mm punch biopsy. Briefly, biopsy material was segmented into smaller fragments, plated onto tissue culture-treated plastic dishes and maintained in HDF medium containing DMEM Glutamax, 1% Penicillin/Streptomycin and 20% fetal calf serum at 37°C and 5% CO2. HDF medium was changed every other day to obtain a 90% confluent monolayer until the cells were passaged at a 1:3 ratio using trypsin. Pluripotent stem cell lines were induced using episomal plasmids following a published protocol with minor modification⁴⁵. All plasmids were a gift by Shinya Yamanaka and obtained via Addgen (http://www.addgene.org). Briefly, 2 µg of pCXLE-hSK (#27080), pCXLE-hUL (#27078) and pCXLE-hOCT3/4-shp53F (#27077) were transfected into 10⁶ HDFs using the Neon microporator device with 100 µl electroporation tips (Settings: 1.650 V, 10ms, 3 timed pulses; Thermo Fisher Scientific) according to the manufacturer's protocol. Transfected cells were resuspended in 10 mL fibroblast medium containing 90% 1× MEM (Thermo Fisher Scientific) supplemented with 10% FCS, and 2.5×10⁴ HDF/cm² were reseeded onto Matrigel coated (0.5 mg/mL) six-well plates. Two days post transfection, fibroblast medium was replaced with TeSR-E7 (Stemcell Technologies) and cells were fed every other day with 2 mL TeSR-E7/well. On day 26-30, post transfection emerging hiPSC colonies were picked and transferred onto Matrigel-coated plastic dishes (Corning; 0.5 mg/mL) in TeSR-E8 medium (Stemcell Technologies) for further expansion. Enzyme-free colony expansion using EDTA was performed every 3-4 days at a 1:6 ratio based on a protocol previously described⁴⁶.

The additional iPSC lines in this study include the human fibroblast derived iPSC line KUCG2⁴⁷ (EBiSC), the human PBMC derived iPSC line FOK1 (Max Planck Institute of Psychiatry) and human fibroblast derived iPSC line ZIP13K5⁴⁸ (ZIP gGmbH).

### Culturing undifferentiated PSCs

The H9-derived *HES5::eGFP* human ES cell (hESC) reporter line and H9-derived *HES5::eGFP* microcephaly *STIL* mutant hESC reporter line were cultured on mitotically inactivated mouse embryonic fibroblasts (MEFs) (Globalstem). Undifferentiated H9-derived human ESC lines were maintained as described previously⁵ in medium containing DMEM/F12, 20% KSR (Knock-out Replacement medium), 1mM Glutamine, 1% Penicillin/Streptomycin, non-essential amino acids (NEM) (100× solution, Thermo-Fisher Scientific) and 50µM beta-mercaptoethanol (Thermo-Fisher Scientific), supplemented daily with 10 ng/ml FGF2 (R&D). Cells were passaged weekly using Dispase (Worthington) to maintain their undifferentiated state. Human iPSC lines used in this study were cultured and maintained on Matrigel-coated dishes (Corning, 0.5 mg/mL) in mTesR1 medium (Stemcell Technologies), and passaged every 3-4 days using 0.5mM EDTA (Thermo Fischer Scientific) to maintain their undifferentiated state.

### Neural induction and rosette formation from human ESCs using the sEBs protocol

On day 0, hESC colonies were removed from the MEF feeder layer by exposure to 4U/ml Dispase (Worthington) and then dissociated with Accutase (Innovative Cell Technologies, Inc.). Single hESCs were plated at a density of 750,000 cells per one 6-well (low attachment plate, Greiner) and cultured in 2 ml of neural induction medium containing 50% of KSR medium (composed of knockout DMEM with 15% KSR, 2mM Glutamine, 1% Penicillin/Streptomycin, 1% NEM and 50µM beta-mercaptoethanol (all from Thermo-Fisher Scientific)) and 50% of N2/Neurobasal medium (1:1). Our N2 medium is homemade and consists of 6.5g of DMEM/F12 powder (Thermo-Fisher Scientific) and supplements containing 0.775g of D-Glucose, 1g of Sodium bicarbonate, 12.5mg Insulin, 5mg Apo-transferin, 30µl of 500µM Sodium Selenite, 100µl of 830nM Putrescine and 100µl of 100µM Progesterone, 1% Penicillin/Streptomycin (Sigma Aldrich). Herein below reference to this homemade N2 medium is made as N2. Neurobasal medium consists of Neurobasal medium (Thermo-Fisher Scientific) as base supplemented with 2mM Glutamine, 1% Penicillin/Streptomycin, 1% NEM and 50µM beta-mercaptoethanol (Thermo-Fisher Scientific). Reference is made to this Neurobasal medium with supplements as NB from hereon. The final prepared medium was also supplemented with 1% B27 without retinoic acid (RA) (Invitrogen) and 10µM Rock Inhibitor (Y-27632, Tocris). Small EBs (sEBs) were allowed to form for 2 days. **On day 2,** medium was changed to 25% KSR medium and 75% N2/NB medium supplemented with B27 without RA and 10µM Rock inhibitor. In addition, sEBs were either untreated (denoted as **Inhibitor-free**)**,** treated with 10µM SB-431542 (Tocris) and 250 ng/ml Noggin (R&D) (denoted **dual SMAD-i),** treated with 3.3µM XAV-939 (1:6000 from a 20mM stock; denoted **WNT-i**), or their combination (denoted **Triple-i). On day 3,** sEBs were collected and plated on culture dishes pre-coated with 15µg/ml polyornithine (Sigma), 1µg/ml Laminin and 1µg/ml Fibronectin (Po/Lam/FN) (BD Biosciences). Medium and factors were complemented as required, with same composition as for day 2 except for Rock inhibitor, and medium was left unchanged for the next 4 days. **On day 7,** medium was changed to 100% N2/NB medium supplemented with 1% B27 without RA, and further supplemented with 5µM SB-431542, 125 ng/ml Noggin and 3.3 µM WNT-i. **On day 9,** medium was replaced and inhibitors were withdrawn and replaced by 100ng/ml FGF8 and 5ng/ml BDNF (R&D). Rosettes were allowed to form until day 12, following which cells were fixed, harvested for analysis, or subjected to terminal differentiation. Neural induction and direct rosette formation could be also obtained by adherence on Matrigel-coated plates as previously described¹⁴ with the modifications defined for this study (such as XAV-939 addition etc.). Long-term propagation of cortical neural progenitors was performed weekly by manually picking rosettes followed by re-plating on Po/Lam/FN coated dishes and adding 100% N2/NB medium containing 1% B27 without RA and with either FGF8 and BDNF (until day 28), or 20ng/ml of FGF2, EGF and BDNF (R&D) (from day 28 and on). For the human ZIP8K8 iPSC line, the same protocol was used, except that undifferentiated cells were first treated with EDTA or trypLE, respectively (instead of Dispase used for hESCs), and then dissociated using Accutase. Single cells were then plated under the same differentiation conditions used for H9 hESCs.

### Derivation and analysis of cerebral organoids

On day 0, *HES5::eGFP* hESC colonies were first incubated with 1ml of Dispase (4U/ml) for 7-10 mins at 37°C in the incubator until colonies detached from feeder cells (MEFs). Human ESC medium (medium composition is described under Culturing undifferentiated PSCs) was used to neutralize Dispase, two hESC medium washes were given, and colonies were allowed to sink to the bottom of the falcon tube. Supernatant was aspirated, 1 ml of Accutase was added along with ROCK inhibitor (10µM), and cells were kept in 37°C water bath for 4 minutes. Colonies were then triturated using a p1000 tip for 15 times until single cells were obtained. The Accutase enzyme was neutralized by washing twice with hESC medium and centrifugation at 270g for 5 minutes. Single cells were re-suspended in 1ml of hESC medium containing FGF2 (4 ng/ml) and ROCK inhibitor (50µM, Tocris). Following cell counting, the volume of the hESC medium was adjusted along with FGF2 and ROCK inhibitor to obtain a concentration of 9000 cells per 150µl. For hiPSC lines, feeder free culture system was used, where cells were first incubated with 1 ml of EDTA (0.5mM) for 2 minutes at 37°C in the incubator, after which EDTA was substituted with 1 ml of Accutase per a 60mm culture dish, and cells were incubated for 3 minutes at 37°C. Cells were then triturated using p1000 tips for 10-15 times until single cells were obtained. Single cell suspension was first washed with mTesR1, another wash was given with hESC medium containing FGF2, and cells were then centrifuged at 270g for 5 minutes. Single cells were re-suspended, counted and plated similarly as done for hESCs. Suspended single cells (9000/150 µl) were plated on a 96 well U-bottom low attachment plate (Corning). On day 1, the plate was inspected for cell aggregation and formation of small EBs. On day 2, half of the medium was aspirated without disturbing aggregates and 150 µl of hESC medium was added along with 1.5x times the corresponding inhibitor molecules SB-431542 (10µM), Noggin (250 ng/ml) and XAV-939 (3.3 µM; 1:6000 from a 20mM stock) and their combinations. FGF2 and ROCK inhibitor were withdrawn once EB size reached approximately 350µm. On day 4, 150µl medium was removed and replaced with fresh 150µl hESC medium along with 1× corresponding inhibitory molecules. On day 6, organoids were transferred into low attachment 24 well plate along with Neural induction medium consisting of N2 (see N2 composition under Neural induction and rosette formation from human ESCs using the sEBs protocol). Every other day, 300µl of medium were aspirated and replaced by an equal volume of fresh N2 medium along with factors until day 11. On day 11, organoids (500-600 µm in size) were embedded in 30µl Matrigel droplets and incubated for 30 minutes in the incubator, after which they were transferred into a 6 well low attachment plate containing N2/NB medium along with 1% B-27 without RA using a sterile spatula. On day 13, a medium change was made using the same medium from day 11. On day 15, the entire supernatant medium was removed and fresh medium containing N2/NB medium along with 1% B-27 *with* RA was given, organoid dishes were transferred onto an orbital shaker, and medium was changed daily. For long term organoid culture, Matrigel (1%) was directly added to the culture medium and medium was changed every two days. Organoids were fixed in 4% paraformaldehyde for 20-40 minutes (RT) depending on their culture age, and then cryoprotected and processed as described under Immunostaining and confocal imaging section.

### Preparation and Sequencing of bulk RNA-Seq Libraries

RNA was purified using the miRNeasy^{™} RNA MiniPrep (Qiagene). RNA-seq libraries for human samples were generated using the Illumina TruSeq RNA Library Preparation Kits, and pooled sampled were sequenced on the Illumina HiSeq 2500 sequencer as 100bp paired-end reads. RNA expression datasets were deposited in GEO as FPKM values (GEO# TBD).

### Description of processed RNA-Seq datasets of human brain transcriptomes

Gene expression data for different brain regions was retrieved from BrainSpan Atlas of the Developing Human Brain (http://human.brain-map.org/) based on extensive RNA-Seq study done by Šestan and colleagues³². Among the samples collected for that study, for our analysis datasets were utilized obtained from 16 brain regions dissected from 8 to 37 gestational weeks, out of which 11 obtained from different neocortical regions and 5 others collected from the hippocampal primordia (future hippocampus), sub-cerebral regions including the diencephalon (future thalamic structures) and the sub-pallium (future striatum), as well as posterior brain regions (cerebellum). The file "RNA-Seq Gencode v10 summarized to genes" containing RPKM values (available at http://www.brainspan.org/static/download.html) was downloaded on 3.08.2017. A detailed description of data processing procedures for generating the above file by the authors is available at (http://help.brain-map.org/display/devhumanbrain/Documentation).

### RNA-Seq data processing and normalization for single and pooled organoids

Raw RNA-Seq reads were mapped to the human reference genome hg19 using STAR mapper⁴⁹. Generated bam files were filtered for uniquely mapped reads using Samtools⁵⁰ and read counts were generated using HTSeq⁵¹ (parameters: -m intersection-strict --nonunique all -r pos -s reverse). For further analysis, all mitochondrial genes were removed from the data. RPKM values were calculated by dividing the number of counts by gene length and sequencing depth, followed by multiplying by 10E9. For data normalization, only RefSeq-annotated genes were considered and their RPKM values were normalized by bringing the samples to the same RPKM sum. For generating heatmaps, a pseudocount of 1 was added to RPKM values, and a log2 base was then taken. Afterwards, all rows (genes) of heatmap matrices were scaled to a range (-0.5, 0.5).

### Combined RNA-Seq data analysis for organoid and human brain datasets

Since raw RNA-Seq datasets for human brain regions have not been accessible, combined comparative analysis of BrainSpan and cerebral organoid samples together was re-processed so as to minimize the processing differences between both datasets. This was achieved first by re-processing organoid datasets similar to as described above for processing organoid datasets alone, such that HTSeq parameter -s was changed to "no". in addition, all values generated by merging both RPKM matrices were quantile normalized. To remove non-biological variations revealed by the correspondence analysis (see description below), the function ComBat⁵² from the sva package⁵³ was applied. ComBat uses an empirical Bayesian framework to adjust data for batch effects and other unmeasured sources of variation.

### Correspondence analysis

Correspondence analysis (CA)⁵⁴ is a projection method that represents variables such as expression values of genes as vectors in a multidimensional space. Similar to principal component analysis (PCA), CA reveals also principal axes of the investigated space. This allows projecting data matrix into a low-dimensional subspace, thereby investigating the main variance in the data. Moreover, in contrast to PCA, CA can simultaneously account for samples in a gene-dimensional space and for genes in a sample-dimensional space - showing the information in a so-called biplot. The interpretation of CA biplot is such that one finds the genes characteristic for a (group of) sample(s) in the direction of this sample (group). The farther away from the center the genes lie, the more characteristic they are for the respective sample(s).

Both correspondence analyses from our study were conducted using the 10,000 genes with the highest expression variance across investigated samples. In the combined analysis of brain and organoid samples, both RPKM matrices were first merged and the new matrix was projected into the three-dimensional subspace. The resulting CA plot shows very clearly that the first principal axis accounts for the technical variation between both datasets (data not shown). Hence, to remove the observed bias ComBat was applied, and the result of the final CA of the ComBat-transformed data is shown in **Fig. 1b****,c.**

### Differential gene expression and gene set enrichment analysis between organoids

From the Allen Brain Atlas dataset, markers were estimated for different brain regions during weeks 12 to 21 by comparing the log2 fold change of the RPKM expression in each regional sample compared to all other regions across weeks 12 to 21. Genes were defined as regionally specific if they had a log fold change of at least 2 when compared to samples from all other regions, excluding striatal and amygdala samples, across all weeks. Furthermore striatal and amygdala specific genes were filtered by removing genes with a log fold change of 2 in amygdala or striatal samples compared to all other regions across weeks 12 to 21. To determine differential gene expression across brain organoids derived by different protocols, DeSeq2⁵⁵ was ran on three pairwise treatment comparisons, Triple-i vs Dual-SMADi, Triple-i vs Inhibitor-free, and Dual-SMADi vs Inhibitor-free, using 8 biological replicates of individual organoids from each protocol. A Gene Set Enrichment Analysis (GSEA)⁵⁶ was then run to determine significance of enrichment of the regional specific gene sets in each of the three comparisons. Finally, in-vivo relative expression levels of protocol-specific and shared regional genes were estimated. First, the summed expression of genes from each regional gene set across region-specific BrainSpain samples (ie. for cortical genes, expression level across all cortical samples) from weeks 12 to 21 was estimated. Then, the average of these summed expressions was calculated for regional genes forming each category of protocol comparison (ie. protocol-specific regional genes being consistently up-regulated in each pairwise comparison, and shared genes being not significantly up or down regulated in any pairwise comparison). Finally, a z-score was estimated from these averages across categories with a minimum of five genes, and plotted in the Venn diagrams in **Fig. 1g****.**

### Differential gene expression and pathway enrichment across microcephaly organoids

A differential gene expression analysis was conducted using DESeq2 to compare day 17 and day 30 heterozygous and homozygous microcephaly pooled organoids vs wild type pooled organoids across Triple-i, Dual SMAD-i and Inhibitor-free organoids. A Gene Set Enrichment Analysis was then performed for day 30 homozygous microcephaly pooled organoids for each regional gene set derived from Allen Brain Atlas samples across weeks 12 - 21 using genes with absolute log 2-fold change at least 1. After this, a Benjamini-Hochberg correction was applied to calculate p-values adjusted for multiple comparisons. Pathway enrichments were estimated using Ingenuity Pathway Analysis software (QIAGEN Inc., https://www.qiagenbioinformatics.com/products/ingenuity-pathway-analysis/) separately for genes with log2 fold change greater than 1 (up-regulated) and log2 fold change less than -1 (down-regulated) between microcephaly and wild type pooled organoids.

### Single cell RNA-Seq procedures

### Organoid dissociation

Day 50 organoids (N=4-6 pooled organoids per sample) derived by Triple-i (from ZIP8K8, ZIP13K5, KUCG2 and FOK1 iPSC cell lines), Dual SMAD-i (from ZIP8K8, ZIP13K5, KUCG2 and FOK1 iPSC cell lines), and Inhibitor-free (from ZIP8K8 and ZIP13K5 cell lines) treatments, day 80 individual organoids derived by Triple-i (ZIP8K8 cell line, N=3 individual organoids) and Dual SMAD-i (ZIP8K8 cell line, N=3 individual organoids), as well as pooled Triple-i organoids (ZIP13K5 cell line, 1 pooled experiment across N=3 organoids) were dissociated into single cells using papain dissociation kit (Worthington). Organoids were dissected using needle into small pieces, then incubated with papain and DNase I solution for 35-45 min, and later triturated and cell suspension was filtered twice through 40-micron filter to obtain single cell suspension. Cells were centrifuged at 300g for 5 min, re-suspended in 0.4% BSA in Dulbecco's Phosphate Buffer Solution (DPBS), counted for viability (>80% were viable) and 1000 cells/ µL were used for generating libraries using 10X Chromium single cell 3' reagent kit V3.1 chemistry.

### Single cell library preparation

Roughly 17,400 single live cells were suspended in 0.4% BSA in DPBS buffer (1000 cells/µL) and subjected for GEM generation and barcoding, library preparation was performed according to the recommended procedures of the manufacture Chromium Single Cell 3' reagent Kits V3.1 chemistry. In day 50 organoids, 10,000 cells were targeted for capturing and 9 cycles were used for cDNA amplification while 12 cycles were performed for library formation. For day 80 organoids, 11 cycles are used both for cDNA amplification and library formation. Obtained libraries were sequenced using Illumina short read sequencing.

### Day 50 single cell RNA-Seq data processing and analysis

Single cell RNA-Seq sequencing data from day 50 organoids was processed using Cell Ranger software version 3.1.0⁵⁷ reference genome hg38 and ensembl reference transcriptome version 93 (http://ftp.ensembl.org/pub/release-93/gtf/homo_sapiens/Homo_sapiens.GRCh38.93.gtf.gz). Cell barcodes which had at least 10,000 UMIs or at least 40% mitochondrial UMIs were filtered from downstream analyses. The raw count matrices from single cells across all day 50 organoids were then loaded into scanpy version 1.5.1. The count data was normalized such that each cell had a total count equal to the median of total counts before normalization using scanpy's pp.normalize_total function. The natural log of these normalized counts was then calculated after adding a pseudocount of 1 using numpy's log1p function. The top 2,000 highly variable genes with a mean normalized expression value between 0.005 and 1.5 were then calculated using scanpy's pp.highly_variable_genes function and subsequent dimension reduction and clustering was applied to the log normalized data after subsetting to these genes. First, a principal component analysis was applied. A neighborhood graph of observations was then constructed from the top 50 principal components using scanpy's pp.neighbors function with n_neighbors = 14. A Uniform Manifold Approximation and Projection (UMAP) embedding was then estimated from the neighborhood graph using parameters min_dist=0.1 and spread=1. The UMAP is shown in **Fig. 2a****-e.** Clusters were then estimated using the Louvain method for community detection⁵⁸ in scanpy with resolution=4.

Doublets were detected by running scrublet version 0.2.3⁵⁹ on each sample separately with input parameter expected_doublet_rate = 0.05 and applying a doublet score threshold of 0.2. Cluster 40 contained over 40% of cells annotated by scrublet as doublets, while all other clusters contained between 1% and 12% of doublets. Therefore, all cells assigned as doublets from scrublet, along with all cells from cluster 40, were annotated as doublets, and subsequently removed from further analyses and plotting. The remaining clusters were manually assigned to a cell type and region based on the differential expression of well-established marker genes.

Differential expression of genes per cluster was estimated using a t-test with scanpy's rank_genes_group function and method t-test_overestim_var. Genes were annotated as significantly differentially expressed within a cluster if they had a log2 fold change of at least 1 and q-value less than 0.05 after applying a Benjamini-Hochberg multiple hypothesis correction to the estimated p-values. Clusters 24 and 31 were annotated as "Unknown" due to the lack of known differentially expressed marker genes in these clusters.

### Merging scRNA-Seq day 50 organoids with Bhaduri et al. scRNASeq datasets

First cells collected at weeks 8 and 10 of in-vitro development from Bhaduri et al. ²⁹ are subset and then processed the scRNA-Seq data in a similar manner as described in their paper. First, the log-normalized count data was loaded into scanpy. Batch, indicated in the metadata, was regressed out using scanpy's regress_out function. The expression was then scaled to have unit variance and zero mean, and values were truncated to a maximum value of 10. The logged expression values in cells from our organoids were similarly scaled and truncated to a maximum value of 10. The top 2,000 highly variable genes with a mean normalized expression value of at least 0.0125 were then estimated from the log-normalized expression data in our organoids and separately across the Bhaduri organoids. Then subset to the union of highly variable genes across both datasets is done, and a principal component analysis is applied to the data. A neighborhood graph was then constructed from the top 50 principal components using a batch-balanced k-NN graph approach⁶⁰ in order to account for batch differences across the studies. Finally, a UMAP embedding was estimated from the batch-corrected neighborhood graph with parameters min_dist=0.1 and spread=1. The UMAP is shown in **Fig. 11a** with cells colored by derivation protocol, and the scaled expression values of FOXG1 are shown in **Fig. 11b****.** Then the Pearson correlation was measured between the average scaled expression levels across all cells within each of our organoid clusters and Bhaduri organoid clusters after subsetting to the union of highly variable genes across both datasets. The Pearson correlations are shown in **Fig. 11c**. Individual clusters from the Bhaduri et al. organoids were then assigned to specific regions based on the differential expression of well-established brain region gene sets. Pearson correlations between our cortical organoid clusters and Bhaduri et al. in-vivo clusters across cells from all developmental weeks were estimated in the same way as described above for the Bhaduri et al. in-vitro clusters, and are shown in **Fig. 4c****.**

### Differential gene expression of day 50 cortical cell types

A differential gene expression analysis was ran comparing dividing and non-dividing cortical NSCs (clusters 29, 4, 15, 0, 19, 27 and 12) in Triple-i and Dual SMAD-i organoids, as well as Triple-i and Inhibitor-free organoids. Genes were tested if they were expressed in at least 1 % of all cortical NSCs from either protocol. Differential expression was estimated using a t-test with scanpy's rank_genes_group function and method t-test_overestim_var. Genes were annotated as significantly differentially expressed within a protocol if they had an absolute log2 fold change of at least 1 and q-value less than 0.05 after applying a Benjamini-Hochberg multiple hypothesis correction to the estimated p-values. Then the significantly differentially expressed oRG-specific genes from Pollen et al.³⁹ were highlighted, 66 genes in total, in **Fig. 4d****,e.**

A differential gene expression analysis was ran comparing cortical clusters within Triple-i, Dual SMAD-i and Inhibitor-free organoids separately. Differential expression was again estimated using a t-test with scanpy's rank_genes_group function and method t-test_overestim_var. Only genes expressed in at least 2% of cells within at least one cluster were tested, and labeled as significantly up-regulated if they had a log2 fold change of at least 1 and q-value less than 0.05 after applying a Benjamini-Hochberg multiple hypothesis correction to the estimated p-values.

### Day 80 single cell RNA-Seq data processing and analysis

Single cell RNA-Seq sequencing data from day 80 Triple-i and Dual SMAD-i organoids was processed using Cell Ranger software version 6.0.1 using the same reference genome as day 50 organoids. Cell barcodes which had at least 10,000 UMIs, at least 20% mitochondrial UMIs, or at least 15% ribosomal UMIs, were filtered from downstream analyses, which were conducted in scanpy version 1.5.1. Similar to day 50 organoids, doublets were detected by running scrublet version 0.2.3⁵⁹ on each sample separately with input parameter expected_doublet_rate = 0.05 and applying a doublet score threshold of 0.2. All estimated doublets were further removed from downstream analyses. The count data was then normalized using scanpy's pp.normalize_total function, after which a log1p normalization was applied. The top 2,000 highly variable genes with a mean normalized expression value between 0.005 and 1.5 were then calculated using scanpy's pp.highly_variable_genes function. The expression values of these genes was then scaled to have unit variance and zero mean, and values were truncated to a maximum value of 10. To remove batch effects, scanorama⁶¹ was employed, a panoramic stitching algorithm, using the scaled expression values as input and treating each individual organoid as its own batch. The top 20 dimensions from the scanorama batch corrected low dimensional embedding was then used to build a k-nearest neighbors graph with n_neighbors = 20. A UMAP embedding was then estimated from the neighborhood graph using parameters min_dist=0.6 and spread=1, and is shown in **Fig. 6j****.** Clusters were then estimated using the Louvain method for community detection in scanpy with resolution=2. Clusters were manually assigned to a cell type and region based on the differential expression of well-established marker genes, including deep and upper layer neuronal marker gene sets^{40, 41}.

### Day 80 single cell RNA-Seq RNA velocity

The velocyto version 0.17.16⁶² was ran on the day 80 scRNA-Seq data to first measure the number of spliced and unspliced UMIs for each gene in each cell. These spliced and unspliced counts were then loaded into python using the scvelo package version 0.1.24⁶³. Then mesenchymal cells were removed, as these cells were not part of the neural lineage. Genes with less than 30 total spliced counts and less than 30 total unspliced counts across all cells in the dataset were filtered, leaving 8,982 genes. Then, the spliced and unspliced counts were normalized separately such that each cell had a total count equal to the median of total counts before normalization, after which a log1p transformation was performed. After this, the spliced and unspliced counts were averaged over the 30 nearest neighbors of each cell using scvelo's pp.moments function, in effect smoothing the data across each cell's local neighborhood. Finally, RNA velocity was estimated using the "deterministic" mode with scvelo's tl.velocity function, which measures the RNA velocity as the deviation from the approximated steady-state equilibrium over the locally averaged spliced and unspliced counts for each cell. The velocity estimates were then embedded onto the UMAP after removing mesenchymal cells using scvelo's tl.velocity_graph and pl.velocity_embedding_stream functions.

### Differential gene expression of day 80 cortical NSCs

A differential gene expression analysis was ran comparing cortical NSCs in ZIP8K8 and ZIP13K5 Triple-i and ZIP8K8 Dual SMAD-i organoids. Genes were tested if they were expressed in at least 1% of all cortical NSCs from either protocol. Differential expression was estimated using a t-test with scanpy's rank_genes_group function and method t-test_overestim_var. Genes were annotated as significantly differentially expressed within a protocol if they had an absolute log2 fold change of at least 0.1 and q-value less than 0.1 after applying a Benjamini-Hochberg multiple hypothesis correction to the estimated p-values. A Fisher's exact test was ran to estimate the enrichment of oRG-specific genes derived from Pollen et al. ³⁹ among the up-regulated genes in Triple-i (p=1.2e-10) and separately among the up-regulated genes in Dual SMAD-I (p=0.1).

### Immunostaining and confocal imaging

Cells were fixed in 4% paraformaldehyde, 0.15% picric acid, permeabilized and blocked with PBS, 1% bovine serum albumin (BSA), 10% FBS and 0.3% Triton solution. Organoids were similarly fixed, washed, cryoprotected with 30% sucrose overnight and then submerged in OCT. Fixed cells or sectioned organoids (10uM slices) were stained with indicated primary antibodies (see below) followed by Alexa Fluor secondary antibodies (Invitrogen). Following staining, cells were imaged in PBS, and organoid sections were mounted on Moviol (Sigma). Fluorescence images were obtained using a confocal microscope LSM710 (Carl Zeiss Micro Imaging, Germany). The confocal images were captured using 10X and 20X objectives (NA=0.3 and 0.8, Plan-Apochromat, respectively). Fluorescence emissions resulting from Ar 488, 543 and 633 nm laser lines for EGFP, CY3 and CY5, respectively, were detected using laser scanning settings and filter sets supplied by the manufacturer. For DAPI detection in all images as well as GFP detection for organoid images, a mode-locked *Ti:Sapphire,* fento second pulsed, multiphoton laser (Chameleon Ultra II, Coherent, Inc.) was used at a wavelength of 720 nm and 920 nm, respectively. Epi fluorescent and phase contrast images were obtained using Nikon Eclipse Ti-E microscope. Fluorescence emissions results from mercury arc lamp. Images were taken using 10X and 20X objectives. Images were generated and analyzed using either Zeiss ZEN 2011 software (Carl Zeiss, Inc.) or NIS elements software (Nikon). All images were exported in TIF and their color levels were identically adjusted per each staining procedure.

### Antibody list

Antibodies for EMX2 (ab94713, 1:50), FOXG1 (ab18259, 1:400), p-VIMENTIN (ab22651, 1:120), SOX2 (ab79351, 1:500), SATB2 (ab51502, 1:50), CTIP2 (ab18465, 1:250), TBR1 (ab31940, 1:500) and CUX1 (ab54583, 1:200) were from Abcam. Antibody for OCT3/4 (sc5279, 1:22), LIFR (sc-515337, 1:100) was from Santa Cruz. Antibody for PAX6 (supernatant, 1:22) was from DSHB. Antibodies for SOX1 (AF3369, 1:40), SOX2 (AF2018, 1:100), and OLIG3 (MAB32456, 1:450) were from R&D. Antibodies for COUP-TF1 (1:500, ABE1425), DCX (1:500, AB2253) and LMX1A (AB10533, 1:1000) were from Millipore. Antibodies for SP8 (HPA054006, 1:50) and MEF2C (HPA005533, 1:100) were from Atlas Antibodies. Antibodies for activated CAS-3 (#9661, 1:500) and TCF7L2 (#2569, 1:500) were from Cell Signaling. Antibody for TTR (AHP1837, 1:500) was from Biorad. Antibodies for HOPX (HPA030180, 1:500), PTPRZ1 (HPA015103, 1:500) and EMX1 (HPA006421, 1:50) were from Sigma-Aldrich.

### Quantitative PCR (qPCR) analysis

RNA was extracted using miRNeasy kit (Qiagene) followed by cDNA reverse transcription kit (Applied Biosystems). 4-6ng of cDNA was subjected to qPCR using our homemade designed primers (see 'Primer set list'), FastStart universal SYBR green (Roche) and ViiA-7cycler (ABI). Threshold cycle values were determined in triplicates and presented as average relative fold over those of HPRT. Fold changes were calculated using the 2^{-Δct} method.

### Primer set list

BRACHYURY Forward, 5'-ACCCAGTTCATAGCGGTGAC-3' and Reverse 5'-CAATTGTCATGGGATTGCAG-3'; FOXA2 Forward, 5'-CCGACTGGAGCAGCTACTATG -3' and Reverse 5'-TGTACGTGTTCATGCCGTTC-3'; HPRT Forward, 5'-TGACACTGGCAAAACAATGCA-3' and Reverse 5'-GGTCCTTTTCACCAGCAAGCT-3'; OCT4 Forward, 5'-CAGCAGATCAGCCACATC-3' and Reverse 5'-CGGTTACAGAACCACACTC-3'; SOX1 Forward, 5'-GCAAGATGGCCCAGGAGAAC-3' and Reverse 5'-CGGACATGACCTTCCACTCG-3'; SOX17 Forward, 5'-AAGATGACTGGCAAGTCGT-3' and Reverse 5'-CTTCAGCCGCTTCACCTG-3'; SOX2 Forward, 5'-GCAAGATGGCCCAGGAGAAC-3' and Reverse 5'-CCGACAAAAGTTTCCACTCGG-3' (SEQ ID NOs 9 to 22).

### Image analysis

Immunostainings for regional markers **Fig. 2** and cortical neuronal markers in **Fig. 6** were processed and analyzed on a dedicated Zen Blue version 3.2 workstation (Zeiss, Germany). Briefly, z-stacked images underwent maximal intensity projection, tiled images were stitched together before proceeding with quantification. Image analysis was performed with the Image analysis module in Zen software suite, and followed a hierarchical strategy. The full images or image subsets were divided into two segregating classes (rosettes vs. non-rosettes/cortical units vs. non-cortical units) by taking advantage of the locally enhanced density of nuclei. Therefore, thresholds were determined from fixed fluorescence intensity values from over-smoothed nuclear counter stain images. Within the two resulting classes, single cells were identified by faint smoothing, rolling ball background subtraction, watershedding and fixed intensity thresholds. The resulting masks were applied to the raw input images and the fluorescence intensity for all channels were calculated within regions of interest as mean intensity. Marker intensity cutoffs were determined manually to classify positive cells and the results were plotted in custom python scripts.

### Statistical analysis

For all qPCR gene expression analysis obtained for iPSC characterization experiments, two-way ANOVA test followed by Tukey's multiple comparison test were applied. Statistical analysis was performed using GraphPad software. For calculating organoid size, ImageJ or Nis-elements (Nikon) software was used. See figure legends for statistical tests used in the corresponding figure. Statistical analysis was performed using GraphPad software and scipy.stats package in python3.

### REFERENCES

1. Alvarez-Buylla, A. & Temple, S. Stem cells in the developing and adult nervous system. Journal of Neurobiology 36, 110 (1998).
2. Florio, M. & Huttner, W.B. Neural progenitors, neurogenesis and the evolution of the neocortex. Development 141, 2182-2194 (2014).
3. Watanabe, K. et al. Directed differentiation of telencephalic precursors from embryonic stem cells. Nat.Neurosci. 8, 288-296 (2005).
4. Elkabetz, Y. & Studer, L. Human ESC-derived neural rosettes and neural stem cell progression. Cold Spring Harb Symp Quant Biol 73, 377-387 (2008).
5. Elkabetz, Y. et al. Human ES cell-derived neural rosettes reveal a functionally distinct early neural stem cell stage. Genes & Development 22, 152-165 (2008).
6. Chambers, S.M. et al. Highly efficient neural conversion of human ES and iPS cells by dual inhibition of SMAD signaling. Nat Biotechnol 27, 275-280 (2009).
7. Gaspard, N. et al. An intrinsic mechanism of corticogenesis from embryonic stem cells. Nature 455, 351-357 (2008).
8. Mariani, J. et al. Modeling human cortical development in vitro using induced pluripotent stem cells. Proc Natl Acad Sci U S A 109, 12770-12775 (2012).
9. Shi, Y., Kirwan, P., Smith, J., Robinson, H.P. & Livesey, F.J. Human cerebral cortex development from pluripotent stem cells to functional excitatory synapses. Nat Neurosci 15, 477-486, S471 (2012).
10. Boissart, C. et al. Differentiation from human pluripotent stem cells of cortical neurons of the superficial layers amenable to psychiatric disease modeling and high-throughput drug screening. Translational psychiatry 3, e294 (2013).
11. Qi, Y. et al. Combined small-molecule inhibition accelerates the derivation of functional cortical neurons from human pluripotent stem cells. Nat Biotechnol 35, 154-163 (2017).
12. Yao, Z. et al. A Single-Cell Roadmap of Lineage Bifurcation in Human ESC Models of Embryonic Brain Development. Cell Stem Cell 20, 120-134 (2017).
13. Maroof, A.M. et al. Directed differentiation and functional maturation of cortical interneurons from human embryonic stem cells. Cell Stem Cell 12, 559-572 (2013).
14. Edri, R. et al. Analysing human neural stem cell ontogeny by consecutive isolation of Notch active neural progenitors. Nature communications 6, 6500 (2015).
15. Lancaster, M.A. & Knoblich, J.A. Generation of cerebral organoids from human pluripotent stem cells. Nat Protoc 9, 2329-2340 (2014).
16. Camp, J.G. et al. Human cerebral organoids recapitulate gene expression programs of fetal neocortex development. Proc Natl Acad Sci U S A 112, 15672-15677 (2015).
17. Quadrato, G. et al. Cell diversity and network dynamics in photosensitive human brain organoids. Nature 545, 48-53 (2017).
18. Pasca, A.M. et al. Functional cortical neurons and astrocytes from human pluripotent stem cells in 3D culture. Nat Methods 12, 671-678 (2015).
19. Qian, X. et al. Brain-Region-Specific Organoids Using Mini-bioreactors for Modeling ZIKV Exposure. Cell 165, 1238-1254 (2016).
20. Eiraku, M. et al. Self-organized formation of polarized cortical tissues from ESCs and its active manipulation by extrinsic signals. Cell Stem Cell 3, 519-532 (2008).
21. Kadoshima, T. et al. Self-organization of axial polarity, inside-out layer pattern, and species-specific progenitor dynamics in human ES cell-derived neocortex. Proc Natl Acad Sci U S A 110, 20284-20289 (2013).
22. Bershteyn, M. et al. Human iPSC-Derived Cerebral Organoids Model Cellular Features of Lissencephaly and Reveal Prolonged Mitosis of Outer Radial Glia. Cell Stem Cell 20, 435-449 e434 (2017).
23. lefremova, V. et al. An Organoid-Based Model of Cortical Development Identifies Non-Cell-Autonomous Defects in Wnt Signaling Contributing to Miller-Dieker Syndrome. Cell Rep 19, 50-59 (2017).
24. Velasco, S. et al. Individual brain organoids reproducibly form cell diversity of the human cerebral cortex. Nature 570, 523-527 (2019).
25. Benito-Kwiecinski, S. et al. An early cell shape transition drives evolutionary expansion of the human forebrain. Cell 184, 2084-2102 e2019 (2021).
26. Xiang, Y. et al. Generation and Fusion of Human Cortical and Medial Ganglionic Eminence Brain Organoids. Current protocols in stem cell biology 47 (2018).
27. Sloan, S.A., Andersen, J., Pasca, A.M., Birey, F. & Pasca, S.P. Generation and assembly of human brain region-specific three-dimensional cultures. Nat Protoc 13, 2062-2085 (2018).
28. Tanaka, Y., Cakir, B., Xiang, Y., Sullivan, G.J. & Park, I.H. Synthetic Analyses of Single-Cell Transcriptomes from Multiple Brain Organoids and Fetal Brain. Cell Rep 30, 1682-1689 e1683 (2020).
29. Bhaduri, A. et al. Cell stress in cortical organoids impairs molecular subtype specification. Nature 578, 142-148 (2020).
30. Lancaster, M.A. et al. Cerebral organoids model human brain development and microcephaly. Nature 501, 373-379 (2013).
31. Zhou, T. et al. High-Content Screening in hPSC-Neural Progenitors Identifies Drug Candidates that Inhibit Zika Virus Infection in Fetal-like Organoids and Adult Brain. Cell Stem Cell 21, 274-283 e275 (2017).
32. Kang, H.J. et al. Spatio-temporal transcriptome of the human brain. Nature 478, 483-489 (2011).
33. Moore SA, lulianella A. Development of the mammalian cortical hem and its derivatives: the choroid plexus, Cajal-Retzius cells and hippocampus. Open Biol. 11(5), 210042 (2021).
34. Achira Roy, Miriam Gonzalez-Gomez, Alessandra Pierani, Gundela Meyer, Shubha Tole. Lhx2 Regulates the Development of the Forebrain Hem System. Cerebral Cortex 25(5), 1361-1372 (2014).
35. Anderson, R.M., Lawrence, A.R., Stottmann, R.W., Bachiller, D. & Klingensmith, J. Chordin and noggin promote organizing centers of forebrain development in the mouse. Development 129, 4975-4987 (2002).
36. Placantonakis, D. et al. BAC transgenesis in human ES cells as a novel tool to define the human neural lineage. Stem Cells (2008).
37. O'Leary, D.D., Chou, S.J. & Sahara, S. Area patterning of the mammalian cortex. Neuron 56, 252-269 (2007).
38. Simeone, A. et al. Two vertebrate homeobox genes related to the drosophila-empty spiracles gene are expressed in the embryonic cerebral-cortex. EMBO Journal 11, 2541-2550 (1992).
39. Pollen, A.A. et al. Molecular Identity of Human Outer Radial Glia during Cortical Development. Cell 163, 55-67 (2015).
40. Molyneaux, B.J., Arlotta, P., Menezes, J.R. & Macklis, J.D. Neuronal subtype specification in the cerebral cortex. Nature reviews. Neuroscience 8, 427-437 (2007).
41. Lodato, S. & Arlotta, P. Generating neuronal diversity in the mammalian cerebral cortex. Annual review of cell and developmental biology 31, 699-720 (2015).
42. Kumar, A., Girimaji, S.C., Duvvari, M.R. & Blanton, S.H. Mutations in STIL, encoding a pericentriolar and centrosomal protein, cause primary microcephaly. American journal of human genetics 84, 286-290 (2009).
43. Ziller, M.J. et al. Dissecting neural differentiation regulatory networks through epigenetic footprinting. Nature 518 355-359 (2015).
44. Ziv, O. et al. Quantitative Live Imaging of Human Embryonic Stem Cell Derived Neural Rosettes Reveals Structure-Function Dynamics Coupled to Cortical Development. PLoS computational biology 11, e1004453 (2015).
45. Okita, K. et al. A more efficient method to generate integration-free human iPS cells. Nat Methods 8, 409-412 (2011).
46. Beers, J. et al. Passaging and colony expansion of human pluripotent stem cells by enzyme-free dissociation in chemically defined culture conditions. Nat Protoc 7, 2029-2040 (2012).
47. Kanton, S. et al. Organoid single-cell genomic atlas uncovers human-specific features of brain development. Nature 574, 418-422 (2019).
48. Tandon, R. et al. Generation of two human isogenic iPSC lines from fetal dermal fibroblasts. Stem Cell Res 33, 120-124 (2018).
49. Dobin, A. et al. STAR: ultrafast universal RNA-seq aligner. Bioinformatics 29, 15-21 (2013).
50. Li, H. et al. The Sequence Alignment/Map format and SAMtools. Bioinformatics 25, 2078-2079 (2009).
51. Anders, S., Pyl, P.T. & Huber, W. HTSeq--a Python framework to work with high-throughput sequencing data. Bioinformatics 31, 166-169 (2015).
52. Johnson, W.E., Li, C. & Rabinovic, A. Adjusting batch effects in microarray expression data using empirical Bayes methods. Biostatistics 8, 118-127 (2007).
53. Leek, J.T., Johnson, W.E., Parker, H.S., Jaffe, A.E. & Storey, J.D. The sva package for removing batch effects and other unwanted variation in high-throughput experiments. Bioinformatics 28, 882-883 (2012).
54. Fellenberg, K. et al. Correspondence analysis applied to microarray data. Proc Natl Acad Sci U S A 98, 10781-10786 (2001).
55. Love, M.I., Huber, W. & Anders, S. Moderated estimation of fold change and dispersion for RNA-seq data with DESeq2. Genome Biol 15, 550 (2014).
56. Subramanian, A. et al. Gene set enrichment analysis: a knowledge-based approach for interpreting genome-wide expression profiles. Proc Natl Acad Sci U S A 102, 15545-15550 (2005).
57. Zheng, G.X. et al. Massively parallel digital transcriptional profiling of single cells. Nature communications 8, 14049 (2017).
58. Blondel, V.D., Guillaume, J.L., Lambiotte, R. & Lefebvre, E. Fast unfolding of communities in large networks. J Stat Mech-Theory E (2008).
59. Wolock, S.L., Lopez, R. & Klein, A.M. Scrublet: Computational Identification of Cell Doublets in Single-Cell Transcriptomic Data. Cell Syst 8, 281-291 e289 (2019).
60. Polanski, K. et al. BBKNN: fast batch alignment of single cell transcriptomes. Bioinformatics 36, 964-965 (2020).
61. Hie, B., Bryson, B. & Berger, B. Efficient integration of heterogeneous single-cell transcriptomes using Scanorama. Nat Biotechnol 37, 685-691 (2019).
62. La Manno, G. et al. RNA velocity of single cells. Nature 560, 494-498 (2018).
63. Bergen, V., Lange, M., Peidli, S., Wolf, F.A. & Theis, F.J. Generalizing RNA velocity to transient cell states through dynamical modeling. Nat Biotechnol 38, 1408-1414 (2020).

## Claims

1. A method for the generation of outer radial glial (oRG) cells of the outer sub-ventricular (oSVZ)-like region in cerebral organoids comprising
(a) culturing primate stem cells in a primate stem cell medium until about day 6, thereby inducing the formation of embryonic bodies;
(b) culturing the embryonic bodies as obtained in step (a) in a neural induction medium until about day 11, thereby inducing the formation of organoids;
wherein an inhibitor of TGF-β, an inhibitor of BMP and an inhibitor of WNT is present from about day 2 until about day 11;
(c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until about day 40, preferably at least until about day 60 and most preferably at least until about day 80, thereby obtaining cerebral organoids with oRG cells in oSVZ-like regions, wherein the organoids in step (c) are subjected to agitation from about day 15 onward, preferably by using an orbital shaker or a spinning bioreactor; and
(d) optionally isolating one or more oRG cells from the oSVZ-like region.

2. A method for determining whether a test compound is an inhibitor or activator of the generation of outer radial glial (oRG) cells comprising
(a) culturing primate stem cells in a primate stem cell medium until about day 6, thereby inducing the formation of embryonic bodies;
(b) culturing the embryonic bodies as obtained in step (a) in a neural induction medium until about day 11, thereby inducing the formation of organoids;
wherein an inhibitor of TGF-β, an inhibitor of BMP, an inhibitor of WNT and the test compound are present from about day 2 until about day 11; and
(c) embedding the organoids as obtained after steps (a) and (b) and if they display a size of at least 300 µm into a hydrogel that mimics the extracellular matrix (ECM), preferably Matrigel and culturing the organoids in a cerebral differentiation medium at least until day 40, thereby obtaining cerebral organoids with oRG cells in pSVZ-like regions, wherein the organoids in step (c) are subjected to agitation from about day 15 onward, preferably by using an orbital shaker or a spinning bioreactor,
wherein the test compound is an inhibitor of the generation of oRG cells if the number of oRG cells in oSVZ-like regions is reduced as compared to the absence of the test compound, and wherein the test compound is an activator of the generation of oRG cells if the number of oRG cells in oSVZ-like regions is increased as compared to the absence of the test compound.

3. The method of claim 1 or 2, wherein the primate stem cells are feeder-dependent or feeder-independent.

4. The method of any one of claims 1 to 3, wherein the primate stem cells are primate embryonic stem cells or primate induced pluripotent stem cells.

5. The method of any one of claims 1 to 4, wherein
(a) the inhibitor of TGF-β is SB-431542,
(b) the inhibitor of BMP is Noggin, and/or
(c) the inhibitor of WNT is XAV-939.

6. The method of any one of claims 1 to 5, wherein the primate stem cell medium and/or the neural induction medium comprises a ROCK inhibitor until the embryonic bodies reach a size of at least 300 µm in diameter, wherein the ROCK inhibitor is preferably Y27632.

7. The method of any one of claims 1 to 6, wherein the primate stem cell medium and/or the neural induction medium comprises medium supplement B27, preferably medium supplement B27 without retionic acid before agitation in step (c) and medium supplement B27 with retionic acid during agitation in step (c).

8. The method of any one of claims 1 to 7, wherein the neural induction medium comprises or is N2 medium.

9. The method of any one of claims 1 to 8, wherein the cerebral differentiation medium comprises N2 medium and Neurobasal medium supplemented with medium supplement B27, preferably medium supplement B27with retionic acid.

10. The method of any one of claims 1 to 9, further comprising the identification of the oRG cells in the cerebral organoids by the detection of the expression of the marker genes LIFR, PTPRZ1 and SOX2.

11. The method of any one of claims 1 to 10, further comprising the identification of the oSVZ-like region in the cerebral organoids by the detection of the expression of the marker genes TBR2, HOPX, and SOX2.

12. An oRG cell that has been produced or is producible by the method of any one of claims 1 to 11.
